# EUROPEAN PATENT APPLICATION

(11) **EP 4 458 820 A1**
(43) Date of publication of application: **06.11.2024**
(21) Application number: 22914967.9
(22) Date of filing: 28.12.2022
(51) Int. Cl.: C07D 403/12, C07D 403/14, C07D 401/12, C07D 401/14, C07D 407/14, C07C 409/14, C07D 471/00, C07D 405/14, C07K 14/705, C07K 14/72, A61K 31/53, A61P 1/16, A61P 9/10, A61P 9/12, A61P 3/04, A61P 3/06, A61P 3/10, A61P 3/00, A61P 9/00

(54) **CLASS OF QUINOLINE COMPOUNDS, PREPARATION METHOD THEREFOR, PHARMACEUTICAL COMPOSITION AND USE THEREOF**

(30) Priority: 28.12.2021 CN 202111627000
(71) Applicant: Shanghai Institute of Materia Medica, Chinese Academy of Sciences, Pudong, Shanghai 201203 (CN)
(72) Inventor: LIU, Hong, Shanghai 201203 (CN); LI, Jia, Shanghai 201203 (CN); WANG, Jiang, Shanghai 201203 (CN); ZANG, Yi, Shanghai 201203 (CN); CHEN, Feiyang, Shanghai 201203 (CN); GAO, Lixin, Shanghai 201203 (CN); ZHAO, Jing, Shanghai 201203 (CN); SUN, Dandan, Shanghai 201203 (CN); WANG, Ke'an, Shanghai 201203 (CN); JIANG, Hualiang, Shanghai 201203 (CN); XIE, Rongrong, Shanghai 201203 (CN)
(74) Representative: Mewburn Ellis LLP
(86) International application number: PCT/CN2022/142988
(87) International publication number: WO 2023/125716

(57) **Abstract**

Provided in the present invention are a class of quinoline compounds and a preparation method therefor, a pharmaceutical composition and the use thereof. Specifically, provided in the present invention are a compound as shown in formula(I), and a stereoisomer, a deuterated compound, a solvate, a metabolite, a pharmaceutically acceptable salt, a eutectic or a prodrug thereof, and a pharmaceutical composition thereof. Further provided in the present invention are a preparation method for the compound and the use thereof in the preparation of a drug for treating diseases mediated by thyroid hormone receptors. Related diseases include, but are not limited to, diseases such as non-alcoholic fatty liver diseases, atherosclerosis, coronary heart disease, hypertension, hypercholesterolemia, hyperlipidemia, hypertriglyceridemia, dyslipidemia, obesity, diabetes, metabolic disorders, lipid metabolism disorders, 1A type glycogen storage diseases, hypothyroidism and thyroid cancer.

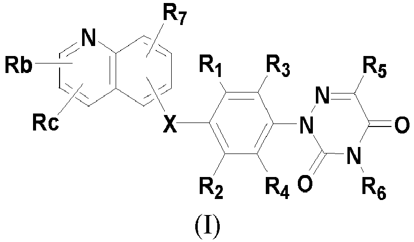

## Description

### Technical field

The present invention belongs to pharmaceutical field, and relates to a class of quinoline compounds, and a stereoisomer, a deuterated compound, a solvate, a metabolite, a pharmaceutically acceptable salt, a eutectic or a prodrug thereof, and a pharmaceutical composition thereof, preparation method therefor, and use in the preparation of medications for thyroid hormone receptors mediated diseases. Related diseases include, but are not limited to, diseases such as non-alcoholic fatty liver diseases, atherosclerosis, coronary heart disease, hypertension, hypercholesterolemia, hyperlipidemia, hypertriglyceridemia, dyslipidemia, obesity, diabetes, metabolic disorders, lipid metabolism disorders, type 1A glycogen storage diseases, hypothyroidism and thyroid cancer.

### Background

Thyroid hormones are critical for normal growth and development and for maintaining metabolic balance. There are two main forms of thyroid hormones present in human circulatory system: triiodothyronine (T3) and tetraiodothyronine(T4). Although T4 is the main form of thyroid secretion, T3 has higher physiological activity. In the body, T4, after being released into the circulatory system via thyroid secretion, is converted to T3 by tissue-specific deiodinase, which is present in all tissues, but mainly in the liver and kidney.

Thyroid hormone TH exerts its physiologic effects primarily through the thyroid hormone receptor TR. TR is a member of the nuclear receptor superfamily and is a ligand T3-induced transcription factor and plays a central role in mediating the action of ligand T3. After being activated by T3, TR exerts important effects on physiological functions and basal metabolism of the body. There are two subtypes of TR receptors, with tissue and functional differences in the human body. The α subtype is mainly distributed in the heart and is mainly used to regulate heart rate. The β subtype is mainly found in liver and is associated with hepatic cholesterol metabolism and the secretion of thyroid-stimulating hormone (TSH) by the pituitary gland.

Tachycardia, arrhythmia, heart failure, as well as fatigue, shortness of breath, and sarcopenia and osteoporosis have been observed in patients with hyperthyroidism, as well as beneficial effects on metabolic diseases, such as reduced blood cholesterol levels and increased basal metabolic rate. Conversely, decreased heart rate, increased cholesterol in blood, and weight gain were observed in hypothyroidism caused by pituitary disorders and congenital dysfunction. This is the reason why the presence of cardiotoxicity of natural thyroid hormones limits their therapeutic use.

Thyroid hormones have been confirmed to lower serum low-density lipoprotein levels. Thyroid hormones stimulate cholesterol metabolism to bile acids by increasing hepatic LDL receptor expression. In animal models, thyroid hormones have been shown to have the beneficial effect of increasing the expression of HDL cholesterol and apo A-1 (one of the major deacylated lipoproteins of HDL) and improving the conversion of LDL to HDL.

Non-alcoholic fatty liver disease is also closely related to thyroid hormones. On the one hand, functions such as conversion and inactivation of thyroid hormones are impaired in NAFLD patients, which may lead to a decrease in serum thyroid hormones; on the other hand, a decrease in thyroid hormone further causes lipid metabolism disorders, and glucose metabolism disorders, and exacerbates the course of NAFLD. It was shown that fatty liver formation was induced in rats using a choline-methionine-deficient diet, and then reversal of fatty liver was observed under T3 administration.

However, endogenous thyroid hormones are non-selective and have side effects that commonly cause hyperthyroidism, especially those related to cardiovascular toxicity. Therefore, the development of thyroid hormone analogs that can avoid hyperthyroidism and the side effects of hyperthyroidism is of great importance to broaden the treatment pathways for hyperlipidemia, hypercholesterolemia, diabetes, fatty degeneration of liver, non-alcoholic fatty liver disease, atherosclerosis, cardiovascular disease, hypothyroidism, thyroid cancer, thyroid disorders, and other related disorders.

### SUMMARY OF THE INVENTION

The present invention provides a class of compounds with good agonist activity on thyroid hormone β receptors, the compound and composition thereof can be used to prepare medications for preventing, treating or alleviating diseases such as non-alcoholic fatty liver diseases, atherosclerosis, coronary heart disease, hypertension, hypercholesterolemia, hyperlipidemia, hypertriglyceridemia, dyslipidemia, obesity, diabetes, metabolic disorders, lipid metabolism disorders, type 1A glycogen storage diseases, hypothyroidism and thyroid cancer in patients.

The present invention also relates to a pharmaceutical composition, wherein the pharmaceutical composition comprises the compound of formula (I) as described in any of the above, or a stereoisomer, a deuterated compound, a solvate, a metabolite, a pharmaceutically acceptable salt, a eutectic or a prodrug thereof; and pharmaceutically acceptable excipients/carriers.

The present invention also relates to a use of the compound of the present invention, or the pharmaceutical composition of the present invention in the preparation of medications, wherein, the medication is used for agonizing thyroid hormone receptors, or for preventing, treating or alleviating diseases mediated by thyroid hormone receptors. The diseases mediated by thyroid hormone receptors are non-alcoholic fatty liver disease, atherosclerosis, coronary heart disease, hypertension, hypercholesterolemia, hyperlipidemia, hypertriglyceridemia, dyslipidemia, obesity, diabetes, metabolic disorder, disorder of lipid metabolism, type 1A glycogen storage disease, hypothyroidism or thyroid cancer.

The present invention relates to a method of using the compound or pharmaceutical composition of the present invention for agonizing thyroid hormone receptors, or for preventing, treating, or alleviating diseases mediated by thyroid hormone receptors, wherein the method is to administer a therapeutically effective amount of the compound or pharmaceutical composition to a individual in need thereof. Moreover, the above-mentioned compounds or the pharmaceutical composition thereof provided by the present invention can be used in conjunction with other therapies or therapeutic agents. The usage method can be simultaneous, sequential, or at regular intervals.

In the first aspect of the present invention, provided is a compound of formula I, or a stereoisomer, a deuterated compound, a solvate, a metabolite, a pharmaceutically acceptable salt, a eutectic or a prodrug thereof, 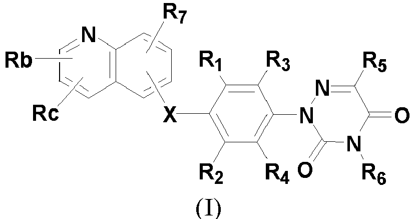
wherein, 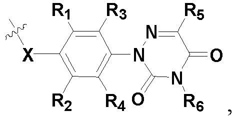 Rb, Rc and R₇ are independently located on any substitutable ring atom of the quinoline ring;
Rb and Rc are independently selected from the group consisting of: D, tritium, halogen, C1-C6 alkyl, C2-C6 alkenyl, C2-C6 alkynyl, C1-C6 alkoxy, C3-C8 cycloalkyl, 3-8-membered heterocyclyl, phenyl, C5-C7 heteroaryl, and C5-C6 substituted heteroaryl; and Rb and Rc are different substitutents;
R₇ is one or more (such as 1, 2, 3, or 4) substituents located on the benzene of the quinoline ring and is selected from the group consisting of: H, deuterium, tritium, halogen, C1-C6 alkyl, and C1-C6 haloalkyl;
X is selected from the group consisting of: -O-, -CH₂-, -C(=O)-, -CH(OH)-, and -S-;
R₁, R₂, R₃ and R₄ are independently selected from the group consisting of: H, D, F, Cl, Br, I, -CN, -OH, -NO₂, -OR₁ₐ, -NR_{1b}R_{1c}, -SR_{1b}, C1-C6 alkyl, C1-C6 alkoxy, C2-C6 alkenyl, C2-C6 alkynyl, C3-C8 cycloalkyl, 3-8-membered heterocycloalkyl, C5-C6aryl, and 5-6-membered heteroaryl;
R₅ is selected from the group consisting of: H, D, F, Cl, Br, I, -CN, -OH, -NO₂, -OR₁ₐ, -NR_{1b}R_{1c}, -SR_{1b}, -S(O)R₁ₐ, -S(O)₂R₁ₐ, -S(O)NR₁ₐR_{1b}, -S(O)₂NR₁ₐR_{1b}, -NHS(O)₂R₁ₐ, -C(O)R₁ₐ, -OC(O)R₁ₐ, -C(O)OR_{1b}, -C(O)NR_{1b}R_{1c}, -NHC(O)R₁ₐ, C1-C6 alkyl, C1-C6 alkoxy, C2-C6 alkenyl , C2-C6 alkynyl, C3-C8 cycloalkyl, 3-8-membered heterocyclyl, C5-C6 aryl and 5-6-membered heteroaryl;
R₆ is selected from the group consisting of: H, D, tritium, halogen, C1-C6 alkyl, amino substituted C1-C6 alkyl, C3-C8 cycloalkyl, 3-8-membered heterocyclyl, -SR_{1b}, -S(O)R₁ₐ, -S(O)₂R₁ₐ, -S(O)NR₁ₐR_{1b}, -C(O)R₁ₐ, -C(O)OR_{1b}, and -C(O)NR_{1b}R_{1c};
unless otherwise specified, each alkyl, alkoxy, alkenyl, alkynyl, cycloalkyl, heterocyclyl, aryl, and heteroaryl mentioned above may optionally have 1-3 substituents selected from the group consisting of: D, tritium, halogen, -CN, -OH, oxo-, -OR₁ₐ, -NR_{1b}R_{1c}, -SR_{1b}, -S(O)R₁ₐ, -S(O)₂R₁ₐ, -S(O)NR₁ₐR_{1b}, -S(O)₂NR₁ₐR_{1b}, - NHS(O)₂R₁ₐ, -C(O)R₁ₐ, - OC(O)R₁ₐ, -C(O)OR_{1b}, - C(O)NR_{1b}R_{1c}, -NHC(O)R₁ₐ, C1-C6 alkyl, and C1-C6 haloalkyl;
R₁ₐ, R_{1b} and R_{1c} are each independently selected from: H, D, C1-C6 alkyl, C2-C6 alkenyl, C2-C6 alkynyl, C3-C8 cycloalkyl, 3-8-membered heterocycloalkyl, C5-C6 aryl and 5-6-membered heteroaryl.

In another preferred embodiment, Rb is selected from the group consisting of: C1-C6 haloalkyl, C3-C8 cycloalkyl, 3-8-membered heterocyclyl, phenyl, and C5-C7 heteroaryl; and phenyl or heteroaryl is optionally substituted by 1-3 substituents selected from the group consisting of: D, tritium, halogen, -CN, -OH, oxo-, -OR₁ₐ, -NR_{1b}R_{1c}, -C(O)R₁ₐ, -C(O)OR_{1b}, -C(O)NR_{1b}R_{1c}, C1-C6 alkyl, and C1-C6 haloalkyl;
Rc is selected from the group consisting of: halogen, C1-C6 alkyl, C1-C6 alkoxy, C1-C6 haloalkyl, and C1-C6 deuterated alkyl.

In another preferred embodiment, Rb is selected from the group consisting of: phenyl, and C5-C7 heteroaryl.

In another preferred embodiment, Rb is selected from the group consisting of: phenyl, and pyridinyl,
Rc is selected from the group consisting of: halogen, C1-C6 alkyl, C1-C6 alkoxy, C1-C6 haloalkyl, and C1-C6 deuterated alkyl.

In another preferred embodiment, the compound of formula I has a structure shown in formula Ia:

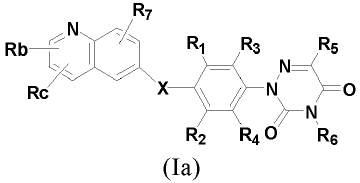

In another preferred embodiment, the compound of formula I has the structure shown in formula Ib or Ic as follows:

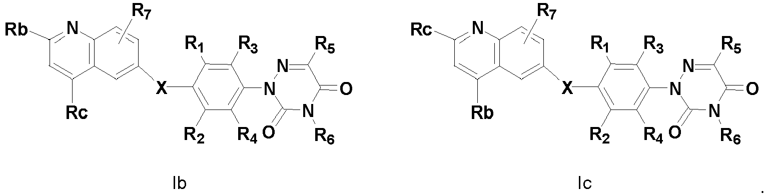

In another preferred embodiment, R₁, R₂, R₃, and R₄ are independently selected from H, D, halogen, and C1-C4 alkyl.

In another preferred embodiment, R₃ and R₄ are independently selected from H, and D; and R₁ and R₂ are independently selected from H, D, halogen, and C1-C4 alkyl.

In another preferred embodiment, R₃ and R₄ are independently H; and R₁ and R₂ are independently halogen.

In another preferred embodiment, R₆ is selected from H, deuterium, C1-C6 alkyl, C1-C6 haloalkyl, C1-C6 deuterated alkyl, and hydroxyl substituted C1-C6 alkyl.

In another preferred embodiment, R₆ is selected from the H, deuterium, C1-C6 alkyl, and C1-C6 deuterated alkyl.

In another preferred embodiment, R₆ is selected from H, deuterium, and C1-C6 alkyl.

In another preferred embodiment, R₆ is selected from H, and deuterium.

In another preferred example, R₁ₐ, R_{1b}, and R_{1c} are independently selected from H, deuterium, C1-C6 alkyl, C3-C8 cycloalkyl, and 3-8-membered heterocycloalkyl, wherein, the alkyl, cycloalkyl and heterocycloalkyl are optionally substituted by one or more deuterium, halogen.

In another preferred example, R₁ₐ, R_{1b}, and R_{1c} are independently selected from H, and C1-C6 alkyl, wherein, the alkyl is optionally substituted by one or more deuterium, halogen; preferably, substituted by one or more deuterium.

In another preferred embodiment, R₅ is selected from the group consisting of: H, D, halogen, -CN, and NHR_{1b}; wherein, R_{1b} is selected from H, D, C1-C6 alkyl, C2-C6 alkenyl, and C2-C6 alkynyl; R₆ is H or D.

In another preferred embodiment, R₅ is selected from H, D, halogen, and -CN.

In another preferred embodiment, R₅ is selected from D, and halogen.

In another preferred embodiment, R₅ is -CN.

In another preferred embodiment, each R₇ is independently H.

In the second aspect of the present invention, provided is a compound shown in formula (II), or a stereoisomer, a deuterated compound, a solvate, a metabolite, a pharmaceutically acceptable salt, a eutectic or a prodrug thereof, 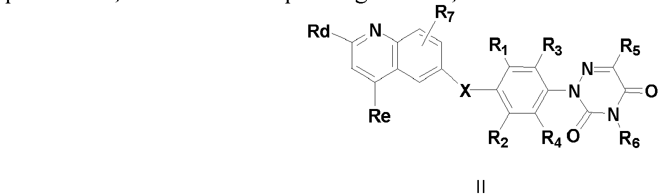 wherein,
Rd and Re are independently selected from the group consisting of: H, halogen, C1-C6 alkyl, and C1-C6 haloalkyl; and at least one of Rd and Re is halogen;
R₇ is one or more (such as 1, 2, 3, or 4) substituents located on the benzene of the quinoline ring and is selected from the group consisting of: H, halogen, C1-C6 alkyl, and C1-C6 haloalkyl;
X is selected from the group consisting of: -O-, -CH₂-, -C(=O)-, -CH(OH)-, and -S-;
R₁, R₂, R₃ and R₄ are independently selected from the group consisting of: H, D, F, Cl, Br, I, -CN, -OH, -NO₂, -OR₁ₐ, -NR_{1b}R_{1c}, -SR_{1b}, C1-C6 alkyl, C1-C6 alkoxy, C2-C6 alkenyl, C2-C6 alkynyl, C3-C8 cycloalkyl, 3-8-membered heterocycloalkyl, C5-C6 aryl, and 5-6-membered heteroaryl;
R₅ is selected from the group consisting of: H, D, F, Cl, Br, I, -CN, -OH, -NO₂, -OR₁ₐ, -NR_{1b}R_{1c}, -SR_{1b}, -S(O)R₁ₐ, -S(O)₂R₁ₐ, -S(O)NR₁ₐR_{1b}, -S(O)₂NR₁ₐR_{1b}, -NHS(O)₂R₁ₐ, -C(O)R₁ₐ, -OC(O)R₁ₐ, -C(O)OR_{1b}, -C(O)NR_{1b}R_{1c}, -NHC(O)R₁ₐ, C1-C6 alkyl, C1-C6 alkoxy, C2-C6 alkenyl, C2-C6 alkynyl, C3-C8 cycloalkyl, 3-8-membered heterocyclyl, C5-C6 aryl and 5-6-membered heteroaryl;
R₆ is selected from the group consisting of: H, D, C1-C6 alkyl, amino substituted C1-C6 alkyl, C3-C8 cycloalkyl, 3-8-membered heterocyclyl, -SR_{1b}, -S(O)R₁ₐ, -S(O)₂R₁ₐ, -S(O)NR₁ₐR_{1b}, -C(O)R₁ₐ, -C(O)OR_{1b}, and -C(O)NR_{1b}R_{1c};
unless otherwise specified, each alkyl, alkoxy, alkenyl, alkynyl, cycloalkyl, heterocyclyl, aryl, and heteroaryl mentioned above may optionally substitued by 1-3 substituents selected from the group consisting of: D, halogen, -CN, -OH, oxo-, -OR₁ₐ, -NR_{1b}R_{1c}, -SR_{1b}, -S(O)R₁ₐ, -S(O)₂R₁ₐ, -S(O)NR₁ₐR_{1b}, -S(O)₂NR₁ₐR_{1b}, -NHS(O)₂R₁ₐ, -C(O)R₁ₐ, - OC(O)R₁ₐ, -C(O)OR_{1b}, - C(O)NR_{1b}R_{1c}, -NHC(O)R₁ₐ, C1-C6 alkyl, and C1-C6 haloalkyl;
R₁ₐ, R_{1b} and R_{1c} are each independently selected from: H, D, C1-C6 alkyl, C2-C6 alkenyl, C2-C6 alkynyl, C3-C8 cycloalkyl, 3-8-membered heterocycloalkyl, C5-C6 aryl and 5-6-membered heteroaryl.

In another preferred embodiment, R₁, R₂, R₃, and R₄ are independently selected from H, D, halogen, and C1-C4 alkyl.

In another preferred embodiment, R₃ and R₄ are independently selected from H, and D; and R₁ and R₂ are independently selected from H, D, halogen, and C1-C4 alkyl.

In another preferred embodiment, R₃ and R₄ are independently H; and R₁ and R₂ are independently halogen.

In another preferred embodiment, R₆ is selected from H, deuterium, C1-C6 alkyl, C1-C6 haloalkyl, C1-C6 deuterated alkyl, and hydroxyl substituted C1-C6 alkyl.

In another preferred embodiment, R₆ is selected from the H, deuterium, C1-C6 alkyl, and C1-C6 deuterated alkyl.

In another preferred embodiment, R₆ is selected from H, deuterium, and C1-C6 alkyl.

In another preferred embodiment, R₆ is selected from H, and deuterium.

In another preferred example, R₁ₐ, R_{1b}, and R_{1c} are independently selected from H, deuterium, C1-C6 alkyl, C3-C8 cycloalkyl, and 3-8-membered heterocycloalkyl, wherein, the alkyl, cycloalkyl and heterocycloalkyl are optionally substituted by one or more deuterium, halogen.

In another preferred example, R₁ₐ, R_{1b}, and R_{1c} are independently selected from H, and C1-C6 alkyl, wherein, the alkyl is optionally substituted by one or more deuterium, halogen; preferably, substituted by one or more deuterium.

In another preferred embodiment, R₅ is selected from the group consisting of: H, D, halogen, -CN, and NHR_{1b}; wherein, R_{1b} is selected from H, D, C1-C6 alkyl, C2-C6 alkenyl, and C2-C6 alkynyl; R₆ is H or D.

In another preferred embodiment, R₅ is selected from H, D, halogen, and -CN.

In another preferred embodiment, R₅ is selected from D, and halogen.

In another preferred embodiment, R₅ is -CN.

In another preferred embodiment, each R₇ is independently H.

In the third aspect of the present invention, provided is a pharmaceutical composition, comprising the compound described in the first aspect or the second aspect of the present invention, or a stereoisomer, a deuterated compound, a solvate, a metabolite, a pharmaceutically acceptable salt, a eutectic or a prodrug thereof; and pharmaceutically acceptable excipients and/or carriers.

In the fourth aspect of the present invention, provided is a use of the compound as described in the first aspect or second aspect of the present invention, or a stereoisomer, a deuterated compound, a solvate, a metabolite, a pharmaceutically acceptable salt, a eutectic or a prodrug thereof in the preparation of a medication, wherein, the medication is used for agonizing thyroid hormone receptors, or for preventing, treating or alleviating diseases mediated by thyroid hormone receptors.

In another preferred embodiment, the diseases mediated by thyroid hormone receptors are selected from the group consisting of: non-alcoholic fatty liver disease, atherosclerosis, coronary heart disease, hypertension, hypercholesterolemia, hyperlipidemia, hypertriglyceridemia, dyslipidemia, obesity, diabetes, metabolic disorder, disorder of lipid metabolism, type 1A glycogen storage disease, hypothyroidism, and thyroid cancer.

The present invention relates to a method of using the compound or pharmaceutical composition of the present invention for agonizing thyroid hormone receptors, or for preventing, treating, or alleviating diseases mediated by thyroid hormone receptors, wherein the method is to administer a therapeutically effective amount of the compound or pharmaceutical composition to a individual in need thereof. Moreover, the above-mentioned compound or the pharmaceutical composition thereof provided by the present invention can be used in conjunction with other therapies or therapeutic agents. The usage method can be simultaneous, sequential, or at regular intervals.

It should be understood that, within the scope of the present invention, each of the above technical features of the present invention and each of the technical features specifically described in the following (such as the embodiments) can be combined with each other to constitute a new or preferred technical solution. Due to space limitations, it will not be repeated herein.

### DESCRIPTION OF THE DRAWINGS

Figure 1 shows the THRβ agonism profile of the compound in *in vitro* evaluation system;
Figure 2 shows the expression results of THRβ downstream gene in liver tissues;
Figure 3 shows the expression results of THR β downstream genes in cardiac tissue;
Figure 4 shows the results of chronic administration of TC in mice;
Figure 5 shows the serum TC/TGL/DL-C results of acute administration in mesocricetus auratus;
Figure 6 shows the expression results of DIO1 gene in mouse liver after the administration of compound 3;
Figure 7 shows the expression results of MHCα in mouse heart after the administration of compound 3;
Figure 8 shows the effect of compound 3 on thyroid hormone levels after administration.

### EMBODIMENTS FOR CARRYING OUT THE INVENTION

After long and intensive research, the inventors unexpectedly discovered a class of quinoline compounds represented by formula I and preparation method therefor. The compound can be used to prepare medications for treating diseases mediated by thyroid hormone receptors, the related diseases include, but are not limited to, diseases such as non-alcoholic fatty liver diseases, atherosclerosis, coronary heart disease, hypertension, hypercholesterolemia, hyperlipidemia, hypertriglyceridemia, dyslipidemia, obesity, diabetes, metabolic disorders, lipid metabolism disorders, type 1A glycogen storage diseases, hypothyroidism and thyroid cancer. Based on the above discoveries, the inventors have completed the present.

### TERMS

Unless otherwise specified in the present invention, the terms of the present invention have the following meanings:
As used herein, "halogen" means F, Cl, Br, I, or isotopes thereof.

"Halo" or "halogen-substituted" means being substituted by one or more halogen selected from F, C1, Br, I, or their isotopes, with an upper limit on the number of halogen substituents equal to the sum of the number of hydrogens that can be substituted in the substituted group, unless special limitation, the number of halogen substituents is any integer between 1 and the upper limit, and when the number of halogen substituents is greater than 1, the substitution can be performed by the same or different halogens.

"Alkyl" means a monovalent straight or branched saturated aliphatic hydrocarbon group, non-limitingly includes methyl, ethyl, n-propyl, isopropyl, n-butyl, sec-butyl, neo-butyl, tert-butyl, n-pentyl, iso-pentyl, neo-pentyl, n-hexyl, and various branched isomers thereof; alkyl may be further substituted with any substituent.

"Deuterated" means the hydrogen atoms on alkyl, cycloalkyl, alkylene, aryl, heteroaryl, alkenyl, alkynyl and the like substituted by at least one isotope of deuterium, and the upper limit of the number of deuterium is equal to the sum of the number of hydrogens that can be substituted in the substituted group, unless special limitation, the number of deuterium substitutions is any integer between 1 and the upper limit, preferably with 1-20 deuterium substitutions, more preferably with 1-10 deuterium substitutions, more preferably with 1-6 deuterium substitutions, and further preferably with 1-3 deuterium substitutions.

"Alkynyl" means a straight or branched monovalent unsaturated hydrocarbon group containing more than one triple bond, unless otherwise specified, alkynyl contains 2-6 carbon atoms, preferably 2-4 carbon atoms, and non-limiting example is ethynyl, propynyl, propargyl, and the like.

"Alkenyl" means a straight or branched monovalent unsaturated hydrocarbon group containing more than one carbon-carbon double bond, and unless otherwise specified, alkynyl contains 2-6 carbon atoms, preferably 2-4 carbon atoms, and non-limiting example is vinyl, propenyl, allyl, 2-butenyl, 1-butenyl, and the like.

"Alkoxy" or "Alkyloxy" means -O-alkyl. Non-limiting example includes, methoxy, ethoxy, n-propoxy, isopropoxy, n-butoxy, sec-butoxy, tert-butoxy, n-pentyloxy, n-hexyloxy, cyclopropoxy, cyclobutoxy and the like.

"Haloalkoxy" refers to an alkoxy substituted with one or more halogens. Non-limiting examples include monofluoromethoxy, difluoromethoxy, trifluoromethoxy, difluoroethoxy, and the like.

"Alkylamino" or "alkyl-amino" means an amino group substituted with one or two alkyl groups, also written as -N-(alkyl)₂ or -NH-alkyl, and the latter also written as monoalkylamino. Non-limiting examples include dimethylamino, monomethylamino, diethylamino, monoethylamino, and the like.

"Optional" or "optionally" means that the event or circumstance subsequently described may, but does not necessarily, occur, and the description includes occasions when the event or circumstance does or does not occur. For example, "alkyl optionally substituted with F" means that it may, but does not necessarily, be substituted with F, and the description includes occasions when it is substituted with F and it is not.

"Pharmaceutically acceptable salt" means a salt formed of a free acid compound of the present invention with non-toxic inorganic bases or organic bases, or a salt formed of a free base compound of the present invention with non-toxic inorganic acid or organic base, while the compound maintains the biological effectiveness and properties of the free acid or the free base.

"Pharmaceutical composition" means a mixture of one or more of the compounds described herein, or a stereoisomer thereof, a solvate, a pharmaceutically acceptable salt or a eutectic thereof and other components, wherein the other components include physiologically/pharmacologically acceptable carriers and/excipients.

"Carrier" means a system that does not cause significant irritation to an organism and does not eliminate the biological activity and property of the administered compound, and modifies the mode of entry and distribution of the drug into the body, controls the rate of release of the drug, and delivers the drug to the target organ, with non-limiting examples including microcapsules and microspheres, nanoparticles, liposomes, etc.

"Excipient" means an agent which is not itself a therapeutic agent and which is used as diluent, accessories, binder and/or vehicle to be added to a pharmaceutical composition in order to improve its disposition or storage properties or to permit or facilitate the formation of the compound or pharmaceutical composition into a unitary dosage form for the administration of a drug. As known to those of skill in the art, pharmaceutical excipients may provide a variety of functions and may be described as wetting agents, buffering agents, suspending agents, lubricants, emulsifiers, disintegrants, absorbents, preservatives, surfactants, colorants, flavoring agents, and sweeteners. Examples of pharmaceutical excipient include, but are not limited to:(1) sugars, such as lactose, dextrose, and sucrose; (2) starches, such as corn starch and potato starch; (3) cellulose and derivatives thereof, such as sodium carboxymethylcellulose, ethylcellulose, cellulose acetate, hydroxypropylmethyl cellulose, hydroxypropyl cellulose, microcrystalline cellulose, and crosslinked carboxymethyl cellulose (e.g., crosslinked sodium carboxymethyl cellulose); (4) tcragacanth gum powder; (5) malt; (6) gelatin; (7) talc; (8) excipients, e.g., cocoa butter and suppository waxes; (9) oils, e.g., peanuct oil, cottonseed oil, safflower oil, sesame oil, olive oil, corn oil, and soybean oil; (10) diols, e.g., propylene glycol; (11) polyols, e.g., glycerol, sorbitol, mannitol, and polyethylene glycol; (12) esters, e.g., ethyl oleate and ethyl lauate; (13) agar; (14)

buffers, e.g., magnesium hydroxide and aluminum hydroxide; (15) alginic acid; (16) nonpyrogenic water; (17) isotonic saline; (18) Ringer's solution; (19) ethanol; (20) pH buffer solution; (21)polyester, polycarbonate and/or polyanhydride; and (22) other non-toxic compatible substances used in pharmaceutical formulations.

"Stereoisomer" are isomers resulting from different spatial arrangements of atoms in a molecule, including cis-trans isomers, enantiomers and conformational isomers.

"Solvate" means substances formed by the compounds of the invention or salts thereof with stoichiometric or non-stoichiometric solvents bound by intermolecular non-covalent forces. When the solvent is water, it is a hydrate.

"Eutectic" refers to the crystal formed by the combination of active pharmaceutical ingredients (API) and cocrystal former (CCF) through hydrogen bonding or other non-covalent bonds, wherein the pure states of API and CCF are both solid at room temperature and there is a fixed stoichiometric ratio between the components. Eutectic is a multi-component crystal containing both binary eutectic formed between two neutral solids and multi-component eutectic formed between a neutral solid and a salt or a solvate.

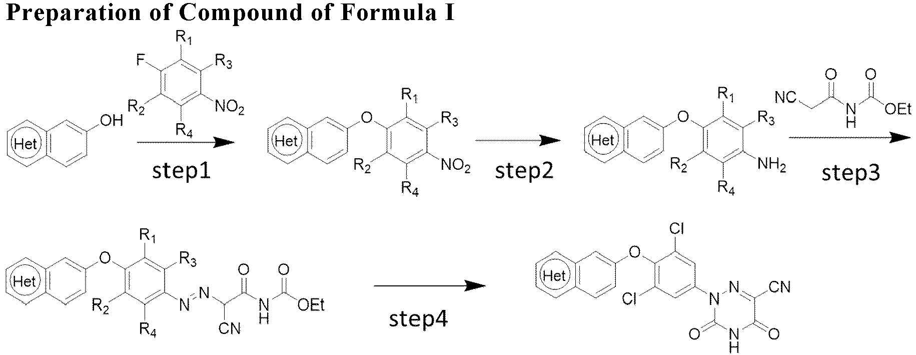

The compounds of the present invention can all be prepared using the route shown above, using the corresponding 6-methoxyquinoline compounds or directly purchasing the commercially available 6-hydroxyquinoline compounds as starting materials, and stirring with potassium carbonate or cesium carbonate with the corresponding p-fluorosubstituted nitrobenzene compounds in DMF for several hours to obtain the raw material of step 2: subsequently, conducting nitro reduction under suitable conditions to obtain the corresponding amino compounds: diazotizing the resulting amino compounds in hydrochloric acid and sodium nitrite and then coupling with N-cyanoacetylurethane: finally, refluxing the compound in acetic acid and sodium acetate at elevated temperatures to obtain the respective embodiments.

Those skilled in the art can prepare the compounds of the present invention in conjunction with the literature as well as known organic synthesis techniques. The starting materials are commercially available and/or compounds described in chemical literature. "Commercial compounds" are obtained from formal commercial sources, including companies such as Bide Pharmaceuticals, Haohong Biotechnology, Titan Technology, Sinopharm, WuXi AppTec, J&k scientific, and Accela ChemBio Co., Ltd.. The testing methods include nuclear magnetic resonance (NMR) and mass spectrometry (MS).

### Pharmaceutical compositions and preparation thereof

Another aspect of the present invention provides a pharmaceutical composition containing a therapeutically effective amount of one or more compounds selected from the above formula I, or pharmaceutically acceptable salts, enantiomers, diastereomers or racemates thereof, and optionally, one or more pharmaceutically acceptable carriers, excipients, adjuvants, accessories, and/or diluents. The ingredients are, for example, odorants, flavors, sweeteners and the like.

The pharmaceutical compositions provided by the invention preferably contains the active ingredient in a weight ratio of 1-99%. Its preferred ratio was that the compounds of the general formula I as active ingredient accounts for 65 wt% to 99 wt% of the total weight, and the remainder are pharmaceutically acceptable carriers, diluents or solutions or salt solutions.

The compounds and pharmaceutical compositions provided by the invention can be in various forms, such as tablets, capsules, powders, syrups, solutions, suspensions, and aerosols, etc, and can be stocked in a suitable solid or liquid carriers or diluents and in suitable sterilized device for injection or drip infusion.

Various dosage forms of the pharmaceutical compositions of the invention can be prepared according to the conventional preparation methods in the pharmaceutical field. A unit dose of the formulation contains 1 mg-700 mg of the compound of formula I. Preferably, the unit dose of the formulation contains 25 mg - 300 mg of the compound of formula I.

The compounds and pharmaceutical compositions of the invention can be used clinically in mammals, including humans and animals and can be administered by mouth, nose, skin, lung, or gastrointestinal tract. Most preferred was oral. Most preferred daily dose is of 50-1400 mg/kg body weight, one-time administration, or 25-700 mg/kg body weight in divided doses. Regardless of any method of administration, the optimal dosage for an individual should be determined depend on the specific treatment. Normally, start with a small dose and gradually increase the dose until the most suitable dose is found.

The present invention was further described hereafter in combination with specific examples. It should be understood that these examples are only used to illustrate the and not to limit the scope of the invention. The experimental methods without specific conditions in the following examples generally follow the conventional conditions or the conditions suggested by the manufacturer. Unless otherwise stated, percentages and parts are caculated by weight.

### Example 1: 2- (3,5-Dichloro-4- ((2- (2-fluoropyridin-4-yl) -4-methylquinolin-6-yl) oxy) phenyl) -3,5-dioxo-2,3,4,5-tetrahydro-1,2,4-triazine-6-carbonitrile

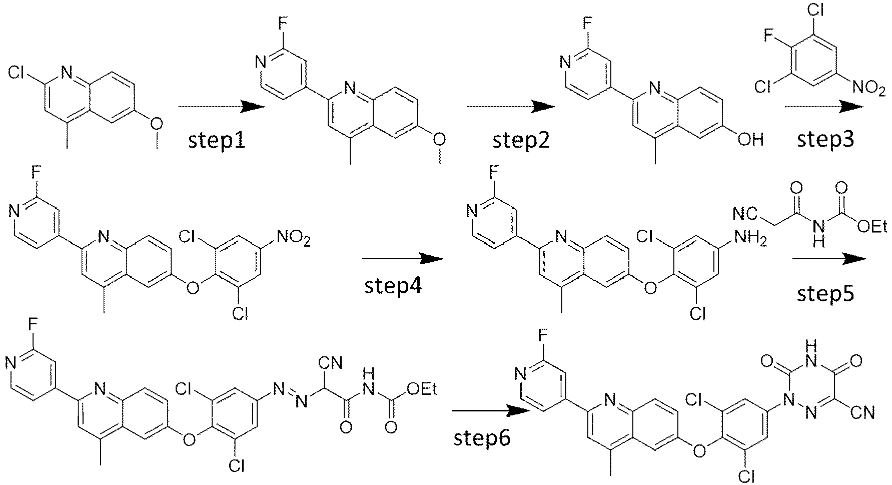

### Step 1: Preparation of 6-methoxy-4-methyl-2- (4-fluoropyridin) quinoline

A mixture of a commercially available raw material 6-methoxy-4-methyl-2-chloroquinoline (2g, 1eq), a catalytic amount of tetrakis(triphenylphosphine)palladium, and excess 2-fluoropyridin-4-borate and excess sodium carbonate in a mixed solution of 20 ml of dioxane and 5 ml of water was reacted overnight under nitrogen protection at 100°C. After the reaction was finished, the reaction solution was diluted with ethyl acetate, filtered to remove solids, and the organic phase was washed three times with saturated saline, dried over anhydrous sodium sulfate, filtered, concentrated under reduced pressure, and then subjected to column chromatography to obtain white powder 6-methoxy-4-methyl-2-phenylquinoline.

### Step 2: Preparation of 6-hydroxy-4-methyl-2- (4-fluoropyridin) quinoline

6-Methoxy-4-methyl-4-(2-fluoropyridin)quinoline was placed in 20 ml of 48% aqueous hydrobromic acid solution and reacted at 90 °C for three days. After the reaction was finished, the resulting solution was adjusted with aqueous sodium bicarbonate to pH 8-9, and extracted with ethyl acetate three times. The combined organic phase was washed with saturated saline, dried over anhydrous sodium sulfate, filtered and concentrated under reduced pressure to obtain a yellow oily compound 6-hydroxy-4-methyl-2-(4-fluoropyridin)quinoline, which was used directly in the next step without purification.

### Step 3: Preparation of 6- (2,6-dichloro-4-nitrophenoxy) -4-methyl-4- (2-fluoropyridin) quinoline

To a solution of 6-hydroxy-4-methyl-2-(4-fluoropyridin)quinoline and 3,5-dichloro-p-fluoronitrobenzene (1.48 g, 0.0070 mol) in 20 ml of DMF was added 5 g of potassium carbonate and stirred for 6 hr at ambient temperature. Then the reaction was quenched by adding 200 ml of water, extracted three times with ethyl acetate, and the organic phases were combined and washed three times with saturated sodium chloride solution. The organic phase was dried over anhydrous sodium sulfate, filtered, concentrated under reduced pressure, and then purified by column chromatography to obtain a light yellow compound 6-(2,6-dichloro-4-nitrophenoxy) -4-methyl-4- (2-fluoropyridin) quinoline.

### Step 4: Preparation of 3,5-dichloro-4- ((4-methyl-2- (4-fluoropyridin) quinolin-6-yl) oxy) aniline

To a solution of 6-(2,6-dichloro-4-nitrophenoxy)-4-methyl-2-phenylquinoline in 15 ml of ethyl acetate and 15 ml of ethanol was added excess stannous chloride and a catalytic amount of hydrochloric acid, and stirred overnight at ambient temperature until the reaction was complete. The reaction solution was adjusted with aqueous sodium carbonate to pH 9-10, filtered to remove the precipitate, and extracted three times with ethyl acetate. The combined organic phase was washed three times with saturated saline, dried over anhydrous sodium sulfate, filtered and concentrated under reduced pressure to obtain a light solid 3,5-dichloro-4- ((4-methyl-2- (4-fluoropyridin) quinolin-6-yl) oxy) aniline

### Step 5: Preparation of ethyl (E) - (2-cyano-2- ((3,5-dichloro-4- ((4-methyl-2-(4-fluoropyridin) quinoline-6-yl) oxy) phenyl) dienyl) acetyl) carbamate

To a flask containing 3,5-dichloro-4- ((4-methyl-2- (4-fluoropyridin) quinolin-6-yl) oxy) aniline was added 10 ml of water, as well as 10 ml of hydrochloric acid, and 3 ml of an aqueous solution of sodium nitrite was added dropwise at 0°C. To another round-bottomed flask was added 15 ml of pyridine and 10 ml of water, and N-cyanoacetylurethane (483.93 mg, 0.0031 mol). Both phases were held at 0°C for 30 min; then the diazonium salt was added dropwise to the other phase, after addition, the temperature was held for 30 min, then the mixture was extracted with ethyl acetate, and the organic phase was washed with saline, dried and concentrated under reduced pressure to obtain a red solid ethyl(E) - (2-cyano-2-((3,5-dichloro-4- ((4-methyl-2- (4-fluoropyridin) quinoline-6-yl) oxy) phenyl) dienyl) acetyl) carbamate (1.2 g, 83.8%), and the crude product was used directly in the next step without purification.

### Step 6: Preparation of 2- (3,5-Dichloro-4- ((2- trifluoromethyl-2- (4-fluoropyridin) quinolin-6-yl) oxy) phenyl) -3,5-dioxo-2,3,4,5-tetrahydro-1,2,4-triazine-6-carbonitrile

To a solution of orange ethyl (E)-(2-cyano-2-((3,5-dichloro-4-((4-methyl-2-(4-fluoropyridin)quinolin-6-yl)oxy)phenyl)die nyl)acetyl)carbamate (1.2 g, 83.8%) in 10 ml of acetic acid was added 1 g of sodium acetate, and heated for 2 hr. at 120°C. The reaction was added with water to precipitate crude red compound, which was then filtered and dried, and then purified by high performance preparative liquid phase separation to obtain white 2- (3,5-Dichloro-4- ((2-(2-fluoropyridin-4-yl) -4-methylquinolin-6-yl) oxy) phenyl) -3,5-dioxo-2,3,4,5-tetrahydro-1,2,4-triazine-6-carbonitrile. 1H NMR (500 MHz, Chloroform) δ 9.55 (s, 1H), 8.91 (s, 1H), 8.58 (s, 1H), 7.97 (d, J = 26.8 Hz, 2H), 7.60 (d, J = 15.0 Hz, 3H), 7.47 (s, 1H), 6.97 (s, 1H), 2.69 (s, 3H).

### Example 2: 2- (3,5-Dichloro-4-((2-phenyl-4-methylquinolin-6-yl) oxy) phenyl) -3,5-dioxo-2,3,4,5-tetrahydro- 1,2,4-triazine-6-carbonitrile

By using phenylborate ester, the compound was prepared, separated and purified by referring to the method in example 1(total yield 22%) M/Z = 534.02(M+1) 1H NMR (500 MHz, Chloroform ) δ 9.56 (s, 1H), 8.33 (s, 2H), 7.92 (s, 1H), 7.67 (s, 1H), 7.57 (d, J = 15.0 Hz, 4H), 7.49 (s, 2H), 6.63 (s, 1H), 2.69 (s, 3H).

### Example 3: 2- (3,5-Dichloro-4- ((2- (4-fluorophenyl) -4-methylquinolin-6-yl) oxy) phenyl) -3,5-dioxo-2,3,4,5-tetrahydro- 1,2,4-triazine-6-carbonitrile

By using 4-fluorophenylborate ester, the compound was prepared, separated and purified by referring to the method in example 1(total yield 22%) M/Z = 534.02(M+1) 1H NMR (500 MHz, Chloroform ) δ 9.53 (s, 2H), 8.25 (s, 4H), 7.92 (s, 2H), 7.67 (s, 2H), 7.58 (s, 4H), 7.49 (s, 3H), 7.31 (s, 3H), 6.63 (s, 2H), 2.69 (s, 6H).

### Example 4: 2- (3,5-Dichloro-4-((2- (4-trifluoromethylphenyl)-4-methylquinolin-6-yl) oxy) phenyl) -3,5-dioxo-2,3,4,5-tetrahydro- 1,2,4-triazine-6-carbonitrile

By using 4-trifluoromethylphenylborate ester, the compound was prepared, separated and purified by referring to the method in example 1(total yield 18%) M/Z =538.04(M+1) 1H NMR (500 MHz, Chloroform) δ 9.57 (s, 10H), 7.94 (d, J = 20.0 Hz, 31H), 7.74 (d, J = 1.8 Hz, 2H), 7.74 - 7.50 (m, 55H), 7.58 (s, 26H), 7.58 (s, 20H), 7.49 (s, 10H), 6.63 (s, 10H), 2.69 (s, 30H).

### Example 5: 2- (3,5-Dichloro-4- ((2- (4-chlorophenyl) -4-methylquinolin-6-yl) oxy) phenyl) -3,5-dioxo-2,3,4,5-tetrahydro- 1,2,4-triazine-6-carbonitrile

By using 4-chlorophenylborate ester, the compound was prepared, separated and purified by referring to the method in example 1(total yield 15%) M/Z = 550.02+1 1H NMR (500 MHz, Chloroform) δ 9.53 (s, 1H), 8.36 (s, 2H), 7.92 (s, 1H), 7.67 (s, 1H), 7.59 (d, J = 10.0 Hz, 4H), 7.49 (s, 1H), 6.63 (s, 1H), 2.69 (s, 3H).

### Example 6: 2- (3,5-Dichloro-4- ((2- (4-bromophenyl) -4-methylquinolin-6-yl) oxy) phenyl) -3,5-dioxo-2,3,4,5-tetrahydro- 1,2,4-triazine-6-carbonitrile

By using 4-bromophenylborate ester, the compound was prepared, separated and purified by referring to the method in example 1(total yield 23%) M/Z = 593.97(M+1) 1H NMR (500 MHz, Chloroform) δ 9.53 (s, 1H), 8.17 (s, 2H), 7.92 (s, 1H), 7.67 (s, 1H), 7.57 (d, J = 15.0 Hz, 4H), 7.49 (s, 2H), 6.63 (s, 1H), 2.69 (s, 3H).

### Example 7: 2- (3,5-Dichloro-4- ((2- (4- cyanophenyl) -4-methylquinolin-6-yl) oxy) phenyl) -3,5-dioxo-2,3,4,5-tetrahydro- 1,2,4-triazine-6-carbonitrile

By using 4-cyanophenylborate ester, the compound was prepared, separated and purified by referring to the method in example 1(total yield 15%) M/Z = 541.05(M+1) 1H NMR (500 MHz, Chloroform) δ 9.57 (s, 1H), 8.34 (s, 2H), 7.91 (d, J = 5.0 Hz, 3H), 7.67 (s, 1H), 7.58 (s, 2H), 7.49 (s, 1H), 6.63 (s, 1H), 2.69 (s, 3H).

### Example 8: 2- (3,5-Dichloro-4-((2-(2-isopropylphenyl)-4-methylquinolin-6-yl) oxy) phenyl) -3,5-dioxo-2,3,4,5-tetrahydro- 1,2,4-triazine-6-carbonitrile

By using 2- (3-chloro-5-fluorophenyl) -4,4,5,5-tetramethyl-1,3,2-dioxaborolane, the compound was prepared, separated and purified by referring to the method in example 1(total yield 17%) M/Z =568.01(M+1) 1H NMR (500 MHz, Chloroform ) δ 9.56 (s, 16H), 8.13 (s, 16H), 7.92 (s, 18H), 7.88 (s, 13H), 7.73 (d, J = 60.0 Hz, 32H), 7.59 (s, 1H), 7.58 (s, 32H), 7.49 (s, 16H), 6.63 (s, 16H), 2.69 (s, 48H).

### Example 9: 2- (3,5-Dichloro-4-((2-(2-isopropylphenyl)-4-methylquinolin-6-yl) oxy) phenyl) -3,5-dioxo-2,3,4,5-tetrahydro- 1,2,4-triazine-6-carbonitrile

By using 2- (2-isopropylphenyl) -4,4,5,5-tetramethyl-1,3,2-dioxaborolane, the compound was prepared, separated and purified by referring to the method in example 1(total yield 16%) M/Z = 558.1(M+1) ¹H NMR (500 MHz, Chloroform ) δ 9.54 (s, 6H), 8.02 (s, 6H), 7.92 (s, 6H), 7.67 (s, 6H), 7.61 - 7.47 (m, 23H), 7.47 (s, 1H), 7.39 (s, 6H), 7.27 (s, 4H), 6.63 (s, 6H), 2.87 (s, 2H), 2.69 (s, 18H), 1.17 (s, 37H).

### Example 10: 2- (3,5-Dichloro-4- ((2- (4-fluoro-3-methylphenyl) -4-methylquinolin-6-yl) oxy) phenyl) -3,5-dioxo-2,3,4,5-tetrahydro -1,2,4-triazine-6-carbonitrile

By using 2- (4-fluoro-3-methylphenyl) -4,4,5,5-tetramethyl-1,3,2-dioxaborolane, the compound was prepared, separated and purified by referring to the method in example 1 (total yield 17%) M/Z = 548.06(M+1) ¹H NMR (500 MHz, Chloroform) δ 9.57 (s, 1H), 8.34 (s, 2H), 7.91 (d, *J* = 5.0 Hz, 3H), 7.67 (s, 1H), 7.58 (s, 2H), 7.49 (s, 1H), 6.63 (s, 1H).

### Example 11: 2- (3,5-Dichloro-4- ((2- (4-cyano-3-fluorophenyl) -4-methylquinolin-6-yl) oxy) phenyl) -3,5-dioxo-2,3,4,5-tetrahydro--1,2,4-triazine-6-carbonitrile

By using 2-fluoro-4- (4,4,5,5-tetramethyl-1,3,2-dioxaborolan-2-yl) benzonitrile, the compound was prepared, separated and purified by referring to the method in example 1(total yield 13%) M/Z = 559.04(M+1) ¹H NMR (500 MHz, Chloroform) δ 9.57 (s, 1H), 8.34 (s, 2H), 7.91 (d, *J* = 5.0 Hz, 3H), 7.67 (s, 1H), 7.58 (s, 2H), 7.49 (s, 1H), 6.63 (s, 1H).

### Example 12: 2- (3,5-Dichloro-4- ((2- (2-cyano-4-fluorophenyl) -4-methylquinolin-6-yl) oxy) phenyl) -3,5-dioxo-2,3,4,5-tetrahydro--1,2,4-triazine-6-carbonitrile

By using 4-trifluoromethyl-4- (4,4,5,5-tetramethyl-1,3,2-dioxaborolan-2-yl) benzonitrile, the compouncd was prepared, separated and purified by referring to the method in example 1 (total yield 18%) M/Z = 609.04(M+1) ¹H NMR (500 MHz, Chloroform) δ 9.57 (s, 1H), 8.34 (s, 2H), 7.91 (d, *J* = 5.0 Hz, 3H), 7.67 (s, 1H), 7.58 (s, 2H), 7.49 (s, 1H), 6.63 (s, 1H).

### Example 13: 2- (3,5-Dichloro-4- ((2- (4-fluoro-3,5-dichlorophenyl) -4-methylquinolin-6-yl) oxy) phenyl) -3,5-dioxo-2,3,4,5-tetrahydro -1,2,4-triazine-6-carbonitrile

By using 2- (3,5-dichloro-4-fluorophenyl) -4,4,5,5-tetramethyl-1,3,2-dioxaborolane, the compound was prepared, separated and purified by referring to the method in example 1 (total yield 22%)M/Z = 601.97(M+1) ¹H NMR (500 MHz, Chloroform ) δ 9.58 (s, 1H), 8.22 (s, 2H), 7.92 (s, 1H), 7.67 (s, 1H), 7.58 (s, 2H), 7.49 (s, 1H), 6.63 (s, 1H), 2.69 (s, 3H).

### Example 14: 2- (3,5-Dichloro-4- ((2- (4- chloro -3,5-methylphenyl) -4-methylquinolin-6-yl) oxy) phenyl) -3,5-dioxo-2,3,4,5-tetrahydro -1,2,4-triazine-6-carbonitrile

By using 2- (3,5-dimethyl-4-chlorophenyl) -4,4,5,5-tetramethyl-1,3,2-dioxaborolane, the compound was prepared, separated and purified by referring to the method in example 1 (total yield 19%) M/Z = 578.05(M+1) ¹H NMR (500 MHz, Chloroform) δ 9.58 (s, 1H), 8.22 (s, 2H), 7.92 (s, 1H), 7.67 (s, 1H), 7.58 (s, 2H), 7.49 (s, 1H), 6.63 (s, 1H), 2.69 (s, 3H).

### Example 15: 2- (3,5-Dichloro-4-((2-(furan-2-yl)-4-methylquinolin-6-yl) oxy) phenyl) -3,5-dioxo-2,3,4,5-tetrahydro-1,2,4-triazine-6-carbonitrile

By using 2-(furan-2-yl)-4,4,5,5-tetramethyl-1,3,2-dioxaborolane, the compound was prepared, separated and purified by referring to the method in example 1 (total yield 20%) M/Z = 506.03(M+1) ¹H NMR (500 MHz, Chloroform) δ 9.58 (s, 1H), 8.22 (s, 2H), 7.92 (s, 1H), 7.67 (s, 1H), 7.58 (s, 2H), 7.49 (s, 1H), 6.63 (s, 1H), 2.69 (s, 3H).

### Example 16: 2- (3,5-Dichloro-4- ((4-methyl-2-(5-methylfuran-2-yl)quinolin-6-yl)oxy) phenyl) -3,5-dioxo-2,3,4,5-tetrahydro -1,2,4-triazine-6-carbonitrile

By using 2-(4-methylfuran-2-yl)-4,4,5,5-tetramethyl-1,3,2-dioxaborolane, the compound was prepared, separated and purified by referring to the method in example 1 (total yield 21%)M/Z = 520.05(M+1) ¹H NMR (500 MHz, Chloroform ) δ 9.56 (s, 1H), 7.94 (s, 1H), 7.60 (d, *J* = 15.0 Hz, 3H), 7.47 (s, 1H), 6.95 (d, *J* = 20.0 Hz, 2H), 5.94 (s, 1H), 2.69 (s, 3H), 2.25 (s, 3H).

### Example 17: 2- (3,5-Dichloro-4-((4-methyl-2-(5-cyclopropylfuran-2-yl)quinolin-6-yl)oxy) phenyl) -3,5-dioxo-2,3,4,5-tetrahydro -1,2,4-triazine-6-carbonitrile

By using 2-(4-cyclopropylfuran-2-yl)-4,4,5,5-tetramethyl-1,3,2-dioxaborolane, the compound was prepared, separated and purified by referring to the method in example 1 (total yield 21%)M/Z = 520.05(M+1) ¹H NMR (500 MHz, Chloroform ) δ 9.56 (s, 1H), 7.94 (s, 1H), 7.60 (d, *J* = 15.0 Hz, 3H), 7.47 (s, 1H), 6.95 (d, *J* = 20.0 Hz, 2H), 5.94 (s, 1H), 2.69 (s, 3H), 2.25 (s, 3H).

### Example 18: 2- (3,5-Dichloro-4-((4-methyl-2-(2-cyano-5-methylfuran-3-yl)quinolin-6-yl)oxy) phenyl) -3,5-dioxo-2,3,4,5-tetrahydro -1,2,4-triazine-6-carbonitrile

By using 5-methyl-3-(4,4,5,5-tetramethyl-1,3,2-dioxoboron-2-yl)furan-2-carbonitrile, the compound was prepared, separated and purified by referring to the method in example 1 (total yield 29%)M/Z = 545.05(M+1) ¹H NMR (500 MHz, Chloroform ) δ 9.56 (s, 1H), 7.94 (s, 1H), 7.60 (d, *J* = 15.0 Hz, 3H), 7.47 (s, 1H), 6.95 (d, *J* = 20.0 Hz, 2H), 5.94 (s, 1H), 2.69 (s, 3H), 2.25 (s, 3H).

### Example 19: 2- (3,5-Dichloro-4- ((2- (thiophen-2-yl) -4-methylquinolin-6-yl) oxy) phenyl) -3,5-dioxo-2,3,4,5-tetrahydro -1,2,4-triazine-6-carbonitrile

By using 2-(furan-2-yl)-4,4,5,5-tetramethyl-1,3,2-dioxaborolane, the compound was prepared, separated and purified by referring to the method in example 1 (total yield 26%)M/Z = 522.01(M+1) ¹H NMR (500 MHz, Chloroform ) δ 9.56 (s, 1H), 7.94 (s, 1H), 7.60 (d, *J* = 15.0 Hz, 3H), 7.47 (s, 1H), 6.95 (d, *J* = 20.0 Hz, 2H), 5.94 (s, 1H), 2.69 (s, 3H), 2.25 (s, 3H).

### Example 20: 2- (3,5-Dichloro-4- ((4-methyl- 2- (4-chlorothiophen-2-yl) quinolin-6-yl) oxy) phenyl) -3,5-dioxo-2,3,4,5-tetrahydro -1,2,4-triazine-6-carbonitrile

By using 2-(4-chlorothiophen-2-yl)-4,4,5,5-tetramethyl-1,3,2-dioxaborolane, the compound was prepared, separated and purified by referring to the method in example 1 (total yield 13%)M/Z = 555.97(M+1) ¹H NMR (500 MHz, Chloroform ) δ 9.56 (s, 1H), 7.94 (s, 1H), 7.60 (d, *J* = 15.0 Hz, 3H), 7.47 (s, 1H), 6.95 (d, *J* = 20.0 Hz, 2H), 5.94 (s, 1H), 2.69 (s, 3H), 2.25 (s, 3H).

### Example 21: 2- (3,5-Dichloro-4- ((4-methyl- 2- (5- cyanothiophen-2-yl) quinolin-6-yl) oxy) phenyl) -3,5-dioxo-2,3,4,5-tetrahydro -1,2,4-triazine-6-carbonitrile

By using 5-(4,4,5,5-tetramethyl-1,3,2-dioxaborolan-2-yl)thiophen-2-carbonitrile, the compound was prepared, separated and purified by referring to the method in example 1 (total yield 15%)M/Z = 547.01(M+1) ¹H NMR (500 MHz, Chloroform ) δ 9.56 (s, 1H), 7.94 (s, 1H), 7.60 (d, *J* = 15.0 Hz, 3H), 7.47 (s, 1H), 6.95 (d, *J* = 20.0 Hz, 2H), 5.94 (s, 1H), 2.69 (s, 3H), 2.25 (s, 3H).

### Example 22: 2- (3,5-Dichloro-4-((4- methyl- 2-(5-methylthiophen-2-yl)quinolin-6-yl) oxy) phenyl) -3,5-dioxo-2,3,4,5-tetrahydro -1,2,4-triazine-6-carbonitrile

By using 2-(5-methylthienyl-2-yl)-4,4,5,5-tetramethyl-1,3,2-dioxaborolane, the compound was prepared, separated and purified by referring to the method in example 1 (total yield 39%)M/Z = 536.03(M+1) ¹H NMR (500 MHz, Chloroform ) δ 9.56 (s, 1H), 7.94 (s, 1H), 7.84 (s, 1H), 7.60 (d, *J* = 15.0 Hz, 3H), 7.47 (s, 1H), 7.02 (d, *J* = 55.0 Hz, 2H), 2.69 (s, 3H), 2.44 (s, 3H).

### Example 23: 2- (3,5-Dichloro-4-((4- methyl-2-(5-trifluoromethylthiophen-2-yl)quinolin-6-yl) oxy) phenyl) -3,5-dioxo-2,3,4,5-tetrahydro -1,2,4-triazine-6-carbonitrile

By using 2-(5-trifluoromethylthienyl-2-yl)-4,4,5,5-tetramethyl-1,3,2-dioxaborolane, the compound was prepared, separated and purified by referring to the method in example 1 (total yield 17%)M/Z = 590.00(M+1) ¹H NMR (500 MHz, Chloroform ) δ 9.56 (s, 1H), 7.94 (s, 1H), 7.84 (s, 1H), 7.60 (d, *J* = 15.0 Hz, 3H), 7.47 (s, 1H), 7.02 (d, *J* = 55.0 Hz, 2H), 2.69 (s, 3H), 2.44 (s, 3H).

### Example 24: 2- (3,5-Dichloro-4- ((2- pyridin-2-yl) -4-methylquinolin-6-yl) oxy) phenyl) -3,5-dioxo-2,3,4,5-tetrahydro-1,2,4-triazine-6-carbonitrile

By using 2-(4,4,5,5-tetramethyl-1,3,2-dioxaborolan-2-yl)pyridine, the compound was prepared, separated and purified by referring to the method in example 1 (total yield 16%)M/Z = 517.05(M+1) ¹H NMR (500 MHz, Chloroform ) δ 9.56 (s, 1H), 7.94 (s, 1H), 7.84 (s, 1H), 7.60 (d, *J* = 15.0 Hz, 3H), 7.47 (s, 1H), 7.02 (d, *J* = 55.0 Hz, 2H), 2.69 (s, 3H), 2.44 (s, 3H).

### Example 25: 2- (3,5-Dichloro-4- ((4-methyl- 2- (5-methylpyridin-2-yl)quinolin-6-yl) oxy) phenyl) -3,5-dioxo-2,3,4,5-tetrahydro-1,2,4-triazine-6-carbonitrile

By using 5-methyl-2-(4,4,5,5-tetramethyl-1,3,2-dioxaborolan-2-yl)pyridine, the compound was prepared, separated and purified by referring to the method in example 1 (total yield 17%)M/Z = 531.07(M+1) ¹H NMR (500 MHz, Chloroform ) δ 9.56 (s, 1H), 7.94 (s, 1H), 7.84 (s, 1H), 7.60 (d, *J* = 15.0 Hz, 3H), 7.47 (s, 1H), 7.02 (d, *J* = 55.0 Hz, 2H), 2.69 (s, 3H), 2.44 (s, 3H).

### Example 26: 2- (3,5-Dichloro-4-((4- methyl-2- (5-fluoropyridin-2-yl) quinolin-6-yl) oxy) phenyl)-3,5-dioxo-2,3,4,5-tetrahydro-1,2,4-triazine -6-carbonitrile

By using 5-fluoro-2-(4,4,5,5-tetramethyl-1,3,2-dioxaborolan-2-yl)pyridine, the compound was prepared, separated and purified by referring to the method in example 1 (total yield 19%)M/Z = 535.04(M+1) ¹H NMR (500 MHz, Chloroform ) δ 9.56 (s, 1H), 7.94 (s, 1H), 7.84 (s, 1H), 7.60 (d, *J* = 15.0 Hz, 3H), 7.47 (s, 1H), 7.02 (d, *J* = 55.0 Hz, 2H), 2.69 (s, 3H), 2.44 (s, 3H).

### Example 27: 2- (3,5-Dichloro-4-((4- methyl-2- (5-chloropyridin-2-yl) quinolin-6-yl) oxy) phenyl)-3,5-dioxo-2,3,4,5-tetrahydro-1,2,4-triazine -6-carbonitrile

By using 5-chloro-2-(4,4,5,5-tetramethyl-1,3,2-dioxaborolan-2-yl)pyridine, the compound was prepared, separated and purified by referring to the method in example 1 (total yield 22%)M/Z = 551.01(M+1) ¹H NMR (500 MHz, Chloroform ) δ 9.54 (s, 1H), 8.97 (s, 1H), 8.46 (s, 1H), 7.94 (s, 1H), 7.75 (s, 1H), 7.60 (d, *J* = 15.0 Hz, 3H), 7.47 (s, 1H), 6.77 (s, 1H), 2.69 (s, 3H).

### Example 28: 2- (3,5-Dichloro-4-((4- methyl-2- (5-bromopyridin-2-yl) quinolin-6-yl) oxy) phenyl)-3,5-dioxo-2,3,4,5-tetrahydro-1,2,4-triazine -6-carbonitrile

By using 5-bromo-2-(4,4,5,5-tetramethyl-1,3,2-dioxaborolan-2-yl)pyridine, the compound was prepared, separated and purified by referring to the method in example 1 (total yield 11%)M/Z = 594.96(M+1) ¹H NMR (500 MHz, Chloroform ) δ 9.54 (s, 1H), 8.97 (s, 1H), 8.46 (s, 1H), 7.94 (s, 1H), 7.75 (s, 1H), 7.60 (d, *J* = 15.0 Hz, 3H), 7.47 (s, 1H), 6.77 (s, 1H), 2.69 (s, 3H).

### Example 29: 2- (3,5-Dichloro-4- ((2-(pyridin-3-yl) -4-methylquinolin-6-yl) oxy) phenyl) -3,5-dioxo-2,3,4,5-tetrahydro-1,2,4-triazine-6-carbonitrile

By using 3-(4,4,5,5-tetramethyl-1,3,2-dioxaborolan-2-yl)pyridine, the compound was prepared, separated and purified by referring to the method in example 1 (total yield 17%)M/Z = 517.05(M+1) ¹H NMR (500 MHz, Chloroform ) δ 9.54 (s, 1H), 8.97 (s, 1H), 8.46 (s, 1H), 7.94 (s, 1H), 7.75 (s, 1H), 7.60 (d, *J* = 15.0 Hz, 3H), 7.47 (s, 1H), 6.77 (s, 1H), 2.69 (s, 3H).

### Example 30: 2- (3,5-Dichloro-4- ((2-(6-methylpyridin-3-yl) -4-methylquinolin-6-yl) oxy) phenyl) -3,5-dioxo-2,3,4,5-tetrahydro-1,2,4-triazine-6-carbonitrile

By using 6-methyl-3-(4,4,5,5-tetramethyl-1,3,2-dioxaborolan-2-yl)pyridine, the compound was prepared, separated and purified by referring to the method in example 1 (total yield 14%)M/Z = 531.07(M+1) ¹H NMR (500 MHz, Chloroform ) δ 9.54 (s, 1H), 8.97 (s, 1H), 8.46 (s, 1H), 7.94 (s, 1H), 7.75 (s, 1H), 7.60 (d, *J* = 15.0 Hz, 3H), 7.47 (s, 1H), 6.77 (s, 1H), 2.69 (s, 3H).

### Example 31: 2- (3,5-Dichloro-4- ((2-(6-fluoropyridin-3-yl) -4-methylquinolin-6-yl) oxy) phenyl) -3,5-dioxo-2,3,4,5-tetrahydro-1,2,4-triazine-6-carbonitrile

By using 6-fluoro-3-(4,4,5,5-tetramethyl-1,3,2-dioxaborolan-2-yl)pyridine, the compound was prepared, separated and purified by referring to the method in example 1 (total yield 15%)M/Z = 535.05(M+1) ¹H NMR (500 MHz, Chloroform ) δ 9.54 (s, 1H), 8.97 (s, 1H), 8.46 (s, 1H), 7.94 (s, 1H), 7.75 (s, 1H), 7.60 (d, *J* = 15.0 Hz, 3H), 7.47 (s, 1H), 6.77 (s, 1H), 2.69 (s, 3H).

### Example 32: 2- (3,5-Dichloro-4- ((2-(6-chloropyridin-3-yl) -4-methylquinolin-6-yl) oxy) phenyl) -3,5-dioxo-2,3,4,5-tetrahydro-1,2,4-triazine-6-carbonitrile

By using 6-chloro-3-(4,4,5,5-tetramethyl-1,3,2-dioxaborolan-2-yl)pyridine, the compound was prepared, separated and purified by referring to the method in example 1 (total yield 11%)M/Z = 551.01(M+1) ¹H NMR (500 MHz, Chloroform ) δ 9.53 (s, 1H), 9.37 (s, 1H), 8.54 (s, 1H), 7.94 (s, 1H), 7.60 (d, *J* = 15.0 Hz, 3H), 7.48 (d, *J* = 10.0 Hz, 2H), 6.97 (s, 1H), 2.69 (s, 3H).

### Example 33: 2- (3,5-Dichloro-4- ((2-(6-bromopyridin-3-yl) -4-methylquinolin-6-yl) oxy) phenyl) -3,5-dioxo-2,3,4,5-tetrahydro-1,2,4-triazine-6-carbonitrile

By using 6-bromo-3-(4,4,5,5-tetramethyl-1,3,2-dioxaborolan-2-yl)pyridine, the compound was prepared, separated and purified by referring to the method in example 1 (total yield 20%)M/Z = 594.96(M+1) ¹H NMR (500 MHz, Chloroform ) δ 9.53 (s, 1H), 9.37 (s, 1H), 8.54 (s, 1H), 7.94 (s, 1H), 7.60 (d, *J* = 15.0 Hz, 3H), 7.48 (d, *J* = 10.0 Hz, 2H), 6.97 (s, 1H), 2.69 (s, 3H).

### Example 34: 2- (3,5-Dichloro-4- ((2-(6-trifluoromethylpyridin-3-yl) -4-methylquinolin-6-yl) oxy) phenyl) -3,5-dioxo-2,3,4,5-tetrahydro-1,2,4-triazine-6-carbonitrile

By using 6-trifluoromethyl-3-(4,4,5,5-tetramethyl-1,3,2-dioxaborolan-2-yl)pyridine, the compound was prepared, separated and purified by referring to the method in example 1 (total yield 17%)M/Z = 585.04(M+1) ¹H NMR (500 MHz, Chloroform ) δ 9.53 (s, 1H), 9.37 (s, 1H), 8.54 (s, 1H), 7.94 (s, 1H), 7.60 (d, *J* = 15.0 Hz, 3H), 7.48 (d, *J* = 10.0 Hz, 2H), 6.97 (s, 1H), 2.69 (s, 3H).

### Example 35: 2- (3,5-Dichloro-4- ((2-(pyridin-4-yl) -4-methylquinolin-6-yl) oxy) phenyl) -3,5-dioxo-2,3,4,5-tetrahydro-1,2,4-triazine-6-carbonitrile

By using 3-(4,4,5,5-tetramethyl-1,3,2-dioxaborolan-4-yl)pyridine, the compound was prepared, separated and purified by referring to the method in example 1 (total yield 18%) M/Z = 517.05(M+1) ¹H NMR (500 MHz, Chloroform ) δ 9.53 (s, 1H), 9.37 (s, 1H), 8.54 (s, 1H), 7.94 (s, 1H), 7.60 (d, *J* = 15.0 Hz, 3H), 7.48 (d, *J* = 10.0 Hz, 2H), 6.97 (s, 1H), 2.69 (s, 3H).

### Example 36: 2- (3,5-Dichloro-4- ((2-(2-methylpyridin-4-yl) -4-methylquinolin-6-yl) oxy) phenyl) -3,5-dioxo-2,3,4,5-tetrahydro-1,2,4-triazine-6-carbonitrile

By using 2-methyl-3-(4,4,5,5-tetramethyl-1,3,2-dioxaborolan-4-yl)pyridine, the compound was prepared, separated and purified by referring to the method in example 1 (total yield 18%) M/Z = 531.07(M+1) ¹H NMR (500 MHz, Chloroform ) δ 9.53 (s, 1H), 9.37 (s, 1H), 8.54 (s, 1H), 7.94 (s, 1H), 7.60 (d, *J* = 15.0 Hz, 3H), 7.48 (d, *J* = 10.0 Hz, 2H), 6.97 (s, 1H), 2.69 (s, 3H).

### Example 37: 2- (3,5-Dichloro-4- ((2-(2-chloropyridin-4-yl) -4-methylquinolin-6-yl) oxy) phenyl) -3,5-dioxo-2,3,4,5-tetrahydro-1,2,4-triazine-6-carbonitrile

By using 2-chloro-3-(4,4,5,5-tetramethyl-1,3,2-dioxaborolan-4-yl)pyridine, the compound was prepared, separated and purified by referring to the method in example 1 (total yield 19%) M/Z = 551.01(M+1) ¹H NMR (500 MHz, Chloroform ) δ 9.55 (s, 1H), 9.08 (s, 1H), 8.91 (s, 1H), 8.37 (s, 1H), 7.94 (s, 1H), 7.60 (d, *J* = 15.0 Hz, 3H), 7.47 (s, 1H), 6.97 (s, 1H), 2.69 (s, 3H).

### Example 38: 2- (3,5-Dichloro-4- ((2- (2-fluoropyridin-4-yl) -4-methylquinolin-6-yl) oxy) phenyl) -3,5-dioxo-2,3,4,5-tetrahydro-1,2,4-triazine-6-carbonitrile

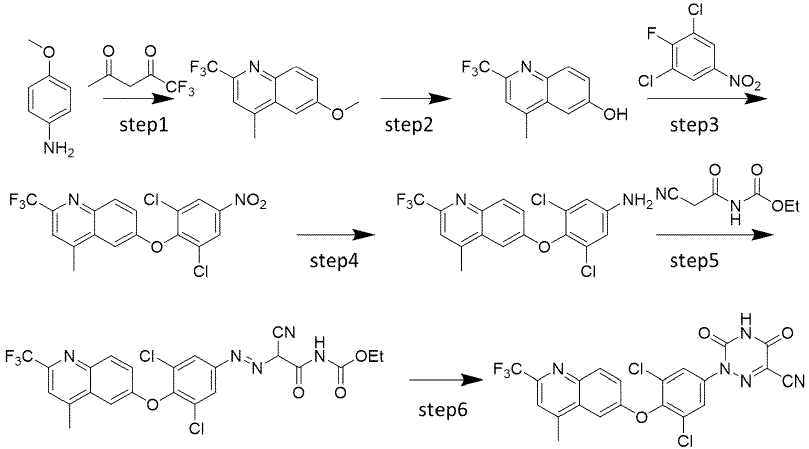

### Step 1: Preparation of 6-methoxy-4-methyl-2- (trifluoromethyl)quinoline

A commercially available methoxylaniline (10g, 0.0801mol) and trifluoroacetyl acetone (15g, 0.9744mol) were added to a mixed solvent of polyphosphate and toluene and reacted at 110 °C for 48 hours. After the reaction was completed, the reaction solution was cooled and added to 2L ice water mixture, adjusted to pH 8-9 with sodium bicarbonate aqueous solution, and then extracted with ethyl acetate. The organic phase was dried over anhydrous sodium sulfate, filtered and concentrated under reduced pressure, and then purified by column chromatography to afford a white crystal 6-methoxy-4-methyl-2- (trifluoromethyl) quinoline (9g, yield 45.95%).

### Step 2: Preparation of 6-hydroxyl-4-methyl-2- (trifluoromethyl)quinoline

6-Methoxy-4-methyl-2- (trifluoromethyl) quinoline(2g, 0.0083mol) was addded to 20 ml of 48% aqueous hydrobromic acid solution and refluxed at 90 °Cfor three days. After the reaction was finished, the resulting solution was adjusted with aqueous sodium bicarbonate to pH 8-9, and extracted with ethyl acetate three times. The combined organic phase was dried over anhydrous sodium sulfate, filtered and concentrated under reduced pressure to obtain a yellow oily compound 6-methoxy-4-methyl-2-(trifluoromethyl)quinoline(1.6g, 84%), the crude product was used directly in the next step without purification.

### Step 3: Preparation of 6- (2,6-dichloro-4-nitrophenoxy) -4-methyl-2-(trifluoromethyl)quinoline

To a solution of 6-methoxy-4-methyl-2(trifluoromethyl)quinoline and 3,5-dichloro-p-fluoronitrobenzene (1.48 g, 0.0070 mol) in 20 ml of DMF was added 5 g of potassium carbonate and stirred for 6 hr at ambient temperature. Then the reaction was quenched by adding 200 ml of water, and extracted with ethyl acetate three times, the organic phases were combined and washed three times with saturated sodium chloride solution. The organic phase was dried over anhydrous sodium sulfate, filtered, concentrated under reduced pressure, and then purified by column chromatography to obtain a light yellow compound 6-(2,6-dichloro-4-nitrophenoxy) -4-methyl-2-(trifluoromethyl)quinoline(1.5g, 51%).

### Step 4: Preparation of 3,5-dichloro-4- ((4-methyl-2- (trifluoromethyl)quinolin-6-yl) oxy) aniline

To a solution of 6- (2,6-dichloro-4-nitrophenoxy) -4-methyl-2-(trifluoromethyl)quinoline(1.5g,0.0031mol) in 15 ml of ethyl acetate and 15 ml of ethanol was added excess stannous chloride and a catalytic amount of hydrochloric acid, and stirred overnight at ambient temperature until the reaction was complete. The reaction solution was adjusted with aqueous sodium carbonate to pH 9-10, filtered to remove the precipitate, extracted three times with ethyl acetate. The combined organic phase was washed three times with saturated saline, dried over anhydrous sodium sulfate, filtered and concentrated under reduced pressure to obtain a light yellow solid 3,5-dichloro-4-((4-methyl-2- (trifluoromethyl)quinolin-6-yl) oxy) aniline(1g, 71.8%).

### Step 5: Preparation of ethyl (E) - (2-cyano-2- ((3,5-dichloro-4- ((4-methyl-2-(trifluoromethyl)quinolin-6-yl) oxy) phenyl) dienyl) acetyl) carbamate

To a flask containing 3,5-dichloro-4- ((4-methyl-2- (trifluoromethyl)quinolin-6-yl) oxy) aniline(1g, 0.0026mol) was added 10 ml of water, as well as 10 ml of hydrochloric acid, and 3 ml of an aqueous solution of sodium nitrite(213mg, 0.0031mol) was added dropwise at 0°C. To another round-bottomed flask was added 15 ml of pyridine and 10 ml of water, and N-cyanoacetylurethane (483.93 mg, 0.0031 mol). Both phases were held at 0°C for 30 min; then the diazonium salt was added dropwise to the other phase, after addition, the temperature was held for 30 min, then the mixture was extracted with ethyl acetate, and the organic phase was washed with saline, dried and concentrated under reduced pressure to give a red solid ethyl(E) - (2-cyano-2- ((3,5-dichloro-4- ((4-methyl-2- (trifluoromethyl)quinolin-6-yl) oxy) phenyl) dienyl) acetyl) carbamate (1.2 g, 83.8%), and the crude product was used directly in the next step without purification.

### Step 6: Preparation of 2- (3,5-Dichloro-4- ((2-trifluoromethyl- 4-methylquinolin-6-yl) oxy) phenyl) -3,5-dioxo-2,3,4,5-tetrahydro-1,2,4-triazine-6-carbonitrile

To a solution of orange ethyl (E)-(2-cyano-2-((3,5-dichloro-4-((4-methyl-2-(trifluoromethyl)quinolin-6-yl)oxy)phenyl)die nyl)acetyl)carbamate (1.2g,83.8%) in 10 ml of acetic acid was added 1 g of sodium acetate, and heated for 2 hr. at 120°C. The reaction was then added with water to precipitate a crude red compound, filtered and dried, and then purified by high performance preparative liquid phase separation to obtain white 2-(3,5-dichloro-4-((2-trifluoromethyl-4-methylquinolin-6-yl)oxy)phenyl)-3,5-dioxo-2,3,4,5-t etrahydro- 1,2,4-triazine-6-carbonitrile, i.e., compound of example 1 (200mg)LCMS m/z =508.01(M+1) ¹H NMR (500 MHz, Chloroform ) δ 9.53 (s, 1H), 8.00 (s, 1H), 7.67 (s, 1H), 7.57 (d, *J* = 10.0 Hz, 3H), 7.31 (s, 1H).

### Example 39: 2- (3,5-Dichloro-4- ((2- (2-bromopyridin-4-yl) -4-methylquinolin-6-yl) oxy) phenyl) -3,5-dioxo-2,3,4,5-tetrahydro-1,2,4-triazine-6-carbonitrile

By using 2-bromo-3-(4,4,5,5-tetramethyl-1,3,2-dioxaborolan-4-yl)pyridine, the compound was prepared, separated and purified by referring to the method in example 1 (total yield 15%)M/Z = 594.96(M+1) ¹H NMR (500 MHz, Chloroform ) δ 9.55 (s, 1H), 9.08 (s, 1H), 8.91 (s, 1H), 8.37 (s, 1H), 7.94 (s, 1H), 7.60 (d, *J* = 15.0 Hz, 3H), 7.47 (s, 1H), 6.97 (s, 1H), 2.69 (s, 3H).

### Example 40: 2- (3,5-Dichloro-4- ((2- (2-trifluoromethylpyridin-4-yl) -4-methylquinolin-6-yl) oxy) phenyl) -3,5-dioxo-2,3,4,5-tetrahydro-1,2,4-triazine-6-carbonitrile

By using 2-trifluoromethyl-3-(4,4,5,5-tetramethyl-1,3,2-dioxaborolan-4-yl)pyridine, the compound was prepared, separated and purified by referring to the method in example 1 (total yield 16%)M/Z = 585.04(M+1) ¹H NMR (500 MHz, Chloroform ) δ 9.55 (s, 1H), 9.08 (s, 1H), 8.91 (s, 1H), 8.37 (s, 1H), 7.94 (s, 1H), 7.60 (d, *J* = 15.0 Hz, 3H), 7.47 (s, 1H), 6.97 (s, 1H), 2.69 (s, 3H).

### Example 41: 2- (3,5-Dichloro-4-((2-chloro-4-methylquinolin-6-yl) oxy) phenyl) -3,5-dioxo-2,3,4,5-tetrahydro -1,2,4-triazine-6-carbonitrile

The compound was prepared, separated and purified by referring to the method in example 1 (total yield 13%)M/Z = 473.98 (M+1) ¹H NMR (500 MHz, Chloroform ) δ 9.56 (s, 1H), 7.93 (s, 1H), 7.58 (s, 2H), 7.51 (s, 1H), 7.42 (d, *J* = 15.0 Hz, 2H), 2.57 (s, 3H).

### Example 42: 2- (3,5-Dichloro-4-((4-chloroquinolin-6-yl) oxy) phenyl) -3,5-dioxo-2,3,4,5-tetrahydro -1,2,4-triazine-6-carbonitrile

By using the commercially available raw material, the compound was prepared, separated and purified by referring to the method in example 1 (total yield 11%)M/Z = 459.97 (M+1) ¹H NMR (500 MHz, Chloroform ) δ 9.53 (s, 1H), 8.75 (s, 1H), 7.95 (s, 1H), 7.91 (s, 1H), 7.58 (s, 2H), 7.52 (s, 1H), 7.42 (s, 1H).

### Example 43: 2- (3,5-Dichloro-4-((4-chloroquinolin-6-yl) oxy) phenyl) -3,5-dioxo-2,3,4,5-tetrahydro -1,2,4-triazine-6-carbonitrile

By using the commercially available raw material, the compound was prepared, separated and purified by referring to the method in example 1 (total yield 17%)M/Z = 503.92 (M+1) ¹H NMR (500 MHz, Chloroform ) δ 9.54 (s, 1H), 8.64 (s, 1H), 7.96 (s, 1H), 7.88 (s, 1H), 7.79 - 7.37 (m, 4H).

### Example 44: 2- (3,5-Dichloro-4- ((4-chloro-2-(trifluoromethyl)quinolin-6-yl) oxy) phenyl) -3,5-dioxo-2,3,4,5-tetrahydro- -1,2,4-triazine-6-carbonitrile

By using the commercially available raw material, the compound was prepared, separated and purified by referring to the method in example 1 (total yield 23%) M/Z = 527.96 (M+1) ¹H NMR (500 MHz, Chloroform) δ 9.54 (s, 1H), 7.99 (d, *J* = 20.0 Hz, 2H), 7.78 - 7.55 (m, 4H).

### Example 45: 2- (3,5-Dichloro-4-((2- methyl -4-phenylquinolin-6-yl) oxy) phenyl -3,5-dioxo-2,3,4,5-tetrahydro -1,2,4-triazine-6-carbonitrile

By using the commercially available raw material, the compound was prepared, separated and purified by referring to the method in example 1 (total yield 26%) M/Z = 516.06 (M+1) ¹H NMR (500 MHz, Chloroform) δ 9.56 (s, 4H), 7.97 (s, 4H), 7.79 (s, 8H), 7.58 (s, 8H), 7.43 (t, *J* = 12.5 Hz, 16H), 7.36 (s, 1H), 7.32 (d, *J* = 40.0 Hz, 7H), 2.69 (s, 12H).

### Example 46: 2- (3,5-Dichloro-4- ((4- methyl-2-vinylquinolin-6-yl) oxy) phenyl) -3,5-dioxo-2,3,4,5-tetrahydro -1,2,4-triazine-6-carbonitrile

By using the commercially available raw material, the compound was prepared, separated and purified by referring to the method in example 1 (total yield 22%) M/Z = 466.04(M+1) ¹H NMR (500 MHz, Chloroform) δ 9.56 (s, 1H), 7.90 (s, 1H), 7.57 (d, *J=* 15.0 Hz, 3H), 7.41 (s, 1H), 7.32 (s, 1H), 7.21 (s, 1H), 6.50 (s, 1H), 5.71 (s, 1H), 2.69 (s, 3H).

### Example 47: 2- (3,5-Dimethyl-4-((4-methyl-2-phenylquinolin-6-yl) oxy) phenyl -3,5-dioxo-2,3,4,5-tetrahydro-1,2,4-triazine-6-carbonitrile

By using the commercially available raw material, the compound was prepared, separated and purified by referring to the method in example 1 (total yield 17%) M/Z = 476.16 (M+1) ¹H NMR (500 MHz, Chloroform) δ 9.57 (s, 1H), 8.33 (s, 2H), 7.92 (s, 1H), 7.67 (s, 1H), 7.55 (s, 2H), 7.49 (s, 2H), 7.43 (s, 2H), 6.63 (s, 1H), 2.69 (s, 3H), 2.15 (s, 6H).

### Example 48: 2- (3,5-Dichloro-4-((4- methyl- 2- (pyrrolidin-1-yl)quinolin-6-yl) oxy) phenyl) -3,5-dioxo-2,3,4,5-tetrahydro -1,2,4-triazine-6-carbonitrile

By using the commercially available raw material, the compound was prepared, separated and purified by referring to the method in example 1 (total yield 17%) M/Z = 509.08(M+1) ¹H NMR (500 MHz, Chloroform) δ 9.55 (s, 103H), 7.64 (d, *J=* 60.0 Hz, 321H), 7.56 - 7.44 (m, 4H), 7.31 (s, 105H), 7.08 (s, 108H), 6.88 (s, 103H), 3.51 (s, 355H), 2.55 (s, 309H), 1.90 (s, 289H).

### Example 49: 2- (3,5-Dichloro-4-((2- methyl-4- (pyrrolidin-1-yl)quinolin-6-yl) oxy) phenyl) -3,5-dioxo-2,3,4,5-tetrahydro -1,2,4-triazine-6-carbonitrile

By using the commercially available raw material, the compound was prepared, separated and purified by referring to the method in example 1 (total yield 11%) M/Z = 509.08(M+1) ¹H NMR (500 MHz, Chloroform ) δ 9.55 (s, 4H), 7.86 (s, 4H), 7.76 (s, 4H), 7.58 (s, 8H), 7.38 (s, 4H), 7.03 (s, 4H), 3.41 (s, 14H), 2.53 (s, 12H), 1.93 (s, 11H).

### Example 50: 2- (3,5-Dichloro-4-((2- methyl -4-vinylquinolin-6-yl) oxy) phenyl) -3,5-dioxo-2,3,4,5-tetrahydro -1,2,4-triazine-6-carbonitrile

By using the commercially available raw material, the compound was prepared, separated and purified by referring to the method in example 1 (total yield 12%) M/Z = 466.04(M+1) ¹H NMR (500 MHz, Chloroform) δ 9.54 (s, 2H), 7.92 (s, 2H), 7.58 (s, 4H), 7.44 - 7.36 (m, 6H), 7.08 (s, 1H), 6.19 (s, 2H), 5.68 (s, 2H), 2.68 (s, 6H).

### Example 51: 2- (3,5-Dichloro-4-((2- chloro-3-methylquinolin-6-yl) oxy) phenyl) -3,5-dioxo-2,3,4,5-tetrahydro -1,2,4-triazine-6-carbonitrile

By using the commercially available raw material, the compound was prepared, separated and purified by referring to the method in example 1 (total yield 11%) M/Z = 476.16(M+1) ¹H NMR (500 MHz, Chloroform) δ 9.57 (s, 1H), 8.07 (s, 1H), 7.83 (d, *J* = 10.0 Hz, 3H), 7.58 - 7.40 (m, 7H), 2.56 (s, 3H), 2.15 (s, 6H).

### Example 52: 2- (3,5-Dichloro-4-((2- chloro-3-methylquinolin-6-yl) oxy) phenyl) -3,5-dioxo-2,3,4,5-tetrahydro -1,2,4-triazine-6-carbonitrile

By using the commercially available raw material, the compound was prepared, separated and purified by referring to the method in example 1 (total yield 10%) M/Z = 473.98(M+1) ¹H NMR (500 MHz, Chloroform) δ 9.56 (s, 9H), 7.79 (d, *J* = 55.0 Hz, 17H), 7.74 - 7.70 (m, 1H), 7.58 (s, 5H), 7.49 (d, *J* = 85.0 Hz, 21H), 7.34 (s, 9H), 2.40 (s, 26H).

### Example 53: 2- (3,5-Dichloro-4-((3-methyl-2-phenylquinolin-6-yl) oxy) phenyl -3,5-dioxo-2,3,4,5-tetrahydro-1,2,4-triazine-6-carbonitrile

By using the commercially available raw material, the compound was prepared, separated and purified by referring to the method in example 1 (total yield 29%) M/Z = 516.06 (M+1) ¹H NMR (500 MHz, Chloroform) δ 9.55 (s, 1H), 8.07 (s, 1H), 7.83 (d, *J* = 10.0 Hz, 3H), 7.58 (s, 2H), 7.58 - 7.41 (m, 5H), 2.56 (s, 3H).

### Example 54: 2- (3,5-Difluoro-4-((4-methyl-2-phenylquinolin-6-yl) oxy) phenyl -3,5-dioxo-2,3,4,5-tetrahydro-1,2,4-triazine-6-carbonitrile

By using the commercially available raw material, the compound was prepared, separated and purified by referring to the method in example 1 (total yield 15%) M/Z = 484.11(M+1) ¹H NMR (500 MHz, Chloroform) δ 9.57 (s, 1H), 8.33 (s, 2H), 7.92 (s, 1H), 7.67 (s, 1H), 7.55 (s, 2H), 7.51 (dd, *J* = 3.4, 1.5 Hz, 1H), 7.41 (d, *J* = 80.0 Hz, 4H), 6.63 (s, 1H), 2.69 (s, 3H).

### Example 55: 2- (3-bromo-5-methyl-4- ((4-methyl-2-phenylquinolin-6-yl) oxy) phenyl -3,5-dioxo-2,3,4,5-tetrahydro -1,2,4-triazine-6-carbonitrile

By using the commercially available raw material, the compound was prepared, separated and purified by referring to the method in example 1 (total yield 16%) M/Z = 540.06(M+1) ¹H NMR (500 MHz, Chloroform) δ 9.57 (s, 1H), 8.33 (s, 2H), 7.92 (s, 1H), 7.67 (s, 1H), 7.64 - 7.43 (m, 5H), 7.40 (s, 1H), 6.63 (s, 1H), 2.69 (s, 3H), 2.15 (s, 3H).

### Example 56: 2- (3-bromo-5-chloro-4-((3-chloro-4-methyl-2-(trifluoromethyl)quinolin-6-yl) oxy) phenyl -3,5-dioxo-2,3,4,5-tetrahydro -1,2,4-triazine-6-carbonitrile

By using the commercially available raw material, the compound was prepared, separated and purified by referring to the method in example 1 (total yield 13%) M/Z = 560.00 (M+1) ¹H NMR (500 MHz, Chloroform) δ 9.56 (s, 40H), 8.33 (s, 83H), 7.92 (s, 42H), 7.67 (s, 41H), 7.65 (s, 3H), 7.56 (t, *J* = 37.5 Hz, 199H), 7.38 (s, 41H), 6.63 (s, 40H), 2.69 (s, 120H).

### Example 57: 2- (3,5-Dichloro-4-((4-chloroquinolin-6-yl) oxy) phenyl) -3,5-dioxo-2,3,4,5-tetrahydro -1,2,4-triazine-6-carbonitrile

By using the commercially available 4-bromo-6-methoxyquinoline as raw material, the compound was prepared, separated and purified by referring to the method in example 1 (total yield 17%) M/Z = 585.92(M+1) ¹H NMR (500 MHz, Chloroform) δ 9.54 (s, 1H), 7.90 (s, 1H), 7.60 (t, *J* = 37.5 Hz, 3H), 7.38 (s, 1H), 2.45 (s, 3H).

### Example 58: 6-amino-2- (3,5-dichloro-4- ((4-methyl-2- (2-fluoropyridin-4-yl) quinolin-6-yl) oxy) phenyl) -1,2,4-triazine-3,5 (2H, 4H) - dione

### Step 1: Preparation of 2- (3,5-Dichloro-4- ((4- methyl-2-(2-fluoropyridin-4-yl) quinolin-6-yl) oxy) phenyl)-3,5-dioxo-2,3,4,5-tetrahydro-1,2,4-triazine-6-carbonitrile

The method in example 1 was applied to obtain product of example 1 and the compound of example 1 was used as raw material and added to a mixed solution of acetic acid and concentrated hydrochloric acid. The reaction was heated at 120 °C for 1 hour, then cooled down, added with water and filtered to remove the precipitated solid, and then vauccm dried to obtain a white powder 2-(3,5-dichloro-4-((4-methyl-2-(2-fluoropyridin-4-yl)quinolin-6-yl)oxy)phenyl)-3,5-dioxo-2, 3,4,5-tetrahydro- 1,2,4-triazine-6-carboxylic acid

### Step 2: Preparation of 6-amino-2- (3,5-dichloro-4- ((2-(4-methyl-2-(2-fluoropyridin-4-yl) quinolin-6-yl) oxy) phenyl) -1,2,4-triazine-3,5 (2H, 4H) - dione

To a solution of 2-(3,5-dichloro-4-((4-methyl-2-(2-fluoropyridin-4-yl)quinolin-6-yl)oxy)phenyl)-3,5-dioxo-2, 3,4,5-tetrahydro-1,2,4-triazine-6-carboxylic acid in tert-butanol was added triethylamine and DPPA, and reacted at 80°C for 24 hr under heating. After the reaction was finished, the reaction was concentrated under reduced pressure to remove tert-butanol and extracted with ethyl acetate three times. The combined organic phase was washed with saturated saline, dried over anhydrous sodium sulfate, filtered and concentrated under reduced pressure, then mixed with trifluoroacetic acid and stirred for 1 hour to remove the protecting group, concentrated under reduced pressure to remove trifluoroacetic acid. The resulting solution was alkalized with aqueous sodium hydroxide, extracted and concentrated under reduced pressure and then separated and purified by high performance preparative liquid phase to obtain a white powder compound: 6-amino-2-(3,5-dichloro-4-((4-methyl-2-(2-fluoropyridin-4-yl)quinolin-6-yl)oxy)phenyl)-1,2, 4-triazine-3,5(2H,4H)-dione, i.e., the compound of example 58. M/Z = 498.03(M+1) ¹H NMR (500 MHz, Chloroform) δ 9.55 (s, 1H), 9.08 (s, 1H), 8.91 (s, 1H), 8.37 (s, 1H), 7.94 (s, 1H), 7.60 (d, *J* = 15.0 Hz, 3H), 7.47 (s, 1H), 6.97 (s, 1H), 2.69 (s, 3H).

### Example 59: 6-amino-2- (3,5-dichloro-4- ((2-(4-chlorophenyl)-4-methylquinolin-6-yl) oxy) phenyl) -1,2,4-triazine-3,5 (2H, 4H) - dione

By using the compound of example 2 as raw material, the method in example 58 was used to afford 6-amino-2-(3,5-dichloro-4-((4-methyl-2-(trifluoromethyl)quinolin-6-yl)oxy)phenyl)-1,2,4-triazine-3,5(2H,4H)-dione M/Z = 506.07(M+1) ¹H NMR (500 MHz, Chloroform) δ 9.08 (s, 1H), 8.33 (s, 2H), 7.76 (dd, *J* = 132.2, 37.9 Hz, 6H), 7.67 (s, 1H), 7.62 (d, *J* = 45.0 Hz, 3H), 7.56 (d, *J* = 15.0 Hz, 4H), 7.49 (s, 2H), 6.63 (s, 1H), 2.69 (s, 3H).

### Example 60: 6-amino-2-(3,5-dichloro-4-((2-(4-fluorophenyl)-4-methylquinolin-6-yl)oxy)phenyl)-1,2,4-triazine-3,5( 2H,4H)-dione

By using the compound of example 3 as raw material, the method in example 58 was used to afford 6-amino-2-(3,5-dichloro-4-((2-(4-fluorophenyl)-4-methylquinolin-6-yl)oxy)phenyl)-1,2,4-tri azine-3,5( 2H,4H)-dione M/Z = 524.06(M+1) ¹H NMR (500 MHz, Chloroform) δ 9.08 (s, 1H), 8.33 (s, 2H), 7.76 (dd, *J* = 132.2, 37.9 Hz, 6H), 7.67 (s, 1H), 7.62 (d, *J* = 45.0 Hz, 3H), 7.56 (d, *J* = 15.0 Hz, 4H), 7.49 (s, 2H), 6.63 (s, 1H), 2.69 (s, 3H).

### Example 61: 6-amino-2-(3,5-dichloro-4-((2-(4-trifluoromethylphenyl)-4-methylquinolin-6-yl)oxy)phe nyl)-1,2,4-triazine-3,5( 2H,4H)-dione

By using the compound of example 4 as raw material, the method in example 58 was used to afford 6-amino-2-(3,5-dichloro-4-((2-(4-trifluoromethylphenyl)-4-methylquinolin-6-yl)oxy)phenyl)-1,2,4-triazine-3,5( 2H,4H)-dione M/Z = 574.06(M+1) ¹H NMR (500 MHz, Chloroform) δ 9.08 (s, 1H), 8.33 (s, 2H), 7.76 (dd, *J* = 132.2, 37.9 Hz, 6H), 7.67 (s, 1H), 7.62 (d, *J* = 45.0 Hz, 3H), 7.56 (d, *J* = 15.0 Hz, 4H), 7.49 (s, 2H), 6.63 (s, 1H), 2.69 (s, 3H).

### Example 62: 6-amino-2-(3,5-dichloro-4-((2-(4-chlorophenyl)-4-methylquinolin-6-yl)oxy)phenyl )-1,2,4-triazine-3,5( 2H,4H)-dione

By using the compound of example 5 as raw material, the method in example 58 was used to afford 6-amino-2-(3,5-dichloro-4-((2-(4-chlorophenyl)-4-methylquinolin-6-yl)oxy)phenyl)-1,2,4-tri azine-3,5( 2H,4H)-dione M/Z = 540.03(M+1) ¹H NMR (500 MHz, Chloroform) δ 9.08 (s, 1H), 8.33 (s, 2H), 7.76 (dd, *J* = 132.2, 37.9 Hz, 6H), 7.67 (s, 1H), 7.62 (d, *J* = 45.0 Hz, 3H), 7.56 (d, *J* = 15.0 Hz, 4H), 7.49 (s, 2H), 6.63 (s, 1H), 2.69 (s, 3H).

### Example 63: 6-amino-2-(3,5-dichloro-4-((2-(4-bromophenyl)-4-methylquinolin-6-yl)oxy)phenyl)-1,2,4 -triazine-3,5( 2H,4H)-dione

By using the compound of example 6 as raw material, the method in example 58 was used to afford 6-amino-2-(3,5-dichloro-4-((2-(4-bromophenyl)-4-methylquinolin-6-yl)oxy)phenyl)-1,2,4-tri azine-3,5( 2H,4H)-dione M/Z =583.98(M+1) ¹H NMR (500 MHz, Chloroform) δ 9.08 (s, 1H), 8.33 (s, 2H), 7.76 (dd, *J* = 132.2, 37.9 Hz, 6H), 7.67 (s, 1H), 7.62 (d, *J* = 45.0 Hz, 3H), 7.56 (d, *J* = 15.0 Hz, 4H), 7.49 (s, 2H), 6.63 (s, 1H), 2.69 (s, 3H).

### Example 64: 6-amino-2-(3,5-dichloro-4-((2-(4-cyanophenyl)-4-methylquinolin-6-yl)oxy)phenyl)-1,2,4-triazine-3,5( 2H,4H)-dione

By using the compound of example 7 as raw material, the method in example 58 was used to afford 6-amino-2-(3,5-dichloro-4-((2-(4-cyanophenyl)-4-methylquinolin-6-yl)oxy)phenyl)-1,2,4-tria zine-3,5( 2H,4H)-dione M/Z = 531.07(M+1) ¹H NMR (500 MHz, Chloroform) δ 9.07 (s, 1H), 8.34 (s, 2H), 7.94 - 7.85 (m, 5H), 7.67 (s, 1H), 7.58 (s, 2H), 7.49 (s, 1H), 6.63 (s, 1H), 2.69 (s, 3H).

### Example 65: 6-amino-2-(3,5-dichloro-4-((2-(3-chloro-5-fluorophenyl)-4-methylquinolin-6-yl)oxy)phen yl)-1,2,4-triazine- 3,5(2H,4H)-dione

By using the compound of example 8 as raw material, the method in example 58 was used to afford 6-amino-2-(3,5-dichloro-4-((2-(3-chloro-5-fluorophenyl)-4-methylquinolin-6-yl)oxy)phenyl) -1,2,4-triazine- 3,5(2H,4H)-dione M/Z = 558.02(M+1) ¹H NMR (500 MHz, Chloroform) δ 9.07 (s, 1H), 8.34 (s, 2H), 7.94 - 7.85 (m, 5H), 7.67 (s, 1H), 7.58 (s, 2H), 7.49 (s, 1H), 6.63 (s, 1H), 2.69 (s, 3H).

### Example 66: 6-amino-2-(3,5-dichloro-4-((2-(2-isopropylphenyl)-4-methylquinolin-6-yl)oxy)phenyl)-1, 2,4-triazine-3,5( 2H,4H)-dione

By using the compound of example 9 as raw material, the method in example 58 was used to afford 6-amino-2-(3,5-dichloro-4-((2-(2-isopropylphenyl)-4-methylquinolin-6-yl)oxy)phenyl)-1,2,4-triazine-3,5( 2H,4H)-dione M/Z = 548.12(M+1) ¹H NMR (500 MHz, Chloroform) δ 9.07 (s, 1H), 8.34 (s, 2H), 7.94 - 7.85 (m, 5H), 7.67 (s, 1H), 7.58 (s, 2H), 7.49 (s, 1H), 6.63 (s, 1H), 2.69 (s, 3H).

### Example 67: 6-amino-2-(3,5-dichloro-4-((2-(4-fluoro-3-methylphenyl)-4-methylquinolin-6-yl)oxy)phe nyl)-1,2,4-triazine- 3,5(2H,4H)-dione

By using the compound of example 10 as raw material, the method in example 58 was used to afford 6-amino-2-(3,5-dichloro-4-((2-(4-fluoro-3-methylphenyl)-4-methylquinolin-6-yl)oxy)phenyl) -1,2,4-triazine- 3,5(2H,4H)-dione M/Z = 538.02(M+1) ¹H NMR (500 MHz, Chloroform) δ 9.07 (s, 1H), 8.34 (s, 2H), 7.94 - 7.85 (m, 5H), 7.67 (s, 1H), 7.58 (s, 2H), 7.49 (s, 1H), 6.63 (s, 1H), 2.69 (s, 3H).

### Example 68: 4-(6-(4-(6-amino-3,5-dioxo-4,5-dihydro-1,2,4-triazin-2(3H)-yl)-2,6-dichlorophenoxy)-4-methylquinolin-2-yl)-2-fluorobenzonitrile

By using the compound of example 11 as raw material, the method in example 58 was used to afford 4-(6-(4-(6-amino-3,5-dioxo-4,5-dihydro-1,2,4-triazin-2(3H)-yl)-2,6-dichlorophenoxy)-4-methylquinolin-2-yl)-2-fluorobenzonitrile M/Z = 549.06(M+1) ¹H NMR (500 MHz, Chloroform) δ 9.07 (s, 1H), 8.34 (s, 2H), 7.94 - 7.85 (m, 5H), 7.67 (s, 1H), 7.58 (s, 2H), 7.49 (s, 1H), 6.63 (s, 1H), 2.69 (s, 3H).

### Example 69: 2-(6-(4-(6-amino-3,5-dioxo-4,5-dihydro-1,2,4-triazin-2(3H)yl)-2,6-dichlorophenoxy)-4-m ethylquinolin-2-yl)-5-(trifluoromethyl)benzonitrile

By using the compound of example 12 as raw material, the method in example 58 was used to afford 2-(6-(4-(6-amino-3,5-dioxo-4,5-dihydro-1,2,4-triazin-2(3H)yl)-2,6-dichlorophenoxy)-4-meth ylquinolin-2-yl)-5-(trifluoromethyl)benzonitrile M/Z = 599.05(M+1) ¹H NMR (500 MHz, Chloroform) δ 9.10 (s, 1H), 8.27 (d, *J* = 5.0 Hz, 2H), 8.00 (s, 1H), 7.75 (dd, *J* = 129.7, 40.4 Hz, 6H), 7.67 (s, 1H), 7.62 (d, *J* = 45.0 Hz, 3H), 7.58 (s, 2H), 7.49 (s, 1H), 6.63 (s, 1H), 2.69 (s, 3H).

### Example 70: 6-amino-2-(3,5-dichloro-4-((2-(3,5-dichloro-4-fluorophenyl)-4-methylquinolin-6-yl)oxy) phenyl)-1,2,4-triazine-3,5(2H,4H)dione

By using the compound of example 13 as raw material, the method in example 58 was used to afford 6-amino-2-(3,5-dichloro-4-((2-(3,5-dichloro-4-fluorophenyl)-4-methylquinolin-6-yl)oxy)phe nyl)-1,2,4-triazine-3,5(2H,4H)dione M/Z = 591.98(M+1) ¹H NMR (500 MHz, Chloroform) δ 9.10 (s, 1H), 8.27 (d, *J* = 5.0 Hz, 2H), 8.00 (s, 1H), 7.75 (dd, *J* = 129.7, 40.4 Hz, 6H), 7.67 (s, 1H), 7.62 (d, *J* = 45.0 Hz, 3H), 7.58 (s, 2H), 7.49 (s, 1H), 6.63 (s, 1H), 2.69 (s, 3H).

### Example 71: 6-amino-2-(3,5-dichloro-4-((2-(4-chloro-3,5-dimethylphenyl)-4-methylquinolin-6-yl)oxy) -1,2,4-triazine-3,5(2H,4H)-dione

By using the compound of example 14 as raw material, the method in example 58 was used to afford 6-amino-2-(3,5-dichloro-4-((2-(4-chloro-3,5-dimethylphenyl)-4-methylquinolin-6-yl)oxy)-1, 2,4-triazine-3,5(2H,4H)-dione M/Z = 568.06(M+1) ¹H NMR (500 MHz, Chloroform) δ 9.10 (s, 1H), 8.27 (d, *J* = 5.0 Hz, 2H), 8.00 (s, 1H), 7.75 (dd, *J* = 129.7, 40.4 Hz, 6H), 7.67 (s, 1H), 7.62 (d, *J* = 45.0 Hz, 3H), 7.58 (s, 2H), 7.49 (s, 1H), 6.63 (s, 1H), 2.69 (s, 3H).

### Example 72: 6-amino-2-(3,5-dichloro-4-((2-(furan-2-yl)-4-methylquinolin-6-yl)oxy)phenyl)-1,2,4-triaz ine-3, 5(2H,4H)-dione

By using the compound of example 15 as raw material, the method in example 58 was used to afford 6-amino-2-(3,5-dichloro-4-((2-(furan-2-yl)-4-methylquinolin-6-yl)oxy)phenyl)-1,2,4-triazine -3, 5(2H,4H)-dione M/Z = 496.05(M+1) ¹H NMR (500 MHz, Chloroform) δ 9.10 (s, 1H), 8.27 (d, *J* = 5.0 Hz, 2H), 8.00 (s, 1H), 7.75 (dd, *J* = 129.7, 40.4 Hz, 6H), 7.67 (s, 1H), 7.62 (d, *J* = 45.0 Hz, 3H), 7.58 (s, 2H), 7.49 (s, 1H), 6.63 (s, 1H), 2.69 (s, 3H).

### Example 73: 6-amino-2-(3,5-dichloro-4-((4-methyl-2-(5-methylfuran-2-yl)quinolin-6-yl)oxy)phenyl)-1 ,2,4-triazine -3,5(2H,4H)-dione

By using the compound of example 16 as raw material, the method in example 58 was used to afford 6-amino-2-(3,5-dichloro-4-((4-methyl-2-(5-methylfuran-2-yl)quinolin-6-yl)oxy)phenyl)-1,2,4 -triazine -3,5(2H,4H)-dione M/Z = 510.07(M+1) ¹H NMR (500 MHz, Chloroform) δ 9.10 (s, 1H), 8.27 (d, *J* = 5.0 Hz, 2H), 8.00 (s, 1H), 7.75 (dd, *J* = 129.7, 40.4 Hz, 6H), 7.67 (s, 1H), 7.62 (d, *J* = 45.0 Hz, 3H), 7.58 (s, 2H), 7.49 (s, 1H), 6.63 (s, 1H), 2.69 (s, 3H).

### Example 74: 6-amino-2-(3,5-dichloro-4-((4-methyl-2-(5-methylfuran-2-yl)quinolin-6-yl)oxy)phenyl)-1 ,2,4-triazine -3,5(2H,4H)-dione

By using the compound of example 17 as raw material, the method in example 58 was used to afford 6-amino-2-(3,5-dichloro-4-((4-methyl-2-(5-methylfuran-2-yl)quinolin-6-yl)oxy)phenyl)-1,2,4 -triazine -3,5(2H,4H)-dione M/Z = 510.07(M+1) ¹H NMR (500 MHz, Chloroform) δ 9.07 (s, 1H), 7.94 (s, 1H), 7.85 (s, 2H), 7.60 (d, *J* = 15.0 Hz, 3H), 7.47 (s, 1H), 7.27 (s, 1H), 6.97 (s, 1H), 6.42 (s, 1H), 2.69 (s, 3H), 2.25 (s, 3H).

### Example 75: 6-amino-2-(3,5-dichloro-4-((4-methyl-2-(5-methylfuran-2-yl)quinolin-6-yl)oxy)phenyl)-1 ,2,4-triazine -3,5(2H,4H)-dione

By using the compound of example 18 as raw material, the method in example 58 was used to afford 6-amino-2-(3,5-dichloro-4-((4-methyl-2-(5-methylfuran-2-yl)quinolin-6-yl)oxy)phenyl)-1,2,4 -triazine -3,5(2H,4H)-dione M/Z = 535.06(M+1) ¹H NMR (500 MHz, Chloroform) δ 9.12 (s, 1H), 8.11 - 7.60 (m, 4H), 7.61 (s, 1H), 7.60 (d, *J* = 15.0 Hz, 3H), 7.47 (s, 1H), 6.97 (s, 1H), 6.37 (s, 1H), 2.69 (s, 3H), 2.25 (s, 3H).

### Example 76: 6-amino-2-(3,5-dichloro-4-((4-methyl-2-(thiophen-2-yl)quinolin-6-yl)oxy)phenyl)-1,2,4-t riazine-3 ,5(2H,4H)-dione

By using the compound of example 19 as raw material, the method in example 58 was used to afford 6-amino-2-(3,5-dichloro-4-((4-methyl-2-(thiophen-2-yl)quinolin-6-yl)oxy)phenyl)-1,2,4-triaz ine-3 ,5(2H,4H)-dione M/Z = 512.03(M+1) ¹H NMR (500 MHz, Chloroform) δ 9.08 (s, 2H), 8.12 - 7.56 (m, 16H), 7.66 (s, 2H), 7.74 - 7.56 (m, 8H), 7.60 (d, *J* = 15.0 Hz, 6H), 7.47 (s, 2H), 7.19 (s, 1H), 6.97 (s, 2H), 2.69 (s, 6H).

### Example 77: 6-amino-2-(3,5-dichloro-4-((2-(4-chlorothiophen-2-yl)-4-methylquinolin-6-yl)oxy)phenyl )-1,2,4-triazine- 3,5(2H,4H)-dione

By using the compound of example 20 as raw material, the method in example 58 was used to afford 6-amino-2-(3,5-dichloro-4-((2-(4-chlorothiophen-2-yl)-4-methylquinolin-6-yl)oxy)phenyl)-1, 2,4-triazine- 3,5(2H,4H)-dione M/Z = 545.99(M+1) ¹H NMR (500 MHz, Chloroform) δ 9.08 (s, 2H), 8.12 - 7.56 (m, 16H), 7.66 (s, 2H), 7.74 - 7.56 (m, 8H), 7.60 (d, *J* = 15.0 Hz, 6H), 7.47 (s, 2H), 7.19 (s, 1H), 6.97 (s, 2H), 2.69 (s, 6H).

### Example 78: 5-(6-(4-(6-amino-3,5-dioxo-4,5-dihydro-1,2,4-triazin-2(3H)-yl)-2,6-dichlorophenoxy)-4-methylquinolin-2-yl)thiophen-2-carbonitrile

By using the compound of example 21 as raw material, the method in example 58 was used to afford 5-(6-(4-(6-amino-3,5-dioxo-4,5-dihydro-1,2,4-triazine-2(3H)-yl)-2,6-dichlorophenoxy)-4-methylquinolin-2-yl)thiophen-2-carbonitrile M/Z = 537.02(M+1) ¹H NMR (500 MHz, Chloroform) δ 9.08 (s, 2H), 8.12 - 7.56 (m, 16H), 7.66 (s, 2H), 7.74 - 7.56 (m, 8H), 7.60 (d, *J* = 15.0 Hz, 6H), 7.47 (s, 2H), 7.19 (s, 1H), 6.97 (s, 2H), 2.69 (s, 6H).

### Example 79: 6-amino-2-(3,5-dichloro-4-((2-(5-methylthiophen-2-yl)-4-methylquinolin-6-yl)oxy)pheny l)-1,2,4-triazine- 3,5(2H,4H)-dione

By using the compound of example 22 as raw material, the method in example 58 was used to afford 6-amino-2-(3,5-dichloro-4-((2-(5-methylthiophen-2-yl)-4-methylquinolin-6-yl)oxy)phenyl)-1 ,2,4-triazine- 3,5(2H,4H)-dione M/Z = 526.04(M+1) ¹H NMR (500 MHz, Chloroform) δ 9.08 (s, 2H), 8.12 - 7.56 (m, 16H), 7.66 (s, 2H), 7.74 - 7.56 (m, 8H), 7.60 (d, *J* = 15.0 Hz, 6H), 7.47 (s, 2H), 7.19 (s, 1H), 6.97 (s, 2H), 2.69 (s, 6H).

### Example 80: 6-amino-2-(3,5-dichloro-4-((2-(5-trifluoromethylthiophen-2-yl)-4-methylquinolin-6-yl)ox y)phenyl)-1,2,4-triazine- 3,5(2H,4H)-dione

By using the compound of example 23 as raw material, the method in example 58 was used to afford 6-amino-2-(3,5-dichloro-4-((2-(5-trifluoromethylthiophen-2-yl)-4-methylquinolin-6-yl)oxy)p henyl)-1,2,4-triazine- 3,5(2H,4H)-dione M/Z = 580.01(M+1) ¹H NMR (500 MHz, Chloroform) δ 9.08 (s, 1H), 8.12 - 7.61 (m, 5H), 7.61 (s, 1H), 7.60 (d, *J* = 15.0 Hz, 3H), 7.47 (s, 1H), 7.02 (d, *J* = 55.0 Hz, 2H), 2.69 (s, 3H).

### Example 81: 6-amino-2-(3,5-dichloro-4-((4-methyl-2-(pyridin-2-yl)quinolin-6-yl)oxy)phenyl)-1,2,4-tri azine-3 ,5(2H,4H)-dione

By using the compound of example 24 as raw material, the method in example 58 was used to afford 6-amino-2-(3,5-dichloro-4-((4-methyl-2-(pyridin-2-yl)quinolin-6-yl)oxy)phenyl)-1,2,4-triazi ne-3 ,5(2H,4H)-dione M/Z = 507.07(M+1) ¹H NMR (500 MHz, Chloroform) δ 9.18 (s, 1H), 9.09 (s, 1H), 8.55 (s, 1H), 7.94 (s, 1H), 7.86 (s, 2H), 7.74 (d, *J =* 5.0 Hz, 2H), 7.59 (d, *J =* 15.0 Hz, 3H), 7.47 (s, 1H), 7.23 (s, 1H), 2.69 (s, 3H).

### Example 82: 6-amino-2-(3,5-dichloro-4-((2-(5-methylpyridin-2-yl)-4-methylquinolin-6-yl)oxy)phenyl) -1,2,4-triazine- 3,5(2H,4H)-dione

By using the compound of example 25 as raw material, the method in example 58 was used to afford 6-amino-2-(3,5-dichloro-4-((2-(5-methylpyridin-2-yl)-4-methylquinolin-6-yl)oxy)phenyl)-1, 2,4-triazine- 3,5(2H,4H)-dione M/Z = 521.08(M+1) ¹H NMR (500 MHz, Chloroform) δ 9.08 (s, 1H), 8.69 (s, 1H), 8.33 (s, 1H), 7.94 (s, 1H), 7.84 (s, 2H), 7.75 (s, 1H), 7.59 (d, *J* = 15.0 Hz, 3H), 7.47 (d, *J* = 5.0 Hz, 2H), 2.69 (s, 3H), 2.32 (s, 3H).

### Example 83: 6-amino-2-(3,5-dichloro-4-((2-(5-fluoropyridin-2-yl)-4-methylquinolin-6-yl)oxy)phenyl)-1,2,4-triazine- 3,5(2H,4H)-dione

By using the compound of example 26 as raw material, the method in example 58 was used to afford 6-amino-2-(3,5-dichloro-4-((2-(5-fluoropyridin-2-yl)-4-methylquinolin-6-yl)oxy)phenyl)-1,2, 4-triazine- 3,5(2H,4H)-dione M/Z = 525.06(M+1) ¹H NMR (500 MHz, Chloroform) δ 9.10 (s, 84H), 8.76 (s, 45H), 8.33 (s, 87H), 7.91 (d, *J=* 33.8 Hz, 252H), 7.86 - 7.85 (m, 6H), 7.75 (s, 84H), 7.60 (d, *J=* 15.0 Hz, 256H), 7.46 (d, *J=* 14.1 Hz, 166H), 2.69 (s, 251H).

### Example 84: 6-amino-2-(3,5-dichloro-4-((2-(5-chloropyridin-2-yl)-4-methylquinolin-6-yl)oxy)phenyl)-1,2,4-triazine- 3,5(2H,4H)-dione

By using the compound of example 27 as raw material, the method in example 58 was used to afford 6-amino-2-(3,5-dichloro-4-((2-(5-chloropyridin-2-yl)-4-methylquinolin-6-yl)oxy)phenyl)-1,2 ,4-triazine- 3,5(2H,4H)-dione M/Z = 541.03(M+1) ¹H NMR (500 MHz, Chloroform) δ 9.10 (s, 1H), 8.97 (s, 1H), 8.46 (s, 1H), 8.12 - 7.52 (m, 7H), 7.75 (s, 1H), 7.85 - 7.52 (m, 4H), 7.60 (d, *J* = 15.0 Hz, 3H), 7.47 (s, 1H), 6.77 (s, 1H), 2.69 (s, 3H).

### Example 85: 6-amino-2-(3,5-dichloro-4-((2-(5-bromopyridin-2-yl)-4-methylquinolin-6-yl)oxy)phenyl)-1,2,4-triazine- 3,5(2H,4H)-dione

By using the compound of example 28 as raw material, the method in example 58 was used to afford 6-amino-2-(3,5-dichloro-4-((2-(5-bromopyridin-2-yl)-4-methylquinolin-6-yl)oxy)phenyl)-1,2 ,4-triazine- 3,5(2H,4H)-dione M/Z = 584.98(M+1) ¹H NMR (500 MHz, Chloroform) δ 9.11 (d, *J* = 11.3 Hz, 2H), 8.52 (s, 1H), 7.94 (s, 1H), 7.87 (s, 2H), 7.74 (d, *J* = 5.0 Hz, 2H), 7.60 (d, *J* = 15.0 Hz, 3H), 7.47 (s, 1H), 2.69 (s, 3H).

### Example 86: 6-amino-2-(3,5-dichloro-4-((4-methyl-2-(pyridin-3-yl)quinolin-6-yl)oxy)phenyl)-1,2,4-tri azine-3 ,5(2H,4H)-dione

By using the compound of example 29 as raw material, the method in example 58 was used to afford 6-amino-2-(3,5-dichloro-4-((4-methyl-2-(pyridin-3-yl)quinolin-6-yl)oxy)phenyl)-1,2,4-triazi ne-3 ,5(2H,4H)-dione M/Z = 507.07(M+1) ¹H NMR (500 MHz, Chloroform) δ 9.63 (s, 2H), 9.08 (s, 2H), 8.71 (d, *J* = 10.0 Hz, 4H), 7.94 (s, 2H), 7.86 (s, 4H), 7.59 (d, *J* = 15.0 Hz, 6H), 7.47 (s, 3H), 6.97 (s, 2H), 2.69 (s, 6H).

### Example 87: 6-amino-2-(3,5-dichloro-4-((4-methyl-2-(6-methylpyridin-3-yl)quinolin-6-yl)oxy)phenyl) -1,2,4-triazine-3 ,5(2H,4H)-dione

By using the compound of example 30 as raw material, the method in example 58 was used to afford 6-amino-2-(3,5-dichloro-4-((4-methyl-2-(6-methylpyridin-3-yl)quinolin-6-yl)oxy)phenyl)-1, 2,4-triazine-3 ,5(2H,4H)-dione M/Z = 521.08(M+1) ¹H NMR (500 MHz, Chloroform) δ 9.33 (s, 1H), 9.07 (s, 1H), 8.36 (s, 1H), 7.94 (s, 1H), 7.84 (s, 2H), 7.60 (d, *J* = 15.0 Hz, 3H), 7.47 (s, 1H), 7.20 (s, 1H), 6.97 (s, 1H), 2.69 (s, 3H), 2.61 (s, 3H).

### Example 88: 6-amino-2-(3,5-dichloro-4-((4-methyl-2-(6-fluoropyridin-3-yl)quinolin-6-yl)oxy)phenyl)-1,2,4-triazine-3 ,5(2H,4H)-dione

By using the compound of example 31 as raw material, the method in example 58 was used to afford 6-amino-2-(3,5-dichloro-4-((4-methyl-2-(6-fluoropyridin-3-yl)quinolin-6-yl)oxy)phenyl)-1,2, 4-triazine-3 ,5(2H,4H)-dione M/Z = 525.06(M+1) ¹H NMR (500 MHz, Chloroform) δ 9.12 (d, *J* = 3.0 Hz, 8H), 8.64 (s, 4H), 7.94 (s, 4H), 7.85 (s, 8H), 7.60 (d, *J* = 15.0 Hz, 12H), 7.44 (d, *J* = 25.6 Hz, 8H), 7.39 (s, 1H), 6.97 (s, 4H), 2.69 (s, 12H).

### Example 89: 6-amino-2-(3,5-dichloro-4-((4-methyl-2-(6-chloropyridin-3-yl)quinolin-6-yl)oxy)phenyl)-1,2,4-triazine-3 ,5(2H,4H)-dione

By using the compound of example 32 as raw material, the method in example 58 was used to afford 6-amino-2-(3,5-dichloro-4-((4-methyl-2-(6-chloropyridin-3-yl)quinolin-6-yl)oxy)phenyl)-1,2 ,4-triazine-3 ,5(2H,4H)-dione M/Z = 541.03(M+1) ¹H NMR (500 MHz, Chloroform) δ 9.38 (s, 1H), 9.09 (s, 1H), 8.52 (s, 1H), 7.94 (s, 1H), 7.86 (s, 2H), 7.60 (d, *J=* 15.0 Hz, 3H), 7.48 (d, *J* = 10.0 Hz, 2H), 6.97 (s, 1H), 2.69 (s, 3H).

### Example 90: 6-amino-2-(3,5-dichloro-4-((4-methyl-2-(6-bromopyridin-3-yl)quinolin-6-yl)oxy)phenyl)-1,2,4-triazine-3 ,5(2H,4H)-dione

By using the compound of example 33 as raw material, the method in example 58 was used to afford 6-amino-2-(3,5-dichloro-4-((4-methyl-2-(6-bromopyridin-3-yl)quinolin-6-yl)oxy)phenyl)-1,2 ,4-triazine-3 ,5(2H,4H)-dione M/Z = 584.98(M+1) ¹H NMR (500 MHz, Chloroform) δ 9.22 (s, 1H), 9.09 (s, 1H), 8.46 (s, 1H), 7.94 (s, 1H), 7.86 (s, 2H), 7.71 (s, 1H), 7.60 (d, *J=* 15.0 Hz, 3H), 7.47 (s, 1H), 6.97 (s, 1H), 2.69 (s, 3H).

### Example 91: 6-amino-2-(3,5-dichloro-4-((4-methyl-2-(6-bromopyridin-3-yl)quinolin-6-yl)oxy)phenyl)-1,2,4-triazine-3 ,5(2H,4H)-dione

By using the compound of example 34 as raw material, the method in example 58 was used to afford 6-amino-2-(3,5-dichloro-4-((4-methyl-2-(6-bromopyridin-3-yl)quinolin-6-yl)oxy)phenyl)-1,2 ,4-triazine-3 ,5(2H,4H)-dione M/Z = 575.05(M+1) ¹H NMR (500 MHz, Chloroform) δ 9.48 (s, 2H), 9.10 (s, 2H), 8.37 (s, 2H), 7.91 (d, *J* = 33.5 Hz, 7H), 7.61 (s, 1H), 7.60 (d, *J* = 15.0 Hz, 6H), 7.39 (d, *J=* 80.0 Hz, 4H), 6.97 (s, 2H), 2.69 (s, 6H).

### Example 92: 6-amino-2-(3,5-dichloro-4-((4-methyl-2-(pyridin-4-yl)quinolin-6-yl)oxy)phenyl)-1,2,4-tri azine-3 ,5(2H,4H)-dione

By using the compound of example 35 as raw material, the method in example 58 was used to afford 6-amino-2-(3,5-dichloro-4-((4-methyl-2-(pyridin-4-yl)quinolin-6-yl)oxy)phenyl)-1,2,4-triazi ne-3 ,5(2H,4H)-dione M/Z = 507.07(M+1) ¹H NMR (500 MHz, Chloroform) δ 9.09 (s, 1H), 8.75 (s, 2H), 8.28 (s, 2H), 7.94 (s, 1H), 7.86 (s, 2H), 7.60 (d, *J* = 15.0 Hz, 3H), 7.47 (s, 1H), 6.97 (s, 1H), 2.69 (s, 3H).

### Example 93: 6-amino-2-(3,5-dichloro-4-((4-methyl-2-(2-methylpyridin-4-yl)quinolin-6-yl)oxy)phenyl) -1,2,4-triazine-3 ,5(2H,4H)-dione

By using the compound of example 35 as raw material, the method in example 58 was used to afford 6-amino-2-(3,5-dichloro-4-((4-methyl-2-(2-methylpyridin-4-yl)quinolin-6-yl)oxy)phenyl)-1, 2,4-triazine-3 ,5(2H,4H)-dione M/Z = 521.08(M+1) ¹H NMR (500 MHz, Chloroform) δ 9.08 (s, 1H), 8.88 (s, 1H), 8.63 (s, 1H), 8.22 (s, 1H), 7.94 (s, 1H), 7.86 (s, 2H), 7.60 (d, *J* = 15.0 Hz, 3H), 7.47 (s, 1H), 6.97 (s, 1H), 2.69 (s, 6H).

### Example 94: 6-amino-2-(3,5-dichloro-4-((4-methyl-2-(2-chloropyridin-4-yl)quinolin-6-yl)oxy)phenyl)-1,2,4-triazine-3 ,5(2H,4H)-dione

By using the compound of example 37 as raw material, the method in example 58 was used to afford 6-amino-2-(3,5-dichloro-4-((4-methyl-2-(2-chloropyridin-4-yl)quinolin-6-yl)oxy)phenyl)-1,2 ,4-triazine-3 ,5(2H,4H)-dione M/Z = 541.03(M+1) ¹H NMR (500 MHz, Chloroform) δ 9.08 (d, *J* = 4.3 Hz, 2H), 8.91 (s, 1H), 8.37 (s, 1H), 7.90 (d, *J* = 35.0 Hz, 3H), 7.60 (d, *J* = 15.0 Hz, 3H), 7.47 (s, 1H), 6.97 (s, 1H), 2.69 (s, 3H).

### Example 95: 6-amino-2-(3,5-dichloro-4-((4-methyl-2-(2-fluoropyridin-4-yl)quinolin-6-yl)oxy)phenyl)-1,2,4-triazine-3 ,5(2H,4H)-dione

By using the compound of example 38 as raw material, the method in example 58 was used to afford 6-amino-2-(3,5-dichloro-4-((4-methyl-2-(2-fluoropyridin-4-yl)quinolin-6-yl)oxy)phenyl)-1,2, 4-triazine-3 ,5(2H,4H)-dione M/Z = 525.06(M+1) ¹H NMR (500 MHz, Chloroform) δ 9.07 (s, 3H), 8.91 (s, 3H), 8.58 (s, 3H), 8.15 - 7.61 (m, 13H), 7.61 (s, 1H), 7.60 (d, *J* = 15.0 Hz, 9H), 7.47 (s, 3H), 6.97 (s, 3H), 2.69 (s, 9H).

### Example 96: 6-amino-2-(3,5-dichloro-4-((4-methyl-2-(2-bromopyridin-4-yl)quinolin-6-yl)oxy)phenyl)-1,2,4-triazine-3 ,5(2H,4H)-dione

By using the compound of example 39 as raw material, the method in example 58 was used to afford 6-amino-2-(3,5-dichloro-4-((4-methyl-2-(2-bromopyridin-4-yl)quinolin-6-yl)oxy)phenyl)-1,2 ,4-triazine-3 ,5(2H,4H)-dione M/Z = 584.98(M+1) ¹H NMR (500 MHz, Chloroform) δ 9.03 (d, *J* = 49.6 Hz, 2H), 8.73 (s, 1H), 8.48 (s, 1H), 7.94 (s, 1H), 7.86 (s, 2H), 7.60 (d, *J* = 15.0 Hz, 3H), 7.47 (s, 1H), 6.97 (s, 1H), 2.69 (s, 3H).

### Example 97: 6-amino-2-(3,5-dichloro-4-((4-methyl-2-(2-trifluoromethylpyridin-4-yl)quinolin-6-yl)oxy )phenyl)-1,2,4-triazine-3 ,5(2H,4H)-dione

By using the compound of example 40 as raw material, the method in example 58 was used to afford 6-amino-2-(3,5-dichloro-4-((4-methyl-2-(2-trifluoromethylpyridin-4-yl)quinolin-6-yl)oxy)ph enyl)-1,2,4-triazine-3 ,5(2H,4H)-dione M/Z = 575.05(M+1) ¹H NMR (500 MHz, Chloroform) δ 9.08 (s, 1H), 8.80 (d, *J* = 5.0 Hz, 2H), 8.33 (s, 1H), 7.94 (s, 1H), 7.86 (s, 2H), 7.60 (d, *J*= 15.0 Hz, 3H), 7.47 (s, 1H), 6.97 (s, 1H), 2.69 (s, 3H).

### Example 98: 6-amino-2-(3,5-dichloro-4-((2-chloro-4-methylquinolin-6-yl)oxy)phenyl)-1,2,4-triazine-3, 5(2H,4H)-dione

By using the compound of example 41 as raw material, the method in example 58 was used to afford 6-amino-2-(3,5-dichloro-4-((2-chloro-4-methylquinolin-6-yl)oxy)phenyl)-1,2,4-triazine-3,5(2 H,4H)-dione M/Z = 464.00(M+1) ¹H NMR (500 MHz, Chloroform) δ 9.08 (s, 1H), 7.93 (s, 1H), 7.84 (s, 2H), 7.58 (s, 2H), 7.51 (s, 1H), 7.42 (d, *J* = 15.0 Hz, 2H), 2.57 (s, 3H).

### Example 99: 6-amino-2-(3,5-dichloro-4((4-chloroquinolin-6-yl)oxy)phenyl)-1,2,4-triazine-3,5(2H,4H)-dione

By using the compound of example 42 as raw material, the method in example 58 was used to afford 6-amino-2-(4-(4-bromoquinolin-6-yl)oxy)-3,5-dichlorophenyl)-1,2,4-triazine-3,5(2H,4H)-dio ne M/Z = 451.66(M+1) ¹H NMR (500 MHz, Chloroform) δ 9.07 (s, 18H), 8.75 (s, 19H), 8.13 - 7.83 (m, 74H), 7.86 (s, 36H), 7.86 (s, 36H), 7.60 (s, 1H), 7.55 (d, *J* = 30.0 Hz, 55H), 7.42 (s, 18H).

### Example 100: 6-amino-2-(4-(4-bromoquinolin-6-yl)oxy)-3,5-dichlorophenyl)-1,2,4-triazine-3,5(2H,4H)-dione

By using the compound of example 43 as raw material, the method in example 58 was used to afford 6-amino-2-(4-(4-bromoquinolin-6-yl)oxy)-3,5-dichlorophenyl)-1,2,4-triazine-3,5(2H,4H)-dio ne M/Z = 493.93(M+1) ¹H NMR (500 MHz, Chloroform) δ 9.07 (s, 1H), 8.64 (s, 1H), 7.96 (s, 1H), 7.86 (d, *J=* 15.5 Hz, 3H), 7.72 - 7.37 (m, 4H).

### Example 101: 6-amino-2-(3,5-dichloro-4-((4-chloro-2-(trifluoromethyl)quinolin-6-yl)oxy)phenyl)-1,2,4-triazine-3,5(2H,4H)-dione

By using the compound of example 44 as raw material, the method in example 58 was used to afford 6-amino-2-(3,5-dichloro-4-((4-chloro-2-(trifluoromethyl)quinolin-6-yl)oxy)phenyl)-1,2,4-tria zine-3,5(2H,4H)-dione M/Z = 517.97(M+1) ¹H NMR (500 MHz, Chloroform) δ 9.07 (s, 1H), 8.01 (s, 1H), 7.97 (s, 1H), 7.87 (s, 2H), 7.78 - 7.55 (m, 4H).

### Example 102: 6-amino-2-(3,5-dichloro-4-((2-methyl-4-phenylquinolin-6-yl)oxy)phenyl-1,2,4-triazine-3, 5(2H,4H)-dione

By using the compound of example 45 as raw material, the method in example 58 was used to afford 6-amino-2-(3,5-dichloro-4-((2-methyl-4-phenylquinolin-6-yl)oxy)phenyl-1,2,4-triazine-3,5(2 H,4H)-dione M/Z = 506.07(M+1) ¹H NMR (500 MHz, Chloroform) δ 9.08 (s, 1H), 7.97 (s, 1H), 7.84 (s, 2H), 7.79 (s, 2H), 7.58 (s, 2H), 7.56 - 7.39 (m, 4H), 7.36 (s, 1H), 7.28 (s, 1H), 2.69 (s, 3H).

### Example 103: 6-amino-2-(3,5-dichloro-4-((4-methyl-2-vinylquinolin-6-yl)oxy)phenyl)-1,2,4-triazine-3,5 (2H,4H)-dione

By using the compound of example 46 as raw material, the method in example 58 was used to afford 6-amino-2-(3,5-dichloro-4-((4-methyl-2-vinylquinolin-6-yl)oxy)phenyl)-1,2,4-triazine-3,5(2 H,4H)-dione M/Z = 456.06(M+1) ¹H NMR (500 MHz, Chloroform) δ 9.08 (s, 4H), 7.88 (d, *J* = 16.1 Hz, 12H), 7.57 (d, *J=* 15.0 Hz, 12H), 7.35 (d, *J=* 56.1 Hz, 9H), 7.27 (s, 1H), 7.21 (s, 2H), 6.50 (s, 4H), 5.71 (s, 4H), 2.69 (s, 12H).

### Example 104: 6-amino-2-(3,5-dimethyl-4-((4-methyl-2-phenylquinolin-6-yl)oxy)phenyl-1,2,4-triazine-3 ,5(2H,4H)-dione

By using the compound of example 47 as raw material, the method in example 58 was used to afford 6-amino-2-(3,5-dimethyl-4-((4-methyl-2-phenylquinolin-6-yl)oxy)phenyl-1,2,4-triazine-3,5( 2H,4H)-dione M/Z = 466.18(M+1) ¹H NMR (500 MHz, Chloroform) δ 9.05 (s, 1H), 8.33 (s, 2H), 7.92 (s, 1H), 7.84 (s, 2H), 7.67 (s, 1H), 7.55 (s, 2H), 7.46 (d, *J* = 30.0 Hz, 4H), 6.63 (s, 1H), 2.69 (s, 3H), 2.15 (s, 6H).

### Example 105: 6-amino-2-(3,5-dichloro-4-((4-methyl-2-(pyrrolidin-1-yl)quinolin-6-oxy)phenyl)-1,2,4-tri azine-3,5(2H,4H)-dione

By using the compound of example 48 as raw material, the method in example 58 was used to afford 6-amino-2-(3,5-dichloro-4-((4-methyl-2-(pyrrolidin-1-yl)quinolin-6-oxy)phenyl)-1,2,4-triazi ne-3,5(2H,4H)-dione M/Z = 499.10(M+1) ¹H NMR (500 MHz, Chloroform) δ 9.10 (s, 66H), 7.84 (s, 132H), 7.64 (d, *J* = 60.0 Hz, 208H), 7.39 (s, 3H), 7.31 (s, 67H), 7.08 (s, 69H), 6.88 (s, 66H), 3.51 (s, 227H), 2.55 (s, 198H), 1.90 (s, 185H).

### Example 106: 6-amino-2-(3,5-dichloro-4-((2-methyl-4-(pyrrolidin-1-yl)quinolin-6-oxy)phenyl)-1,2,4-tri azine-3,5(2H,4H)-dione

By using the compound of example 49 as raw material, the method in example 58 was used to afford 6-amino-2-(3,5-dichloro-4-((2-methyl-4-(pyrrolidin-1-yl)quinolin-6-oxy)phenyl)-1,2,4-triazi ne-3,5(2H,4H)-dione M/Z = 499.10 (M+1) ¹H NMR (500 MHz, Chloroform) δ 9.07 (s, 2H), 7.86 (d, *J* = 3.1 Hz, 6H), 7.76 (s, 2H), 7.58 (s, 4H), 7.38 (s, 2H), 7.03 (s, 2H), 3.41 (s, 7H), 2.53 (s, 6H), 1.93 (s, 6H).

### Example 107: 6-amino-2-(3,5-dichloro-4-((2-methyl-4-vinylquinolin-6-yl)oxy)phenyl)-1,2,4-triazine-3,5 (2H,4H)-dione

By using the compound of example 50 as raw material, the method in example 58 was used to afford 6-amino-2-(3,5-dichloro-4-((2-methyl-4-vinylquinolin-6-yl)oxy)phenyl)-1,2,4-triazine-3,5(2 H,4H)-dione M/Z = 456.06(M+1) ¹H NMR (500 MHz, Chloroform) δ 9.10 (s, 2H), 7.88 (d, *J* = 37.5 Hz, 6H), 7.58 (s, 4H), 7.44 - 7.36 (m, 6H), 7.08 (s, 1H), 6.19 (s, 2H), 5.68 (s, 2H), 2.68 (s, 6H).

### Example 108: 6-amino-2-(3,5-dimethyl-4-((3-methyl-2-phenylquinolin-6-yl)oxy)phenyl-1,2,4-triazine-3 ,5(2H,4H)-dione

By using the compound of example 51 as raw material, the method in example 58 was used to afford 6-amino-2-(3,5-dimethyl-4-((3-methyl-2-phenylquinolin-6-yl)oxy)phenyl-1,2,4-triazine-3,5( 2H,4H)-dione M/Z = 466.18(M+1) ¹H NMR (500 MHz, Chloroform) δ 9.05 (s, 1H), 8.07 (s, 1H), 7.83 (t, *J* = 5.0 Hz, 5H), 7.58 - 7.40 (m, 7H), 2.56 (s, 3H), 2.15 (s, 6H).

### Example 109: 6-amino-2-(3,5-dichloro-4-((2-chloro-3-methylquinolin-6-yl)oxy)phenyl)-1,2,4-triazine-3, 5(2H,4H)-dione

By using the compound of example 52 as raw material, the method in example 58 was used to afford 6-amino-2-(3,5-dichloro-4-((2-chloro-3-methylquinolin-6-yl)oxy)phenyl)-1,2,4-triazine-3,5(2 H,4H)-dione M/Z = 464.00(M+1) ¹H NMR (500 MHz, Chloroform) δ 9.08 (s, 1H), 7.84 (d, *J* = 6.6 Hz, 3H), 7.74 (s, 1H), 7.58 (s, 2H), 7.41 (s, 1H), 7.34 (s, 1H), 2.40 (s, 3H).

### Example 110: 6-amino-2-(3,5-dichloro-4-((3-methyl-2-phenylquinolin-6-yl)oxy)phenyl-1,2,4-triazine-3, 5(2H,4H)-dione

By using the compound of example 53 as raw material, the method in example 58 was used to afford 6-amino-2-(3,5-dichloro-4-((3-methyl-2-phenylquinolin-6-yl)oxy)phenyl-1,2,4-triazine-3,5(2 H,4H)-dione M/Z = 506.07(M+1) ¹H NMR (500 MHz, Chloroform) δ 9.08 (s, 1H), 8.07 (s, 1H), 7.88 - 7.74 (m, 5H), 7.58 (s, 2H), 7.56 - 7.41 (m, 5H), 2.56 (s, 3H).

### Example 111: 6-amino-2-(3,5-difluoro-4-((4-methyl-2-phenylquinolin-6-yl)oxyphenyl)-1,2,4-triazine-3, 5(2H,4H)-dione

By using the compound of example 54 as raw material, the method in example 58 was used to afford 6-amino-2-(3,5-difluoro-4-((4-methyl-2-phenylquinolin-6-yl)oxyphenyl)-1,2,4-triazine-3,5(2 H,4H)-dione M/Z = 474.13(M+1) ¹H NMR (500 MHz, Chloroform) δ 9.10 (s, 2H), 8.33 (s, 4H), 7.92 (s, 2H), 7.84 (s, 4H), 7.67 (s, 2H), 7.55 (s, 4H), 7.52 - 7.51 (m, 1H), 7.41 (d, *J* = 80.0 Hz, 8H), 6.63 (s, 2H), 2.69 (s, 6H).

### Example 112: 6-amino-2-(3-bromo-5-methyl-4-(4-methyl-2-phenylquinolin-6-yl)oxyphenyl)-1,2,4-triazi ne-3,5(2H,4H)-dione

By using the compound of example 55 as raw material, the method in example 58 was used to afford 6-amino-2-(3-bromo-5-methyl-4-(4-methyl-2-phenylquinolin-6-yl)oxyphenyl)-1,2,4-triazine-3,5(2H,4H)-dione M/Z = 530.07(M+1) ¹H NMR (500 MHz, Chloroform) δ 9.06 (s, 1H), 8.33 (s, 2H), 8.10 - 7.58 (m, 4H), 7.67 (s, 1H), 7.67 (s, 1H), 7.55 (s, 2H), 7.48 (d, *J=* 10.0 Hz, 3H), 7.40 (s, 1H), 6.63 (s, 1H), 2.69 (s, 3H), 2.15 (s, 3H).

### Example 113: 6-amino-2-(3-bromo-5-chloro-4-(4-methyl-2-phenylquinolin-6-yl)oxyphenyl)-1,2,4-triazi ne-3,5(2H,4H)-dione

By using the compound of example 56 as raw material, the method in example 58 was used to afford 6-amino-2-(3-bromo-5-chloro-4-(4-methyl-2-phenylquinolin-6-yl)oxyphenyl)-1,2,4-triazine-3,5(2H,4H)-dione M/Z = 550.02(M+1) ¹H NMR (500 MHz, Chloroform) δ 9.08 (s, 2H), 8.33 (s, 4H), 8.10 - 7.56 (m, 11H), 7.65 (d, *J* = 15.0 Hz, 5H), 7.65 (d, *J* = 15.0 Hz, 4H), 7.55 (s, 3H), 7.49 (s, 5H), 7.38 (s, 2H), 6.63 (s, 2H), 2.69 (s, 6H).

### Example 114: 6-amino-2-(3-bromo-5-chloro-4-((3-chloro-4-methyl-2-(trifluoromethyl)quinolin-6-yl)ox yphenyl)-1,2,4-triazine-3,5(2H,4H)-dione

By using the compound of example 57 as raw material, the method in example 58 was used to afford 6-amino-2-(3-bromo-5-chloro-4-((3-chloro-4-methyl-2-(trifluoromethyl)quinolin-6-yl)oxyph enyl)-1,2,4-triazine-3,5(2H,4H)-dione M/Z = 575.94(M+1) ¹H NMR (500 MHz, Chloroform) δ 9.08 (s, 1H), 8.08 - 7.55 (m, 5H), 7.65 (d, *J=* 10.0 Hz, 2H), 7.65 (d, *J=* 10.0 Hz, 2H), 7.45 (d, *J* = 65.0 Hz, 2H), 2.45 (s, 3H).

### Example 115: 2-(3,5-dichloro-4-((2-ethyl-4-methylquinolin-6-yl)oxy)phenyl)-3,5-dioxo-2,3,4,5-tetrahyd ro-1,2,4-triazine-6-carbonitrile

By using the corresponding borate ester as raw material, the method in example 1 was used to afford 2-(3,5-dichloro-4-((2-ethyl-4-methylquinolin-6-yl)oxy)phenyl)-3,5-dioxo-2,3,4,5-tetrahydro-1,2,4-triazine-6-carbonitrile M/Z =468.03(M+1) ¹H NMR (500 MHz, Chloroform) δ 9.08 (s, 1H), 8.08 - 7.55 (m, 5H), 7.65 (d, *J* = 10.0 Hz, 2H), 7.65 (d, *J* = 10.0 Hz, 2H), 7.45 (d, *J* = 65.0 Hz, 2H), 2.45 (s, 3H).

### Example 116: 2-(3,5-dichloro-4-((2-isopropyl-4-methylquinolin-6-yl)oxy)phenyl)-3,5-dioxo-2,3,4,5-tetr ahydro-1,2,4-triazine-6-carbonitrile

By using the corresponding borate ester as raw material, the method in example 1 was used to afford 2-(3,5-dichloro-4-((2-isopropyl-4-methylquinolin-6-yl)oxy)phenyl)-3,5-dioxo-2,3,4,5-tetrahy dro-1,2,4-triazine-6-carbonitrile M/Z = 482.02(M+1) ¹H NMR (500 MHz, Chloroform) δ 9.08 (s, 1H), 8.08 - 7.55 (m, 5H), 7.65 (d, *J* = 10.0 Hz, 2H), 7.65 (d, *J* = 10.0 Hz, 2H), 7.45 (d, *J* = 65.0 Hz, 2H), 2.45 (s, 3H).

### Example 117: 2-(3,5-dichloro-4-((2-cyclopropyl-4-methylquinolin-6-yl)oxy)phenyl)-3,5-dioxo-2,3,4,5-te trahydro-1,2,4-triazine-6-carbonitrile

By using the corresponding borate ester as raw material, the method in example 1 was used to afford 2-(3,5-dichloro-4-((2-cyclopropyl-4-methylquinolin-6-yl)oxy)phenyl)-3,5-dioxo-2,3,4,5-tetra hydro-1,2,4-triazine-6-carbonitrile M/Z = 480.01(M+1) ¹H NMR (500 MHz, Chloroform) δ 9.08 (s, 1H), 8.08 - 7.55 (m, 5H), 7.65 (d, *J* = 10.0 Hz, 2H), 7.65 (d, *J* = 10.0 Hz, 2H), 7.45 (d, *J* = 65.0 Hz, 2H), 2.45 (s, 3H).

### Example 118: 2-(3,5-dichloro-4-((2-cyclobutyl-4-methylquinolin-6-yl)oxy)phenyl)-3,5-dioxo-2,3,4,5-tet rahydro-1,2,4-triazine-6-carbonitrile

By using the corresponding borate ester as raw material, the method in example 1 was used to afford 2-(3,5-dichloro-4-((2-cyclobutyl-4-methylquinolin-6-yl)oxy)phenyl)-3,5-dioxo-2,3,4,5-tetrah ydro-1,2,4-triazine-6-carbonitrile M/Z = 494.01(M+1) ¹H NMR (500 MHz, Chloroform) δ 9.08 (s, 1H), 8.08 - 7.55 (m, 5H), 7.65 (d, *J* = 10.0 Hz, 2H), 7.65 (d, *J* = 10.0 Hz, 2H), 7.45 (d, *J* = 65.0 Hz, 2H), 2.45 (s, 3H).

### Example 119: 2-(3,5-dichloro-4-((2-cyclopentyl-4-methylquinolin-6-yl)oxy)phenyl)-3,5-dioxo-2,3,4,5-te trahydro-1,2,4-triazine-6-carbonitrile

By using the corresponding borate ester as raw material, the method in example 1 was used to afford 2-(3,5-dichloro-4-((2-cyclopentyl-4-methylquinolin-6-yl)oxy)phenyl)-3,5-dioxo-2,3,4,5-tetra hydro-1,2,4-triazine-6-carbonitrile M/Z = 508.02(M+1) ¹H NMR (500 MHz, Chloroform) δ 9.08 (s, 1H), 8.08 - 7.55 (m, 5H), 7.65 (d, *J* = 10.0 Hz, 2H), 7.65 (d, *J* = 10.0 Hz, 2H), 7.45 (d, *J* = 65.0 Hz, 2H), 2.45 (s, 3H).

### Example 120: 2-(3,5-dichloro-4-((2-cyclohexyl-4-methylquinolin-6-yl)oxy)phenyl)-3,5-dioxo-2,3,4,5-tet rahydro-1,2,4-triazine-6-carbonitrile

By using the corresponding borate ester as raw material, the method in example 1 was used to afford 2-(3,5-dichloro-4-((2-cyclohexyl-4-methylquinolin-6-yl)oxy)phenyl)-3,5-dioxo-2,3,4,5-tetrah ydro-1,2,4-triazine-6-carbonitrile M/Z = 521.11(M+1) ¹H NMR (500 MHz, Chloroform) δ 9.08 (s, 1H), 8.08 - 7.55 (m, 5H), 7.65 (d, *J* = 10.0 Hz, 2H), 7.65 (d, *J* = 10.0 Hz, 2H), 7.45 (d, *J* = 65.0 Hz, 2H), 2.45 (s, 3H).

### Example 121: 2-(3,5-dichloro-4-((2-(4,4-dimethylcyclohexyl)-4-methylquinolin-6-yl)oxy)phenyl)-3,5-di oxo-2,3,4,5-tetrahydro-1,2,4-triazine-6-carbonitrile

By using the corresponding borate ester as raw material, the method in example 1 was used to afford 2-(3,5-dichloro-4-((2-(4,4-dimethylcyclohexyl)-4-methylquinolin-6-yl)oxy)phenyl)-3,5-diox o-2,3,4,5-tetrahydro-1,2,4-triazine-6-carbonitrile M/Z = 550.15(M+1) ¹H NMR (500 MHz, Chloroform) δ 9.08 (s, 1H), 8.08 - 7.55 (m, 5H), 7.65 (d, *J* = 10.0 Hz, 2H), 7.65 (d, *J* = 10.0 Hz, 2H), 7.45 (d, *J* = 65.0 Hz, 2H), 2.45 (s, 3H).

### Example 122: 2-(3,5-dichloro-4-((2-(4,4-difluorocyclohexyl)-4-methylquinolin-6-yl)oxy)phenyl)-3,5-dio xo-2,3,4,5-tetrahydro-1,2,4-triazine-6-carbonitrile

By using the corresponding borate ester as raw material, the method in example 1 was used to afford 2-(3,5-dichloro-4-((2-(4,4-difluorocyclohexyl)-4-methylquinolin-6-yl)oxy)phenyl)-3,5-dioxo -2,3,4,5-tetrahydro-1,2,4-triazine-6-carbonitrile M/Z = 558.04(M+1) ¹H NMR (500 MHz, Chloroform) δ 9.08 (s, 1H), 8.08 - 7.55 (m, 5H), 7.65 (d, *J* = 10.0 Hz, 2H), 7.65 (d, *J* = 10.0 Hz, 2H), 7.45 (d, *J* = 65.0 Hz, 2H), 2.45 (s, 3H).

### Example 123: 2-(3,5-dichloro-4-((4-methyl-2-(4-(trifluoromethyl)cyclohexyl)quinolin-6-yl)oxy)phenyl)-3,5-dioxo-2,3,4,5-tetrahydro-1,2,4-triazine-6-carbonitrile

By using the corresponding borate ester as raw material, the method in example 1 was used to afford 2-(3,5-dichloro-4-((4-methyl-2-(4-(trifluoromethyl)cyclohexyl)quinolin-6-yl)oxy)phenyl)-3,5 -dioxo-2,3,4,5-tetrahydro-1,2,4-triazine-6-carbonitrile M/Z = 590.03(M+1)¹H NMR (500 MHz, Chloroform-d) δ 7.85 (d, J = 8.2 Hz, 1H), 7.67 (s, 2H), 7.48 (d, J = 1.7 Hz, 1H), 7.14 (dd, J = 8.1, 1.9 Hz, 1H), 7.02 (d, J = 1.1 Hz, 1H), 2.96 - 2.88 (m, 1H), 2.71 (s, 3H), 2.37 - 2.25 (m, 1H), 2.18 - 2.06 (m, 2H), 2.00 - 1.81 (m, 4H), 1.78 - 1.67 (m, 2H).

### Example 124: 2-(4-((2-(bicyclo[2.2.1]heptan-2-yl)-4-methylquinolin-6-yl)oxy)-3,5-dichlorophenyl)-3,5-dioxo-2,3,4,5-tetrahydro-1,2,4-triazine-6-carbonitrile

By using the corresponding borate ester as raw material, the method in example 1 was used to afford 2-(4-((2-(bicyclo[2.2.1]heptan-2-yl)-4-methylquinolin-6-yl)oxy)-3,5-dichlorophenyl)-3,5-dio xo-2,3,4,5-tetrahydro-1,2,4-triazine-6-carbonitrile M/Z = 534.32(M+1)¹H NMR (500 MHz, Chloroform-d) δ 7.85 (d, J = 8.2 Hz, 1H), 7.67 (s, 2H), 7.48 (d, J = 1.8 Hz, 1H), 7.17 - 7.09 (m, 2H), 2.88 (dt, J = 6.0, 4.3 Hz, 1H), 2.71 (s, 3H), 2.26 (tt, J = 5.3, 3.5 Hz, 1H), 2.13 (dp, J = 6.0, 3.8 Hz, 1H), 2.01 (ddd, J = 12.5, 5.3, 4.5 Hz, 1H), 1.76 - 1.69 (m, 1H), 1.72 - 1.66 (m, 1H), 1.69 - 1.63 (m, 2H), 1.66 - 1.59 (m, 1H), 1.63 - 1.54 (m, 1H), 1.57 - 1.49 (m, 1H), 1.46 - 1.38 (m, 1H).

### Example 125: 2-(3,5-dichloro-4-((4-methyl-2-(3-methylcyclohexyl)quinolin-6-yl)oxy)phenyl)-3,5-dioxo-2,3,4,5-tetrahydro-1,2,4-triazine-6-carbonitrile

By using the corresponding borate ester as raw material, the method in example 1 was used to afford 2-(3,5-dichloro-4-((4-methyl-2-(3-methylcyclohexyl)quinolin-6-yl)oxy)phenyl)-3,5-dioxo-2, 3,4,5-tetrahydro-1,2,4-triazine-6-carbonitrile M/Z = 536.39(M+1)¹H NMR (500 MHz, Chloroform-d) δ 7.85 (d, J = 8.2 Hz, 1H), 7.67 (s, 2H), 7.48 (d, J = 1.8 Hz, 1H), 7.14 (dd, J = 8.2, 1.9 Hz, 1H), 7.12 - 7.09 (m, 1H), 2.98 - 2.90 (m, 1H), 2.71 (s, 3H), 1.91 - 1.82 (m, 1H), 1.78 - 1.70 (m, 1H), 1.73 - 1.67 (m, 1H), 1.70 - 1.63 (m, 2H), 1.66 - 1.55 (m, 1H), 1.57 - 1.48 (m, 1H), 1.47 - 1.31 (m, 4H), 0.93 (d, J = 6.2 Hz, 3H).

### Example 126: 2-(3,5-dichloro-4-((4-methyl-2-(2-methylcyclohexyl)quinolin-6-yl)oxy)phenyl)-3,5-dioxo-2,3,4,5-tetrahydro-1,2,4-triazine-6-carbonitrile

By using the corresponding borate ester as raw material, the method in example 1 was used to afford 2-(3,5-dichloro-4-((4-methyl-2-(2-methylcyclohexyl)quinolin-6-yl)oxy)phenyl)-3,5-dioxo-2, 3,4,5-tetrahydro-1,2,4-triazine-6-carbonitrile M/Z = 536.40(M+1)¹H NMR (500 MHz, Chloroform-*d*) δ 7.85 (d, *J* = 8.2 Hz, 1H), 7.67 (s, 2H), 7.48 (d, *J* = 1.8 Hz, 1H), 7.14 (dd, *J* = 8.1, 1.9 Hz, 1H), 7.03 (s, 1H), 2.71 (s, 3H), 2.54 (dtd, *J* = 7.3, 6.0, 1.5 Hz, 1H), 2.21 (dddd, *J* = 12.6, 9.5, 6.8, 6.0 Hz, 1H), 1.99 - 1.85 (m, 2H), 1.74 - 1.30 (m, 6H), 0.95 (dd, *J* = 6.8, 1.5 Hz, 3H).

### Example 127: 2-(3,5-dichloro-4-((4-methyl-2-(4-methylcyclohexyl)quinolin-6-yl)oxy)phenyl)-3,5-dioxo-2,3,4,5-tetrahydro-1,2,4-triazine-6-carbonitrile

By using the corresponding borate ester as raw material, the method in example 1 was used to afford 2-(3,5-dichloro-4-((4-methyl-2-(4-methylcyclohexyl)quinolin-6-yl)oxy)phenyl)-3,5-dioxo-2, 3,4,5-tetrahydro-1,2,4-triazine-6-carbonitrile M/Z = 536.41(M+1)¹H NMR (500 MHz, Chloroform-*d*) δ 7.85 (d, *J* = 8.2 Hz, 1H), 7.67 (s, 2H), 7.48 (d, *J* = 1.8 Hz, 1H), 7.14 (dd, *J* = 8.1, 1.9 Hz, 1H), 7.02 (d, *J* = 0.9 Hz, 1H), 2.93 - 2.84 (m, 1H), 2.71 (s, 3H), 1.88 (ddt, *J* = 12.0, 8.8, 6.0 Hz, 2H), 1.78 (ddt, *J* = 12.3, 8.6, 5.9 Hz, 2H), 1.60 (ddt, *J* = 12.0, 8.6, 5.9 Hz, 2H), 1.55 - 1.44 (m, 1H), 1.39 - 1.29 (m, 2H), 0.92 (d, *J* = 6.1 Hz, 3H).

### Example 128: 2-(3,5-dichloro-4-(4-methyl-2-(3,3,5,5-tetramethylcyclohexyl)quinolin-6-yl)oxy)phenyl)-3,5-dioxo-2,3,4,5-tetrahydro-1,2,4-triazine-6-carbonitrile

By using the corresponding borate ester as raw material, the method in example 1 was used to afford 2-(3,5-dichloro-4-(4-methyl-2-(3,3,5,5-tetramethylcyclohexyl)quinolin-6-yl)oxy)phenyl)-3,5-dioxo-2,3,4,5-tetrahydro-1,2,4-triazine-6-carbonitrile M/Z = 578.48(M+1)¹H NMR (500 MHz, Chloroform-*d*) δ 7.67 (s, 1H), 7.48 (d, *J* = 1.7 Hz, 0H), 7.20 - 7.11 (m, 1H), 3.15 - 3.07 (m, 0H), 2.71 (s, 1H), 1.86 (dd, *J* = 12.3, 5.5 Hz, 1H), 1.75 (dd, *J* = 12.3, 5.5 Hz, 1H), 0.94 (s, 2H), 0.89 (s, 2H).

### Example 129: 6-amino-2-(3,5-dichloro-4-(2-ethyl-4-methylquinolin-6-yl)oxy)phenyl)-1,2,4-triazine-3,5( 2H,4H)-dione

By using the compound of example 115 as raw material, the method in example 58 was used to afford 6-amino-2-(3,5-dichloro-4-(2-ethyl-4-methylquinolin-6-yl)oxy)phenyl)-1,2,4-triazine-3,5(2H ,4H)-dione M/Z = 458.21(M+1)¹H NMR (500 MHz, Chloroform-*d*) δ 9.74 (s, 1H), 7.82 (d, *J* = 8.2 Hz, 1H), 7.67 (s, 2H), 7.47 (d, *J* = 1.8 Hz, 1H), 7.17 - 7.11 (m, 2H), 5.17 (s, 2H), 2.82 (q, *J* = 7.1 Hz, 2H), 2.65 (s, 2H), 1.29 (t, *J* = 7.1 Hz, 3H).

### Example 130: 6-amino-2-(3,5-dichloro-4-(2-isopropyl-4-methylquinolin-6-yl)oxy)phenyl)-1,2,4-triazine -3,5(2H,4H)-dione

By using the compound of example 116 as raw material, the method in example 58 was used to afford 6-amino-2-(3,5-dichloro-4-(2-isopropyl-4-methylquinolin-6-yl)oxy)phenyl)-1,2,4-triazine-3, 5(2H,4H)-dione M/Z = 472.28(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.8 Hz, 1H), 7.17 - 7.11 (m, 2H), 5.17 (s, 2H), 3.15 (qd, *J* = 6.9, 6.3 Hz, 1H), 2.71 (s, 3H), 1.30 (d, *J* = 6.6 Hz, 6H).

### Example 131: 6-amino-2-(3,5-dichloro-4-((2-cyclopropyl-4-methylquinolin-6-yl)oxy)phenyl)-1,2,4-triaz ine-3,5(2H,4H)-dione

By using the compound of example 117 as raw material, the method in example 58 was used to afford 6-amino-2-(3,5-dichloro-4-((2-cyclopropyl-4-methylquinolin-6-yl)oxy)phenyl)-1,2,4-triazine -3,5(2H,4H)-dione M/Z = 470.28(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.7 Hz, 1H), 7.23 (d, *J* = 1.0 Hz, 1H), 7.14 (dd, *J* = 8.1, 1.9 Hz, 1H), 5.17 (s, 2H), 2.71 (s, 3H), 2.46 - 2.38 (m, 1H), 1.15 (dq, *J* = 5.6, 0.8 Hz, 4H).

### Example 132: 6-amino-2-(3,5-dichloro-4-((2-cyclobutyl-4-methylquinolin-6-yl)oxy)phenyl)-1,2,4-triazi ne-3,5(2H,4H)-dione

By using the compound of example 118 as raw material, the method in example 58 was used to afford 6-amino-2-(3,5-dichloro-4-((2-cyclobutyl-4-methylquinolin-6-yl)oxy)phenyl)-1,2,4-triazine-3,5(2H,4H)-dione M/Z = 484.21(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.7 Hz, 1H), 7.18 - 7.11 (m, 2H), 5.17 (s, 2H), 3.08 - 3.01 (m, 1H), 2.71 (s, 3H), 2.03 - 1.80 (m, 6H), 1.83 - 1.69 (m, 1H).

### Example 133: 6-amino-2-(3,5-dichloro-4-((2-cyclopentyl-4-methylquinolin-6-yl)oxy)phenyl)-1,2,4-triazi ne-3,5(2H,4H)-dione

By using the compound of example 119 as raw material, the method in example 58 was used to afford 6-amino-2-(3,5-dichloro-4-((2-cyclopentyl-4-methylquinolin-6-yl)oxy)phenyl)-1,2,4-triazine -3,5(2H,4H)-dione M/Z = 498.14(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.8 Hz, 1H), 7.14 (dd, *J* = 8.1, 1.9 Hz, 1H), 7.02 (d, *J* = 1.1 Hz, 1H), 5.17 (s, 2H), 3.20 - 3.12 (m, 1H), 2.71 (s, 3H), 1.91 - 1.67 (m, 7H).

### Example 134: 6-amino-2-(3,5-dichloro-4-((2-cyclohexyl-4-methylquinolin-6-yl)oxy)phenyl)-1,2,4-triazi ne-3,5(2H,4H)-dione

By using the compound of example 120 as raw material, the method in example 58 was used to afford 6-amino-2-(3,5-dichloro-4-((2-cyclohexyl-4-methylquinolin-6-yl)oxy)phenyl)-1,2,4-triazine-3,5(2H,4H)-dione M/Z = 512.29(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.8 Hz, 1H), 7.14 (dd, *J* = 8.1, 1.9 Hz, 1H), 7.02 (d, *J* = 1.1 Hz, 1H), 5.17 (s, 2H), 2.96 - 2.88 (m, 1H), 2.71 (s, 3H), 1.77 - 1.60 (m, 7H), 1.58 - 1.45 (m, 4H), 1.47 - 1.35 (m, 1H).

### Example 135: 6-amino-2-(3,5-dichloro-4-((2-(4,4-dimethylcyclohexyl)-4-methylquinolin-6-yl)oxy)phen yl)-1,2,4-triazine-3,5(2H,4H)-dione

By using the compound of example 121 as raw material, the method in example 58 was used to afford 6-amino-2-(3,5-dichloro-4-((2-(4,4-dimethylcyclohexyl)-4-methylquinolin-6-yl)oxy)phenyl)-1,2,4-triazine-3,5(2H,4H)-dione M/Z = 540.35(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.8 Hz, 1H), 7.14 (dd, *J* = 8.2, 1.9 Hz, 1H), 7.02 (d, *J* = 0.9 Hz, 1H), 5.17 (s, 2H), 2.94 - 2.86 (m, 1H), 2.71 (s, 3H), 1.88 (dddd, *J* = 12.5, 9.9, 7.2, 5.5 Hz, 2H), 1.79 - 1.68 (m, 2H), 1.54 (ddd, *J* = 12.4, 9.7, 7.1 Hz, 2H), 1.44 (ddd, *J* = 12.5, 9.9, 7.2 Hz, 2H), 0.86 (s, 5H).

### Example 136: 6-amino-2-(3,5-dichloro-4-((2-(4,4-difluorocyclohexyl)-4-methylquinolin-6-yl)oxy)phenyl )-1,2,4-triazine-3,5(2H,4H)-dione

By using the compound of example 122 as raw material, the method in example 58 was used to afford 6-amino-2-(3,5-dichloro-4-((2-(4,4-difluorocyclohexyl)-4-methylquinolin-6-yl)oxy)phenyl)-1,2,4-triazine-3,5(2H,4H)-dione M/Z = 548.27(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.8 Hz, 1H), 7.14 (dd, *J* = 8.1, 1.9 Hz, 1H), 7.02 (d, *J* = 0.9 Hz, 1H), 5.17 (s, 2H), 3.05 - 2.96 (m, 1H), 2.71 (s, 3H), 2.15 - 1.86 (m, 9H).

### Example 137: 6-amino-2-(3,5-dichloro-4-((4-methyl-2-(4-(trifluoromethyl)cyclohexyl)quinolin-6-yl)oxy )phenyl)-1,2,4-triazine-3,5(2H,4H)-dione

By using the compound of example 123 as raw material, the method in example 58 was used to afford 6-amino-2-(3,5-dichloro-4-((4-methyl-2-(4-(trifluoromethyl)cyclohexyl)quinolin-6-yl)oxy)ph enyl)-1,2,4-triazine-3,5(2H,4H)-dione M/Z = 580.29(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.7 Hz, 1H), 7.14 (dd, *J* = 8.2, 1.9 Hz, 1H), 7.02 (d, *J* = 0.9 Hz, 1H), 5.17 (s, 2H), 2.96 - 2.88 (m, 1H), 2.71 (s, 3H), 2.37 - 2.25 (m, 1H), 2.18 - 2.06 (m, 2H), 2.00 - 1.81 (m, 4H), 1.78 - 1.67 (m, 2H).

### Example 138: 6-amino-2-(4-(2-(bicyclo[2.2.1]heptan-2-yl)-4-methylquinolin-6-yl)oxy)-3,5-dichlorophen yl)-1,2,4-triazine-3,5(2H,4H)-dione

By using the compound of example 124 as raw material, the method in example 58 was used to afford 6-amino-2-(4-(2-(bicyclo[2.2.1]heptan-2-yl)-4-methylquinolin-6-yl)oxy)-3,5-dichlorophenyl) -1,2,4-triazine-3,5(2H,4H)-dione M/Z = 524.30(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.8 Hz, 1H), 7.14 (dd, *J* = 8.2, 1.9 Hz, 1H), 7.11 (d, *J* = 0.9 Hz, 1H), 5.17 (s, 2H), 2.88 (dt, *J* = 6.0, 4.3 Hz, 1H), 2.71 (s, 3H), 2.26 (tt, *J* = 5.3, 3.6 Hz, 1H), 2.13 (dp, *J* = 6.0, 3.8 Hz, 1H), 2.01 (ddd, *J* = 12.5, 5.3, 4.5 Hz, 1H), 1.76 - 1.69 (m, 1H), 1.72 - 1.66 (m, 1H), 1.69 - 1.63 (m, 2H), 1.66 - 1.59 (m, 1H), 1.63 - 1.54 (m, 1H), 1.57 - 1.49 (m, 1H), 1.47 - 1.38 (m, 1H).

### Example 139: 6-amino-2-(3,5-dichloro-4-((4-methyl-2-(3-methylcyclohexyl)quinolin-6-yl)oxy)phenyl)-1 ,2,4-triazine-3,5(2H,4H)-dione

By using the compound of example 125 as raw material, the method in example 58 was used to afford 6-amino-2-(3,5-dichloro-4-((4-methyl-2-(3-methylcyclohexyl)quinolin-6-yl)oxy)phenyl)-1,2, 4-triazine-3,5(2H,4H)-dione M/Z = 526.32(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.8 Hz, 1H), 7.14 (dd, *J* = 8.1, 1.9 Hz, 1H), 7.12 - 7.09 (m, 1H), 5.17 (s, 2H), 2.99 - 2.90 (m, 1H), 2.71 (s, 3H), 1.91 - 1.82 (m, 1H), 1.78 - 1.49 (m, 6H), 1.47 - 1.32 (m, 3H), 0.93 (d, *J* = 6.2 Hz, 3H).

### Example 140: 6-amino-2-(3,5-dichloro-4-((4-methyl-2-(2-methylcyclohexyl)quinolin-6-yl)oxy)phenyl)-1 ,2,4-triazine-3,5(2H,4H)-dione

By using the compound of example 126 as raw material, the method in example 58 was used to afford 6-amino-2-(3,5-dichloro-4-((4-methyl-2-(2-methylcyclohexyl)quinolin-6-yl)oxy)phenyl)-1,2, 4-triazine-3,5(2H,4H)-dione M/Z = 526.32(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.7 Hz, 1H), 7.14 (dd, *J* = 8.1, 1.9 Hz, 1H), 7.03 (s, 1H), 5.17 (s, 2H), 2.71 (s, 3H), 2.54 (dtd, *J* = 7.2, 5.9, 1.5 Hz, 1H), 2.21 (dddd, *J* = 12.6, 9.5, 6.8, 5.9 Hz, 1H), 1.99 - 1.85 (m, 2H), 1.74 - 1.40 (m, 5H), 1.35 (ddt, *J* = 11.8, 8.2, 5.8 Hz, 1H), 0.95 (dd, *J* = 6.8, 1.5 Hz, 3H).

### Example 141: 6-amino-2-(3,5-dichloro-4-((4-methyl-2-(4-methylcyclohexyl)quinolin-6-yl)oxy)phenyl)-1 ,2,4-triazine-3,5(2H,4H)-dione

By using the compound of example 127 as raw material, the method in example 58 was used to afford 6-amino-2-(3,5-dichloro-4-((4-methyl-2-(4-methylcyclohexyl)quinolin-6-yl)oxy)phenyl)-1,2, 4-triazine-3,5(2H,4H)-dione M/Z = 526.32(M+1)¹H NMR (500 MHz, Chloroform-*d*) δ 9.74 (s, 1H), 7.85 (d, *J* = 8.2 Hz, 1H), 7.67 (s, 2H), 7.48 (d, *J* = 1.7 Hz, 1H), 7.14 (dd, *J* = 8.2, 1.9 Hz, 1H), 7.02 (d, *J* = 0.9 Hz, 1H), 5.17 (s, 2H), 2.93 - 2.84 (m, 1H), 2.71 (s, 3H), 1.88 (ddt, *J* = 12.0, 8.8, 5.9 Hz, 2H), 1.78 (ddt, *J* = 12.3, 8.6, 6.0 Hz, 2H), 1.60 (ddt, *J* = 12.0, 8.6, 5.9 Hz, 2H), 1.54 - 1.44 (m, 1H), 1.39 - 1.29 (m, 2H), 0.92 (d, *J* = 6.1 Hz, 3H).

### Example 142: 6-amino-2-(3,5-dichloro-4-((4-methyl-2-(3,3,5,5-tetramethylcyclohexyl)quinolin-6-yl)oxy )phenyl)-1,2,4-triazine-3,5(2H,4H)-dione

By using the compound of example 128 as raw material, the method in example 58 was used to afford 6-amino-2-(3,5-dichloro-4-((4-methyl-2-(3,3,5,5-tetramethylcyclohexyl)quinolin-6-yl)oxy)ph enyl)-1,2,4-triazine-3,5(2H,4H)-dione M/Z = 568.40(M+1)¹H NMR (500 MHz, Chloroform-*d*) δ 7.67 (s, 1H), 7.48 (d, *J* = 1.7 Hz, 0H), 7.20 - 7.11 (m, 1H), 5.17 (s, 1H), 3.15 - 3.07 (m, 0H), 2.71 (s, 1H), 1.86 (dd, *J* = 12.3, 5.5 Hz, 1H), 1.75 (dd, *J* = 12.3, 5.5 Hz, 1H), 0.94 (s, 2H), 0.89 (s, 2H).

### Biological assay

### 1.1 Evaluation of in vitro THR β agonist activity at the molecular level

At the molecular level, the binding ability of the compound to thyroid hormone receptor β (THR β) was measured by AlphaScreen detection. Firstly, recombinant GST-THR β fusion proteins were constructed, followed by compound-receptor binding reactions in 384-well plates. A mixture of recombinant protein, agonist, co-regulator, AlphaScreen^{®} acceptor microbeads and AlphaScreen^{®} donor microbeads was reacted in Tris-HCl (pH 7.4) buffer, and if the compounds were able to bind to the acceptor protein, the relative positions of the AlphaScreen^{®} acceptor beads and AlphaScreen^{®} donor beads would change, and fluorescence of a specific wavelength would emitt. The fluorescence signal intensity was measured by a fluorescence detector Envision to characterize the binding activity of the compounds to THPβ, and the compound EC₅₀ was calculated by software Graphpad Prism 7.

### 1.2 Evaluation of in vitro THR β agonist activity at the cellular level

To detect their binding activity at the cellular level, FH-THRα/β expression plasmid, pal-luciferase reporter plasmid and RL-TK plasmid were utilized, and all three were co-transfected into 293T cells and then inoculated in 96-well flat-bottomed microplates, and the cells were cultured for 24 h to ensure the expression of the plasmids. The TRα/β agonist to be tested was added and detected after 18 h using the Dual-Luciferase Reporter Assay System with firefly luciferase as the reporter gene and sea kidney luciferase as the internal reference gene. The efficiency of the compounds to activate TRα/β receptors can be reflected by the intensity of the biofluorescence detected by the fluorescence detector Envision.

### 1.3 Pharmacokinetic studies

The preliminary pharmacokinetic studies in mice or rats of the target compounds with excellent activity obtained from the above studies were conducted to investigate the pharmacokinetic properties of the compounds under oral, intraperitoneal and intravenous administration in detail, and to calculate the parameters of *in vivo* exposure (AUC), peak concentration (Cₘₐₓ), time to peak (Tₘₐₓ), half-life period (T_{1/2}) and bioavailability (F), which would provide a clue for further structural modification and optimization of the compounds, as well as a basis for in vivo dosage design.

### 1.4 In vivo efficacy evaluation

### 1.4.1 Acute pharmacodynamic evaluation

Mice or rats were used for single oral administration, and liver and heart were taken at 3 time points after administration to detect the expression of THRβ receptor downstream genes (DIO1, ME, CYP7α1, LDLR) in the target organ-liver, and to detect the expression of THRβ receptor downstream genes (DIO1, MHCα) in the organ involved in toxicity-heart.

### 1.4.1 Chronic pharmacodynamic evaluation

NAFLD model was established using high fat diet in obob mice and NASH model was established by high-fat, high-fructose diet. TC and TG levels in the liver were detected after long-term administration of the test compounds or the positive compound MGL-3196 for 3 weeks.

### 1.5 In vivo safety evaluation

Normal SD rats, which underwent thyroidectomy and recovered for 1-2 weeks, were grouped by body weight and then given the test compounds or the positive compound MGL-3196. Cardiac tissues were taken after euthanasia 6 h after the administration of the drug to examine the expression of the αMHC gene in the heart after single dose of the drug.

### Experimental results

### 2.1 In vitro THRβ agonistic activity and selectivity testing

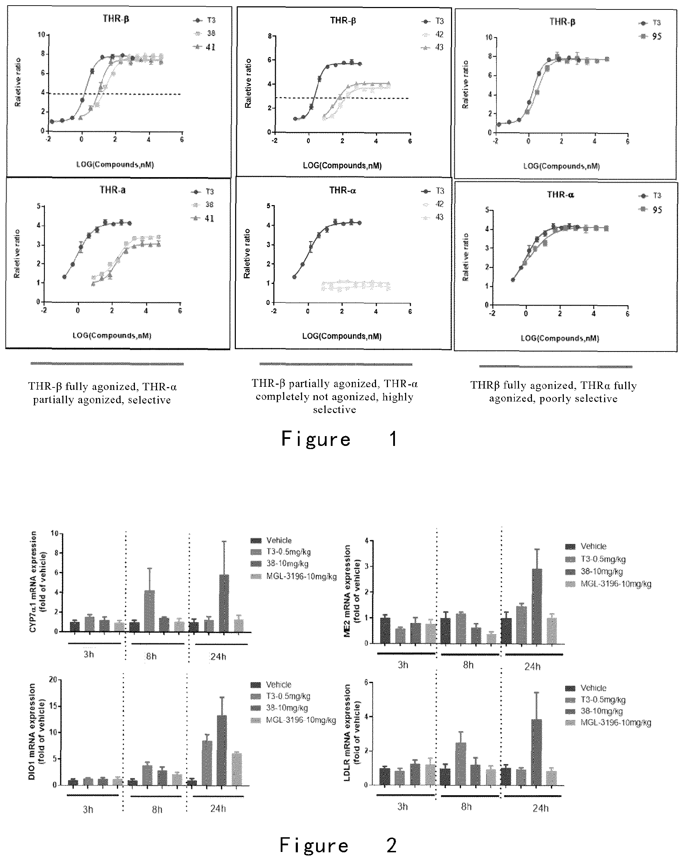

The above synthesized compounds were initially tested for THRβ agonistic activity in vitro at molecular level and cellular level respectively. Several compounds were found to have good THRβ agonist activity and good selectivity for THRα, wherein the EC50 value of compounds 38 and 95 were 13.2 nM and 2.5 nM, respectively (e.g., Table 1), which were 2.5-fold and 39 fold higher than that of the phase III clinical drug Resmetirom (MGL-3196)(EC50 = 129.5 nM). There are now three classes of agonists obtained from *in vitro* activity evaluation as well as conformational relationship studies: 1) THRβ fully agonized, THRα partially agonized, selective, 2) THRβ partially agonized, THRα not agonized, highly selective, and 3) THRβ fully agonized, THRα fully agonized, poorly selective. The results of molecular experiments were shown in figure 1.

Subsequently, the agonistic activity of this class of compounds on THRβ and THRα was tested at cellular level, and the results were shown in figure 2. The cellular results of this class of compounds were consistent with the molecular results, and in the cellular experiments, the cell EC₅₀ values of the compounds 2, 3, and 38 were found to be 1026 nM, 911 nM, and 165.9 nM, respectively, which indicated significant agonistic activity compared to that of MGL-3196 as 3415 nM. Compound 2 and compound 3 were particularly prominent, with almost complete inactivation of the THRα receptor and a selectivity of more than 1,000-fold, much better than 17-fold of MGL-3196, and meanwhile possessed the characteristics of partial agonists. The selectivity of compound 38 reached 40 times, significantly better than that of MGL-3196, and maintained complete agonist activity. This class of compounds are highly promising for development.

Among them, compounds 3, 42 and 43 are partial agonists with the agonistic activity on THRβ less than 70% along with good selectivity, suggesting that this class of compounds were THRβ partial agonists with good THRα selectivity.

**Table 1 Compound activity data obtained from in vitro molecular evaluation system**

| **Compound** | **THR-β** | | **THR-α** | | **Selectivity** |
|---|---|---|---|---|---|
| | **EC₅₀(nM)** | **THR-β Rel % Activity of T3** | **EC₅₀(nM)** | **THR-α Rel % Activity of T3** | |
| **T3** | 3.4 | 100.0 | 1.8 | 100.0 | 1 |
| **MGL3196** | 129.5 | 88.5 | 1251.2 | 74.0 | 17 |
| **2** | 60.3 | 74.0 | 776.5 | 59.7 | 25 |
| **3** | 76.9 | 55.2 | >50000 | - | >1000 |
| **15** | 71.7 | 84.3 | 279.6 | 62.5 | 3.9 |
| **17** | 295.8 | 73.7 | 1244.0 | 57.6 | 4.2 |
| **19** | 379.1 | 71.8 | >50000 | | - |
| **21** | 173.6 | 84.2 | 515.2 | 56.0 | 2.9 |
| **38** | 13.2 | 103.4 | 208.5 | 78.1 | 40 |
| **42** | 93.3 | 62.3 | >50000 | - | >1000 |
| **43** | 61.4 | 67.2 | >50000 | - | >1000 |
| **60** | 8.3 | 83.0 | 57.2 | 62.7 | 6.89 |
| **95** | 2.5 | 122.8 | 12.2 | 98.3 | 12 |
| **99** | 31.5 | 76.27 | 247.8 | 67.55 | 7.85 |
| **134** | 119.6 | 92.3 | 429.3 | 38.4 | 4.2 |

**Table 2 Compound activity data obtained from in vitro cellular evaluation system**

| **Compound** | **THR-β** | | **THR-α** | | **Selectivity** |
|---|---|---|---|---|---|
| | **EC₅₀(nM)** | **THR-β Rel % Activity of T3** | **EC₅₀(nM)** | **THR-α Rel % Activity of T3** | |
| **T3** | 2.3 | 100.0 | 0.6 | 100.0 | 1 |
| **MGL3196** | 3415.1 | 67.1 | 10446.5 | 32.6 | 17 |
| **2** | 1026.4 | 45.7 | > 10000 | - | > 1000 |
| **3** | 911.7 | 55.2 | > 50000 | - | > 1000 |
| **38** | 165.9 | 104.2 | 1633.2 | 68.1 | 52 |
| **42** | 354.2 | 60.1 | > 50000 | - | > 1000 |
| **43** | 586.04 | 30.31 | > 50000 | - | > 1000 |
| **95** | 5.6 | 113.1 | 380 | 136.8 | 350 |

### 2.2 Pharmacokinetic test

Based on the favorable *in vitro* TRβ agonist activity and selectivity of compound 38, compound **38** was selected for preliminary evaluation of druggability and studies on pharmacokinetic properties in mice. As shown in Table 3, compound 38 had good pharmacokinetic properties with a half-life period up to 4.44 hours, high *in vivo* exposure of 156427 h*ng/mL and bioavailability of 75.8%.

**Table 3. 38 Pharmacokinetic Data**

| Admin. | Animal No. | T_{1/2} | Tₘₐₓ | Cₘₐₓ | AUCₗₐₛₜ | CL_{_obs} | Vss_{_obs} | F |
|---|---|---|---|---|---|---|---|---|
| | | (h) | (h) | (ng/mL) | (h*ng/mL) | (mL/min/kg) | (mL/kg) | (%) |
| p.o. | 1 | 5.01 | 0.50 | 25987 | 161847 | - | - | 75.8 |
| | 2 | 4.18 | 1.00 | 23595 | 176967 | - | - | |
| | 3 | 4.13 | 1.00 | 19689 | 130466 | - | - | |
| | **Mean** | **4.44** | **0.83** | **23090** | **156427** | | - | |
| | **SD** | **0.50** | **0.29** | **3179** | **23719** | - | - | |
| i.v. | 4 | 3.93 | - | - | 119063 | 0.691 | 220 | |
| | 5 | 4.30 | - | 95351 | 95351 | 0.859 | 270 | |
| | 6 | 4.77 | - | 95119 | 95119 | 0.856 | 267 | |
| | **Mean** | **4.33** | - | - | **103178** | **0.802** | **252** | |
| | **SD** | **0.42** | **-** | **-** | **13758** | **0.096** | **28** | |

In order to further evaluate the preliminary druggability of the compound, tissue distribution studies in mice were carried out, as shown in **Table 4,** the tissue distribution data of compound 38 showed more distribution in the liver and less distribution in the heart, and the concentration of the drug in the liver was 23 times higher than that in the heart, which reflected a good hepatic targeting, suggesting that the cardiotoxicity of the compound was weak.

**Table 4. Oral administration of compound 38 in mice at 10 mg/kg, tissue distribution data**

| Animal No. | Time | Plasma (ng/mL) | Mean | Liver (ng/g) | Mean | Heart (ng/g) | Mean |
|---|---|---|---|---|---|---|---|
| 1 | 3 h | 9345 | 7864 | 4300 | 3668 | 189 | 154 |
| 2 | | 8371 | | 3778 | | 115 | |
| 3 | | 7102 | | 3241 | | 199 | |
| 4 | | 6639 | | 3353 | | 113 | |
| 5 | 8 h | 2473 | 3220 | 582 | 617 | 39.9 | 50.5 |
| 6 | | 4555 | | 895 | | 72.2 | |
| 7 | | 2005 | | 264 | | 26.4 | |
| 8 | | 3847 | | 729 | | 63.3 | |
| 9 | 24 h | 93.6 | 164 | 16.3 | 22.0 | BLQ | 0.00 |
| 10 | | 281 | | 29.5 | | BLQ | |
| 11 | | 192 | | 27.8 | | BLQ | |
| 12 | | 88.6 | | 14.5 | | BLQ | |

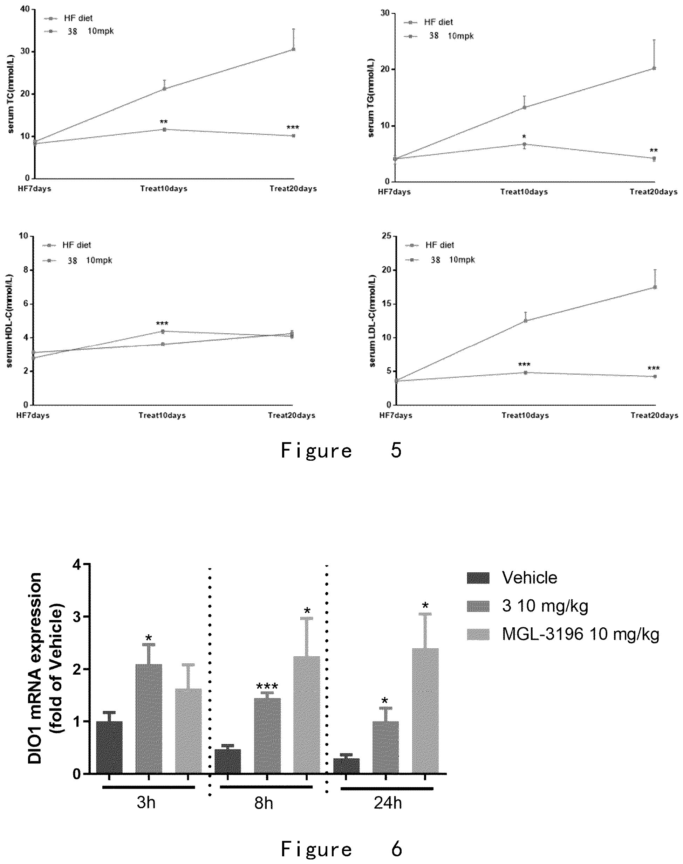

In addition, due to the favorable activity and complete TRβ selectivity presented by compound 3 in *in vitro* experiments, a preliminary druggability evaluation of compound 3 was also performed and a series of pharmacokinetic studies were also performed in mice, with the results shown in Figure 5. Compound 3 possessed excellent pharmacokinetic properties with half-life period up to 4.71 hours, *in vivo* exposure of 79,362 h*ng/mL and bioavailability of 36.3%.

**Table 5. Pharmacokinetic Data of Compound 3**

| Admin. | Animal No. | T_{1/2} | T_{max\} | Cₘₐₓ | AUCₗₐₛₜ | CL_{_obs} | VSS_{_obs} | F |
|---|---|---|---|---|---|---|---|---|
| | | (h) | (h) | (ng/mL) | (h*ng/mL) | (mL/min/kg) | (mL/kg) | (%) |
| p.o. | 1 | 4.31 | 1.00 | 9646 | 62498 | - | - | 36.3 |
| | 2 | 4.80 | 1.00 | 12800 | 100901 | - | - | |
| | 3 | 5.02 | 0.50 | 9108 | 74686 | - | - | |
| | **Mean** | 4.71 | 0.83 | 10518 | 79362 | - | - | |
| | **SD** | 0.36 | 0.29 | 1994 | 19624 | - | - | |
| i.v. | 4 | 6.40 | - | - | 117735 | 0.67 | 289 | |
| | 5 | 5.10 | - | - | 106173 | 0.76 | 253 | |
| | 6 | 6.62 | - | - | 104125 | 0.75 | 315 | |
| | **Mean** | 6.04 | - | - | 109344 | 0.73 | 286 | |
| | **SD** | 0.82 | - | - | 7338 | 0.05 | 31 | |

*In vivo* tissue distribution studies in mice were also performed for compound 3 to further evaluate its preliminary druggability and the results were shown in Table 6. It can be seen that the distribution of the compound in the liver was much larger than that in the heart, about 10 times, and that the distribution of compound 3 in the liver was very little, which posed a positive effect on the development of cardiotoxicity.

**Table 6. Oral administration of compound 38 in mice at 10 mg/kg, tissue distribution data**

| No. | Time (h) | 7/19/31 | 8/20/32 | 9/21/33 | Mean | SD |
|---|---|---|---|---|---|---|
| | 3.00 | 5452 | 5796 | 4534 | 5261 | 652 |
| plasma (ng/mL) | 8.00 | 3326 | 4487 | 2490 | 3434 | 1003 |
| | 24.0 | 165 | 335 | 412 | 304 | 126 |
| | 3.00 | 1883 | 1478 | 1383 | 1581 | 266 |
| Liver (ng/g) | 8.00 | 689 | 614 | 469 | 591 | 112 |
| | 24.0 | 23.3 | 39.5 | 56.7 | 39.8 | 16.7 |
| | 3.00 | 197 | 215 | 169 | 194 | 24 |
| Heart (ng/g) | 8.00 | 139 | 196 | 116 | 150 | 41 |
| | 24.0 | 6.36 | 13.7 | 20.5 | 13.5 | 7.1 |

### 2.3 In vivo pharmacological studies

The highly active and selective compound 38 was chose for acute pharmacodynamic evaluation *in vivo.* C57 mice were used to investigate the expression of THRβ receptor downstream genes in the target organ, the liver tissue, after a single administration, and the results showed that the positive control group of **T3** (an endogenous, highly active, non-selective thyroid hormone; 0.5 mg/kg) significantly up-regulated the expression of CYP7α1 and LDLR genes in the liver after 8 hours of administration; DIO1 gene expression in the liver was significantly up-regulated after 8 and 24 h of administration; compound **38** (10 mg/kg) significantly up-regulated the expression of CYP7α1, ME2, and LDLR genes in the liver after 24 h of administration; and DIO1 gene expression in the liver was significantly up-regulated after 8 and 24 h of administration, which was superior to that of MGL3196 at the same dose(see Figure 2).

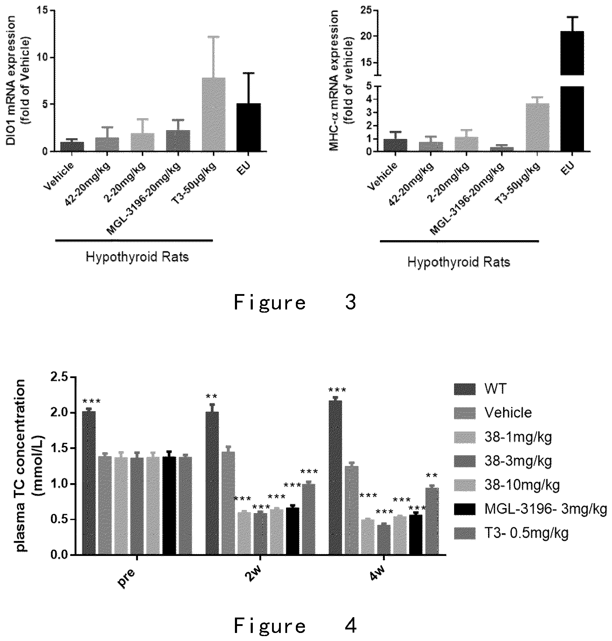

Downstream gene expression in the heart in thyroidectomized rats showed neither compound **38** nor MGL3196 had significant effects on cardiac DIO1 and MHCα genes (see Figure 3), suggesting that compound 38 was of high safety for the heart.

Pharmacodynamic evaluation of compounds in CDAHFD feed-induced NAFLD model mice showed that plasma TC levels were decreased in both test compound and positive compound, MGL-3196, after 2 and 4 weeks of long-term administration, and both the 1 mg/kg and 10 mg/kg dosage groups of Compound 38 decreased the plasma TC levels (see Figure 4).

Acute pharmacodynamic evaluation of compounds in high-fat feed -induced golden hamster model mice showed that compound 38 caused a significant decrease in serum TC/TG levels and LCL-C levels at a dose of 10 mg/kg after 21 days of test compound administration (see Figure 5).

A preliminary *in vivo* polar pharmacodynamic evaluation of the partial agonist compound **3** was also performed, with the experimental method the same as for compound **38.** The results showed that compound **3** significantly up-regulated hepatic DIO1 gene expression 3h after administration (see Figure 6), with a fast accentuation of the drug effect, and the effect began to diminish after 8 h of administration, but still effective compared with the negative control group, which may have different application scenarios compared with MGL-3196.

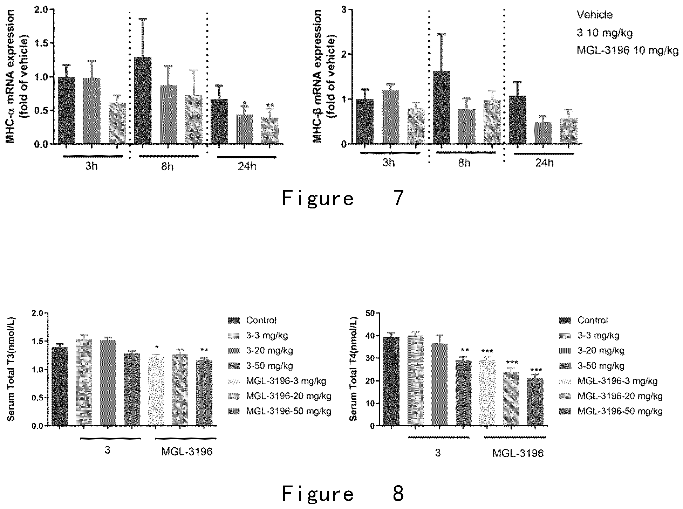

In C57 mice, a preliminary assessment of the safety of Compound 3 after administration found that Compound 3 did not result in upregulation of MHCα in the heart, and the overall trend was consistent with that of MGL-3196, indicating that compound 3 possessed high safety (Figure 7).

The inhibitory effect of compound 3 on the thyroid axis was verified in C57 mice, and the results were shown in Figure 8, where Compound 3 did not affect thyroid hormone levels when administered at high doses, suggesting a better safety compared with MGL-3196.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound of formula (I), or a stereoisomer, a deuterated compound, a solvate, a metabolite, a pharmaceutically acceptable salt, a eutectic or a prodrug thereof, 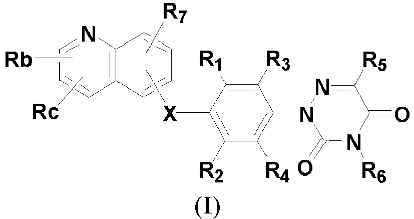
wherein, 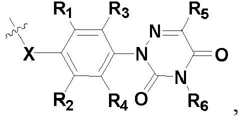 Rb, Rc and R₇ are independently located on any substitutable ring atom of the quinoline ring;
Rb and Rc are independently selected from the group consisting of: D, tritium, halogen, C1-C6 alkyl, C2-C6 alkenyl, C2-C6 alkynyl, C1-C6 alkoxy, C3-C8 cycloalkyl, 3-8-membered heterocyclyl, phenyl, C5-C7 heteroaryl, and C5-C6 substituted heteroaryl; and Rb and Rc are different substitutents;
R₇ is one or more (such as 1, 2, 3, or 4) substituents located on the benzene of the quinoline ring and is selected from the group consisting of: H, deuterium, tritium, halogen, C1-C6 alkyl, and C1-C6 haloalkyl;
X is selected from the group consisting of: -O-, -CH₂-, -C(=O)-, -CH(OH)-, and -S-;
R₁, R₂, R₃ and R₄ are independently selected from the group consisting of: H, D, F, Cl, Br, I, -CN, -OH, -NO₂, -OR₁ₐ, -NR_{1b}R_{1c}, -SR_{1b}, C1-C6 alkyl, C1-C6 alkoxy, C2-C6 alkenyl, C2-C6 alkynyl, C3-C8 cycloalkyl, 3-8-membered heterocycloalkyl, C5-C6aryl, and 5-6-membered heteroaryl;
R₅ is selected from the group consisting of: H, D, F, Cl, Br, I, -CN, -OH, -NO₂, -OR₁ₐ, -NR_{1b}R_{1c}, -SR_{1b}, -S(O)R₁ₐ, -S(O)₂R₁ₐ, -S(O)NR₁ₐR_{1b}, -S(O)₂NR₁ₐR_{1b}, -NHS(O)₂R₁ₐ, -C(O)R₁ₐ, -OC(O)R₁ₐ, -C(O)OR_{1b}, -C(O)NR_{1b}R_{1c}, -NHC(O)R₁ₐ, C1-C6 alkyl, C1-C6 alkoxy, C2-C6 alkenyl, C2-C6 alkynyl, C3-C8 cycloalkyl, 3-8-membered heterocyclyl, C5-C6 aryl and 5-6-membered heteroaryl;
R₆ is selected from the group consisting of: H, D, tritium, halogen, C1-C6 alkyl, amino substituted C1-C6 alkyl, C3-C8 cycloalkyl, 3-8-membered heterocyclyl, -SR_{1b}, -S(O)R₁ₐ, -S(O)₂R₁ₐ, -S(O)NR₁ₐR_{1b}, -C(O)R₁ₐ, -C(O)OR_{1b}, and -C(O)NR_{1b}R_{1c};
unless otherwise specified, each alkyl, alkoxy, alkenyl, alkynyl, cycloalkyl, heterocyclyl, aryl, and heteroaryl mentioned above may optionally have 1-3 substituents selected from the group consisting of: D, tritium, halogen, -CN, -OH, oxo-, -OR₁ₐ, -NR_{1b}R_{1c}, -SR_{1b}, -S(O)R₁ₐ, -S(O)₂R₁ₐ, -S(O)NR₁ₐR_{1b}, -S(O)₂NR₁ₐR_{1b}, - NHS(O)₂R₁ₐ, -C(O)R₁ₐ, - OC(O)R₁ₐ, -C(O)OR_{1b}, - C(O)NR_{1b}R_{1c}, -NHC(O)R₁ₐ, C1-C6 alkyl, and C1-C6 haloalkyl;
R₁ₐ, R_{1b} and R_{1c} are each independently selected from: H, D, C1-C6 alkyl, C2-C6 alkenyl, C2-C6 alkynyl, C3-C8 cycloalkyl, 3-8-membered heterocycloalkyl, C5-C6 aryl and 5-6-membered heteroaryl.

2. The compound according to claim 1, or a stereoisomer, a deuterated compound, a solvate, a metabolite, a pharmaceutically acceptable salt, a eutectic or a prodrug thereof, wherein, Rb is selected from the group consisting of: C1-C6 haloalkyl, C3-C8 cycloalkyl, 3-8-membered heterocyclyl, phenyl, and C5-C7 heteroaryl; and the phenyl or heteroaryl is optionally substituted by 1-3 substituents selected from the group consisting of: D, tritium, halogen, -CN, -OH, oxo-, -OR₁ₐ, -NR_{1b}R_{1c}, -C(O)R₁ₐ, -C(O)OR_{1b}, -C(O)NR_{1b}R_{1c}, C1-C6 alkyl, and C1-C6 haloalkyl;
Rc is selected from the group consisting of: halogen, C1-C6 alkyl, C1-C6 alkoxy, C1-C6 haloalkyl, and C1-C6 deuterated alkyl.

3. The compound according to claim 1, or a stereoisomer, a deuterated compound, a solvate, a metabolite, a pharmaceutically acceptable salt, a eutectic or a prodrug thereof, wherein, the compound of formula (I) has a structure shown in formula (Ia) as follows:

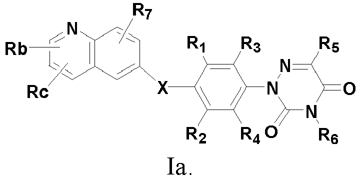

4. The compound according to claim 1, or a stereoisomer, a deuterated compound, a solvate, a metabolite, a pharmaceutically acceptable salt, a eutectic or a prodrug thereof, wherein, the compound of formula I has the structure shown in formula Ib or formula Ic as follows:

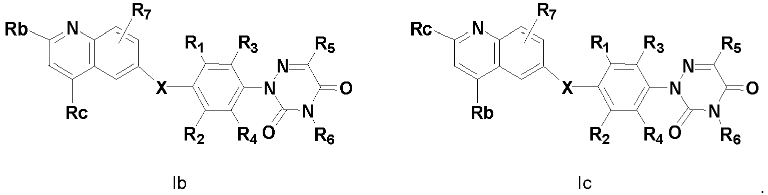

5. The compound according to claim 1, or a stereoisomer, a deuterated compound, a solvate, a metabolite, a pharmaceutically acceptable salt, a eutectic or a prodrug thereof, wherein, R₁, R₂, R₃, and R₄ are independently selected from H, D, halogen and C1-C4 alkyl.

6. The compound according to claim 1, or a stereoisomer, a deuterated compound, a solvate, a metabolite, a pharmaceutically acceptable salt, a eutectic or a prodrug thereof, wherein, R₅ is selected from the group consisting of: H, D, halogen, -CN, and NHR_{1b}; wherein, R_{1b} is selected from H, D, C1-C6 alkyl, C2-C6 alkenyl, and C2-C6 alkynyl; R₆ is H or D.

7. The compound according to claim 1, comprising a stereoisomer, a deuterated compound, a solvate, a metabolite, a pharmaceutically acceptable salt, a eutectic or a prodrug thereof:
| No. | Structure | name |
|---|---|---|
| 1 | 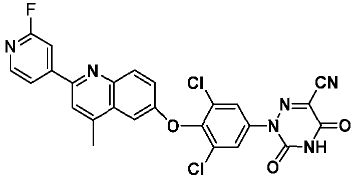 | 2-(3,5-Dichloro-4-((2-(2-fluoropyridin-4-yl) -4-methylquinolin-6-yl)oxy)phenyl) -3,5-dioxo-2,3,4,5-tetrahydro-1,2,4-tri azine-6-carbonitrile |
| 2 | 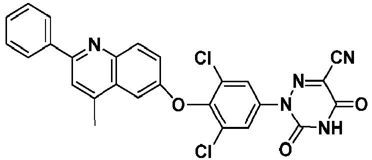 | 2-(3,5-Dichloro-4-((2-phenyl-4-methylq uinolin-6-yl)oxy)phenyl) -3,5-dioxo-2,3,4,5-tetrahydro-1,2,4-triazine-6-carbonitrile |
| 3 | 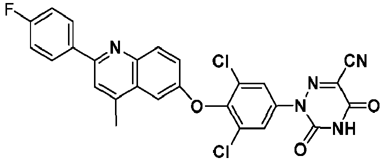 | 2-(3,5-Dichloro-4-((2-(4-fluorophenyl) -4-methylquinolin-6-yl)oxy)phenyl) -3,5-dioxo-2,3,4,5-tetrahydro-1,2,4-triazine-6-carbonitrile |
| 4 | 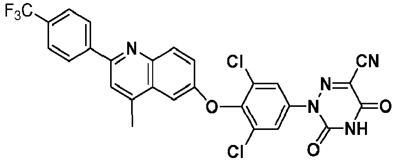 | 2-(3,5-Dichloro-4-((2-(4-trifluoromethylphenyl)-4-methylqu inolin-6-yl)oxy)phenyl) -3,5-dioxo-2,3,4,5-tetrahydro-1,2,4-triazine-6-carbonitrile |
| 5 | 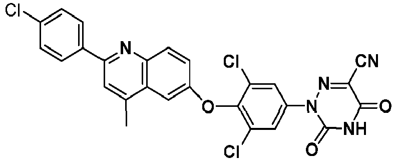 | 2-(3,5-Dichloro-4-((2-(4-chlorophenyl) -4-methylquinolin-6-yl)oxy)phenyl) -3,5-dioxo-2,3,4,5-tetrahydro-1,2,4-triazine-6-carbonitrile |
| 6 | 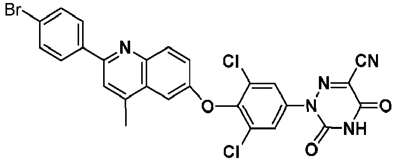 | 2-(3,5-Dichloro-4-((2-(4-bromophenyl) -4-methylquinolin-6-yl)oxy)phenyl) -3,5-dioxo-2,3,4,5-tetrahydro-1,2,4-triazine-6-carbonitrile |
| 7 | 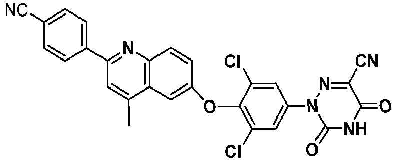 | 2-(3,5-Dichloro-4-((2-(4-cyanophenyl) -4-methylquinolin-6-yl)oxy)phenyl) -3,5-dioxo-2,3,4,5-tetrahydro-1,2,4-triazine-6-carbonitrile |
| 8 | 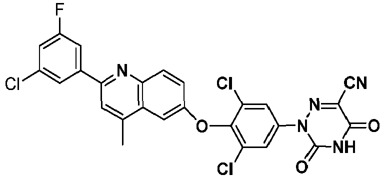 | 2-(3,5-Dichloro-4-((2-(3-chloro-5-fluorophenyl) -4-methylquinolin-6-yl)oxy)phenyl) -3,5-dioxo-2,3,4,5-tetrahydro -1,2,4-triazine-6-carbonitrile |
| 9 | 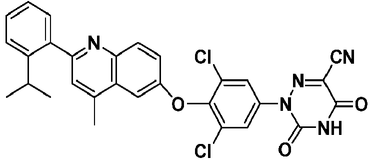 | 2-(3,5-Dichloro-4-((2-(2-isopropylphen yl)-4-methylquinolin-6-yl)oxy) phenyl)-3,5-dioxo-2,3,4,5-tetrahydro-1,2,4-triazine-6-carbonitrile |
| 10 | 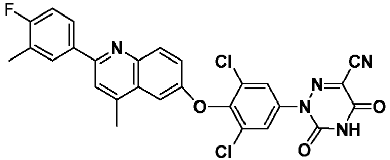 | 2-(3,5-Dichloro-4-((2-(4-fluoro-3-methylphenyl) -4-methylquinolin-6-yl)oxy)phenyl) -3,5-dioxo-2,3,4,5-tetrahydro -1,2,4-triazine-6-carbonitrile |
| 11 | 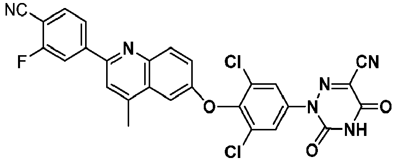 | 2-(3,5-Dichloro-4-((2-(4-cyano-3-fluorophenyl) -4-methylquinolin-6-yl)oxy)phenyl) -3,5-dioxo-2,3,4,5-tetrahydro--1,2,4-triazine-6-carbonitrile |
| 12 | 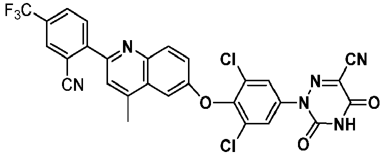 | 2-(3,5-Dichloro-4-((2-(2-cyano-4-fluorophenyl) -4-methylquinolin-6-yl)oxy)phenyl) -3,5-dioxo-2,3,4,5-tetrahydro--1,2,4-triazine-6-carbonitrile |
| 13 | 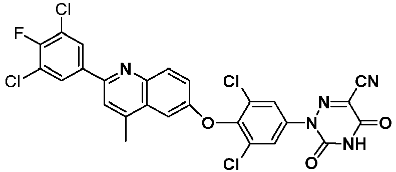 | 42-(3,5-Dichloro-4-((2-(4-fluoro-3,5-dichlorophenyl) -4-methylquinolin-6-yl)oxy)phenyl) -3,5-dioxo4,5-tetrahydro -1,2,4-triazine-6-carbonitrile |
| 14 | 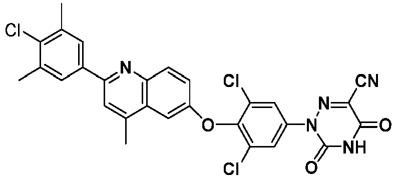 | 2-(3,5-Dichloro-4-((2-(4-chloro-3,5-dimethylphenyl) -4-methylquinolin-6-yl)oxy)phenyl) -3,5-dioxo4,5-tetrahydro -1,2,4-triazine-6-carbonitrile |
| 15 | 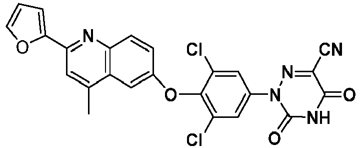 | 2-(3,5-Dichloro-4-((2-(furan-2-yl)-4-me thylquinolin-6-yl)oxy)phenyl) -3,5-dioxo-2,3,4,5-tetrahydro--1,2,4-triazine-6-carbonitrile |
| 16 | 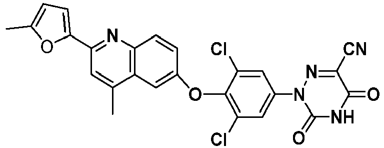 | 2-(3,5-Dichloro-4-((4-methyl-2-(5-methylfuran-2-yl)qui nolin-6-yl)oxy)phenyl) -3,5-dioxo-2,3,4,5-tetrahydro -1,2,4-triazine-6-carbonitrile |
| 17 | 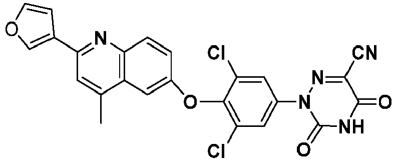 | 2-(3,5-Dichloro-4-((2-(furan-3-yl)-4-me thylquinolin-6-yl)oxy)phenyl) -3,5-dioxo-2,3,4,5-tetrahydro-1,2,4-tri azine-6-carbonitrile |
| 18 | 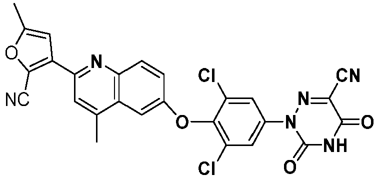 | 2-(3,5-Dichloro-4-((4-methyl-2-(2-cyano-5methylfuran-3-yl)quinolin-6-yl)oxy)phenyl) -3,5-dioxo-2,3,4,5-tetrahydro -1,2,4-triazine-6-carbonitrile |
| 19 | 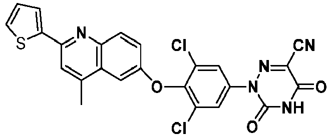 | 2-(3,5-Dichloro-4-((2-(thiophen-2-yl) -4-methylquinolin-6-yl)oxy)phenyl) -3,5-dioxo-2,3,4,5-tetrahydro -1,2,4-triazine-6-carbonitrile |
| 20 | 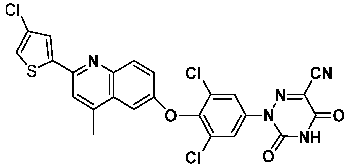 | 2-(3,5-Dichloro-4-((4-methyl-2-(4-chlorothiophen-2-yl)quinolin-6-yl) oxy)phenyl) -3,5-dioxo-2,3,4,5-tetrahydro -1,2,4-triazine-6-carbonitrile |
| 21 | 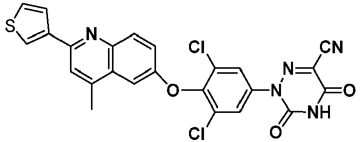 | 2-(3,5-Dichloro-4-((2-(thiophen-3-yl) -4-methylquinolin-6-yl)oxy)phenyl) -3,5-dioxo-2,3,4,5-tetrahydro -1,2,4-triazine-6-carbonitrile |
| 22 | 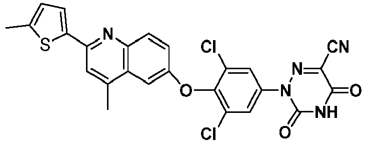 | 2-(3,5-Dichloro-4-((4-methyl-2-(5-methylthiophen-2-yl) quinolin-6-yl)oxy)phenyl) -3,5-dioxo-2,3,4,5-tetrahydro -1,2,4-triazine-6-carbonitrile |
| 23 | 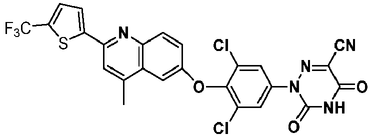 | 2-(3,5-Dichloro-4-((4-methyl-2-(5-trifluoromethylthiophen-2-yl)qui nolin-6-yl)oxy)phenyl) -3,5-dioxo-2,3,4,5-tetrahydro -1,2,4-triazine-6-carbonitrile |
| 24 | 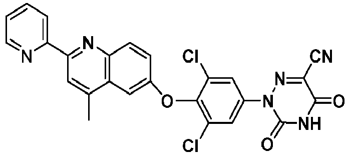 | 2-(3,5-Dichloro-4-((2-(pyridin-2-yl) -4-methylquinolin-6-yl)oxy)phenyl) -3,5-dioxo-2,3,4,5-tetrahydro-1,2,4-tri azine-6-carbonitrile |
| 25 | 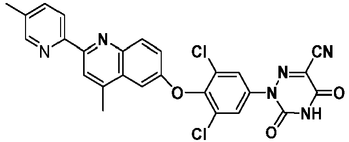 | 2-(3,5-Dichloro-4-((4-methyl-2-(5-methylpyridin-2-yl)quinolin-6-yl) oxy)phenyl) -3,5-dioxo-2,3,4,5-tetrahydro-1,2,4-tri azine-6-carbonitrile |
| 26 | 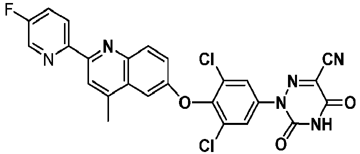 | 2-(3,5-Dichloro-4-((4-methyl-2-(5-fluoropyridin-2-yl)quinolin-6-yl) oxy) phenyl)-3,5-dioxo-2,3,4,5-tetrahydro-1,2,4-triazine-6-carbonitrile |
| 27 | 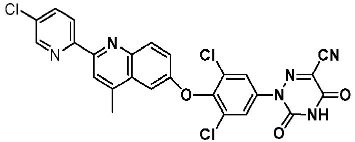 | 2-(3,5-Dichloro-4-((4-methyl-2-(5-chloropyridin-2-yl)quinolin-6-yl) oxy) phenyl)-3,5-dioxo-2,3,4,5-tetrahydro-1,2,4-triazine-6-carbonitrile |
| 28 | 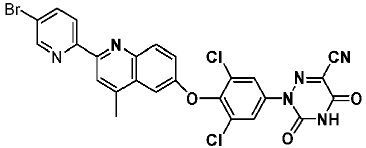 | 2-(3,5-Dichloro-4-((4-methyl-2-(5-bromopyridin-2-yl)quinolin-6-yl) oxy) phenyl)-3,5-dioxo-2,3,4,5-tetrahydro-1,2,4-triazine-6-carbonitrile |
| 29 | 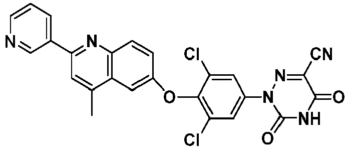 | 2-(3,5-Dichloro-4-((2-(pyridin-3-yl) -4-methylquinolin-6-yl)oxy)phenyl) -3,5-dioxo-2,3,4,5-tetrahydro-1,2,4-tri azine-6-carbonitrile |
| 30 | 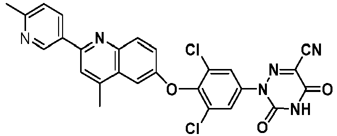 | 2-(3,5-Dichloro-4-((2-(6-methylpyridin-3-yl) -4-methylquinolin-6-yl)oxy)phenyl) -3,5-dioxo-2,3,4,5-tetrahydro-1,2,4-tri azine-6-carbonitrile |
| 31 | 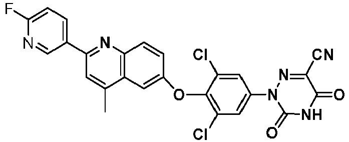 | 2-(3,5-Dichloro-4-((2-(6-fluoropyridin-3-yl) -4-methylquinolin-6-yl)oxy)phenyl) -3,5-dioxo-2,3,4,5-tetrahydro-1,2,4-tri azine-6-carbonitrile |
| 32 | 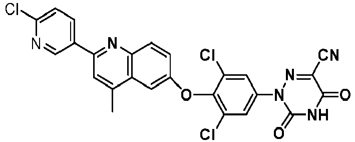 | 2-(3,5-Dichloro-4-((2-(6-chloropyridin-3-yl) -4-methylquinolin-6-yl)oxy)phenyl) -3,5-dioxo-2,3,4,5-tetrahydro-1,2,4-tri azine-6-carbonitrile |
| 33 | 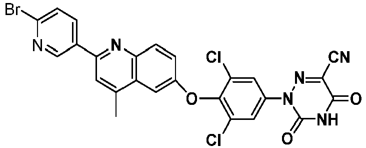 | 2-(3,5-Dichloro-4-((2-(6-bromopyridin-3-yl) -4-methylquinolin-6-yl)oxy)phenyl) -3,5-dioxo-2,3,4,5-tetrahydro-1,2,4-tri azine-6-carbonitrile |
| 34 | 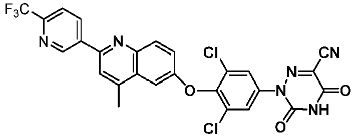 | 2-(3,5-Dichloro-4-((2-(6-trifluoromethylpyridin-3-yl) -4-methylquinolin-6-yl)oxy)phenyl) -3,5-dioxo-2,3,4,5-tetrahydro-1,2,4-tri azine-6-carbonitrile |
| 35 | 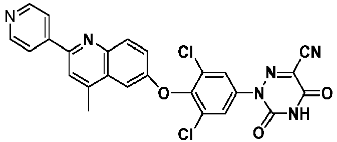 | 2-(3,5-Dichloro-4-((2-(pyridin-4-yl) -4-methylquinolin-6-yl)oxy)phenyl) -3,5-dioxo-2,3,4,5-tetrahydro-1,2,4-tri azine-6-carbonitrile |
| 36 | 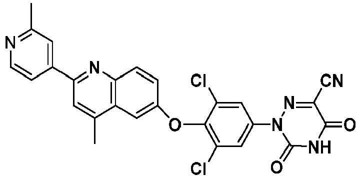 | 2-(3,5-Dichloro-4-((2-(2-methylpyridin-4-yl) -4-methylquinolin-6-yl)oxy)phenyl) -3,5-dioxo-2,3,4,5-tetrahydro-1,2,4-tri azine-6-carbonitrile |
| 37 | 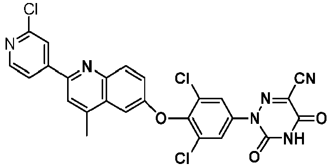 | 2-(3,5-Dichloro-4-((2-(2-chloropyridin-4-yl) -4-methylquinolin-6-yl)oxy)phenyl) -3,5-dioxo-2,3,4,5-tetrahydro-1,2,4-tri azine-6-carbonitrile |
| 38 | 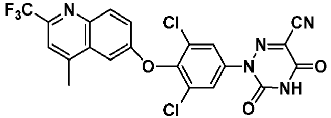 | 2-(3,5-Dichloro-4-((2-trifluoromethyl-4-methylquinolin-6-yl)oxy)phenyl) -3,5-dioxo-2,3,4,5-tetrahydro-1,2,4-triazine-6-carbonitrile |
| 39 | 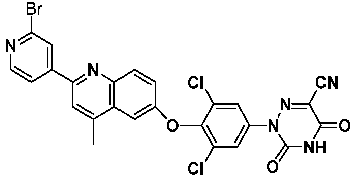 | 2-(3,5-Dichloro-4-((2-(2-bromopyridin-4-yl) -4-methylquinolin-6-yl)oxy)phenyl) -3,5-dioxo-2,3,4,5-tetrahydro-1,2,4-tri azine-6-carbonitrile |
| 40 | 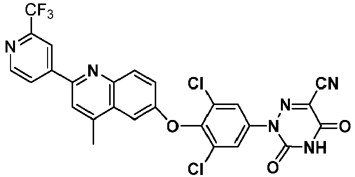 | 2-(3,5-Dichloro-4-((2-(2-trifluoromethylpyridin-4-yl) -4-methylquinolin-6-yl)oxy)phenyl) -3,5-dioxo-2,3,4,5-tetrahydro-1,2,4-tri azine-6-carbonitrile |
| 41 | 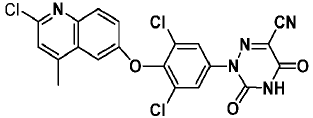 | 2-(3,5-Dichloro-4-((2-chloro-4-methylq uinolin-6-yl)oxy)phenyl) -3,5-dioxo-2,3,4,5-tetrahydro -1,2,4-triazine-6-carbonitrile |
| 42 | 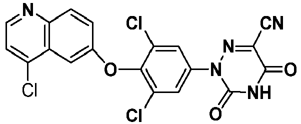 | 2-(3,5-Dichloro-4-((4-chloroquinolin-6-yl)oxy)phenyl) -3,5-dioxo-2,3,4,5-tetrahydro -1,2,4-triazine-6-carbonitrile |
| 43 | 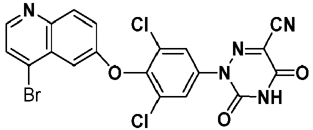 | 2-(3,5-Dichloro-4-((4-bromoquinolin-6-yl)oxy)phenyl) -3,5-dioxo-2,3,4,5-tetrahydro -1,2,4-triazine-6-carbonitrile |
| 44 | 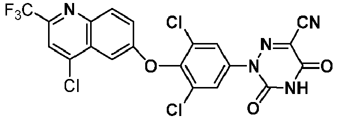 | 2-(3,5-Dichloro-4-((4-chloro-2-(trifluoromethyl)quinolin -6-yl)oxy)phenyl) -3,5-dioxo-2,3,4,5-tetrahydro--1,2,4-triazine-6-carbonitrile |
| 45 | 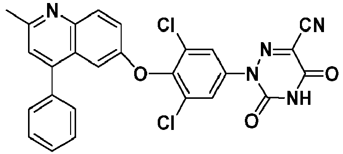 | 2-(3,5-Dichloro-4-((2-methyl -4-phenylquinolin-6-yl)oxy)phenyl -3,5-dioxo-2,3,4,5-tetrahydro -1,2,4-triazine-6-carbonitrile |
| 46 | 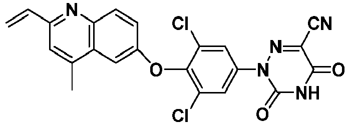 | 2-(3,5-Dichloro-4-((4-methyl-2-vinylquinolin-6-yl)oxy) phenyl)-3,5-dioxo-2,3,4,5-tetrahydro -1,2,4-triazine-6-carbonitrile |
| 47 | 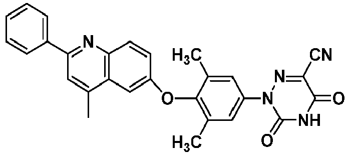 | 2-(3,5-Dimethyl-4-((4-methyl-2-phenyl quinolin-6-yl)oxy)phenyl -3,5-dioxo-2,3,4,5-tetrahydro-1,2,4-tri azine-6-carbonitrile |
| 48 | 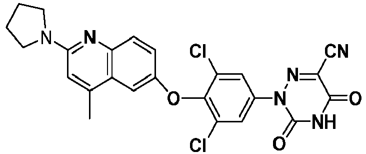 | 2-(3,5-Dichloro-4-((4-methyl-2-(pyrrolidin-1-yl)quinolin-6-yl)oxy) phenyl)-3,5-dioxo-2,3,4,5-tetrahydro -1,2,4-triazine-6-carbonitrile |
| 49 | 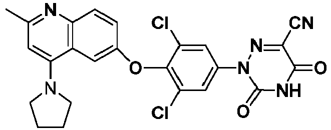 | 2-(3,5-Dichloro-4-((2-methyl-4-(pyrrolidin-1-yl)quinolin-6-yl)oxy) phenyl)-3,5-dioxo-2,3,4,5-tetrahydro -1,2,4-triazine-6-carbonitrile |
| 50 | 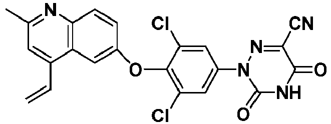 | 2-(3,5-Dichloro-4-((2-methyl-4-vinylquinolin-6-yl)oxy) phenyl)-3,5-dioxo-2,3,4,5-tetrahydro -1,2,4-triazine-6-carbonitrile |
| 51 | 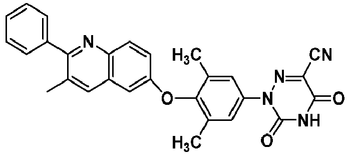 | 2-(3,5-Dimethyl-4-((3-methyl-2-phenyl quinolin-6-yl)oxy)phenyl -3,5-dioxo-2,3,4,5-tetrahydro-1,2,4-tri azine-6-carbonitrile |
| 52 | 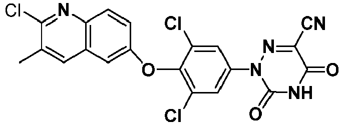 | 2-(3,5-Dichloro-4-((2-chloro-3-methylq uinolin-6-yl)oxy)phenyl) -3,5-dioxo-2,3,4,5-tetrahydro -1,2,4-triazine-6-carbonitrile |
| 53 | 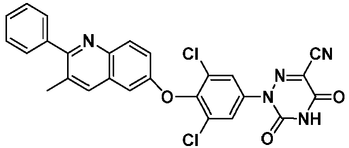 | 2-(3,5-Dichloro-4-((3-methyl -2-phenylquinolin-6-yl)oxy)phenyl -3,5-dioxo-2,3,4,5-tetrahydro -1,2,4-triazine-6-carbonitrile |
| 54 | 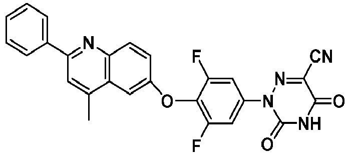 | 2-(3,5-Difluoro-4-((4-methyl-2-phenylq uinolin-6-yl)oxy)phenyl -3,5-dioxo-2,3,4,5-tetrahydro-1,2,4-tri azine-6-carbonitrile |
| 55 | 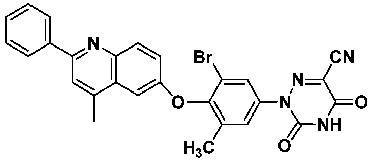 | 2-(3-bromo-5-methyl-4-((4-methyl-2-phenylquinolin-6-yl) oxy)phenyl -3,5-dioxo-2,3,4,5-tetrahydro -1,2,4-triazine-6-carbonitrile |
| 56 | 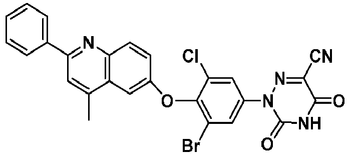 | 2-(3-bromo-5-chloro-4-((4-methyl-2-phenylquinolin-6-yl) oxy)phenyl -3,5-dioxo-2,3,4,5-tetrahydro -1,2,4-triazine-6-carbonitrile |
| 57 | 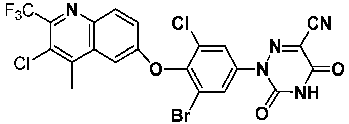 | 2-(3-bromo-5-chloro-4-((3-chloro-4-methyl-2-(trifluoromethy l)quinolin-6-yl)oxy)phenyl -3,5-dioxo-2,3,4,5-tetrahydro -1,2,4-triazine-6-carbonitrile |
| 58 | 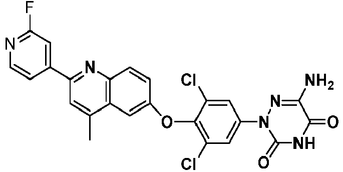 | 6-amino-2-(3,5-dichloro-4-((4-methyl-2-(2-fluoropyridin-4-yl) quinolin-6-yl)oxy)phenyl) -1,2,4-triazine-3,5(2H,4H)-dione |
| 59 | 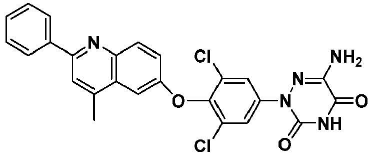 | 6-amino-2-(3,5-dichloro-4-((4-methyl -2-phenylquinolin-6-yl)oxy)phenyl)-1,2, 4-triazine-3,5(2H,4H)-dione |
| 60 | 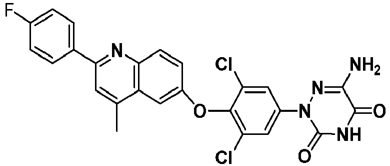 | 6-amino-2-(3,5-dichloro-4-((2-(4-fluo rophenyl)-4-methylquinolin-6-yl)oxy) phenyl)-1,2,4-triazine-3,5(2H,4H)-di one |
| 61 | 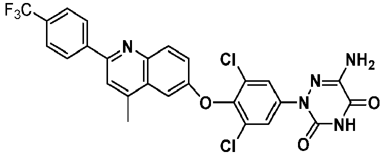 | 6-amino-2-(3,5-dichloro-4-((2-(4-trifl uoromethylphenyl)-4-methylquinolin-6-yl)oxy)phenyl)-1,2,4-triazine-3,5(2 H,4H)-dione |
| 62 | 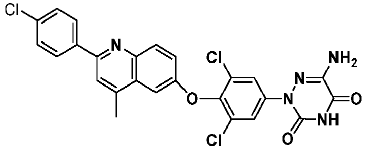 | 6-amino-2-(3,5-dichloro-4-((2-(4-chlorophenyl)-4-methylquinoli n-6-yl)oxy)phenyl) -1,2,4-triazine-3,5(2H,4H)-dione |
| 63 | 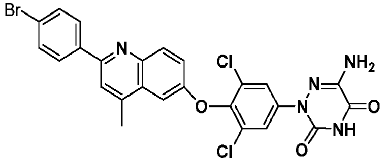 | 6-amino-2-(3,5-dichloro-4-((2-(4-bro mophenyl)-4-methylquinolin-6-yl)oxy )phenyl)-1,2,4-triazine-3,5(2H,4H)-di one |
| 64 | 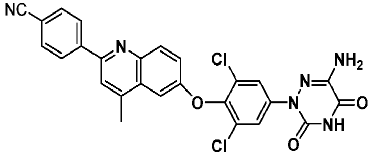 | 6-amino-2-(3,5-dichloro-4-((2-(4-cya nophenyl)-4-methylquinolin-6-yl)oxy )phenyl)-1,2,4-triazine-3,5(2H,4H)-di one |
| 65 | 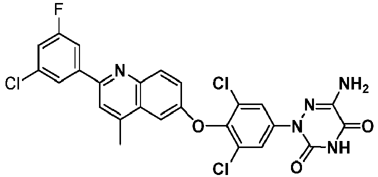 | 6-amino-2-(3,5-dichloro-4-((2-(3-chlo ro-5-fluorophenyl)-4-methylquinolin-6-yl)oxy)phenyl)-1,2,4-triazine-3,5(2H,4H)-dione |
| 66 | 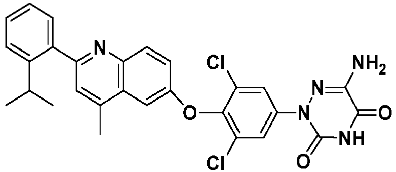 | 6-amino-2-(3,5-dichloro-4-((2-(2-isop ropylphenyl)-4-methylquinolin-6-yl)o xy)phenyl)-1,2,4-triazine-3,5(2H,4H) -dione |
| 67 | 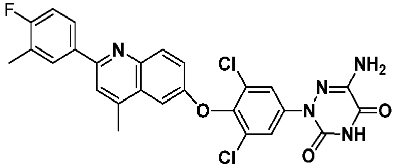 | 6-amino-2-(3,5-dichloro-4-((2-(4-fluo ro-3-methylphenyl)-4-methylquinolin -6-yl)oxy)phenyl)-1,2,4-triazine-3,5(2H,4H)-dione |
| 68 | 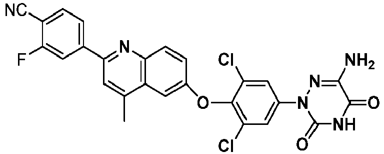 | 4-(6-(4-(6-amino-3,5-dioxo-4,5-dihyd ro-1,2,4-triazin-2(3H)-yl)-2,6-dichlor ophenoxy)-4-methylquinolin-2-yl)-2-fluorobenzoni trile |
| 69 | 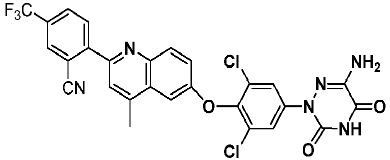 | 2-(6-(4-(6-amino-3,5-dioxo-4,5-dihyd ro-1,2,4-triazin-2(3H)yl)-2,6-dichloro phenoxy)-4-methylquinolin-2-yl)-5-(t rifluoromethyl)benzonitrile |
| 70 | 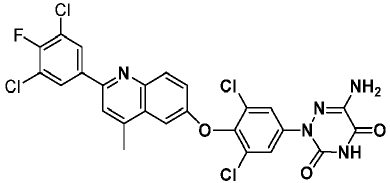 | 6-amino-2-(3,5-dichloro-4-((2-(3,5-di chloro-4-fluorophenyl)-4-methylquin olin-6-yl)oxy)phenyl)-1,2,4-triazine-3 ,5(2H,4H)dione |
| 71 | 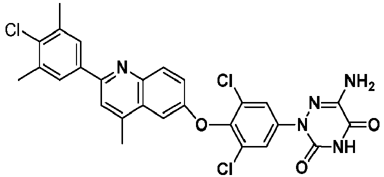 | 6-amino-2-(3,5-dichloro-4-((2-(4-chlo ro-3,5-dimethylphenyl)-4-methylquin olin-6-yl)oxy)-1,2,4-triazine-3,5(2H,4 H)-dione |
| 72 | 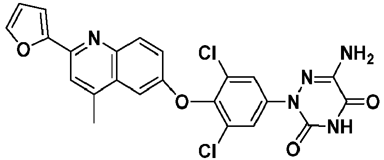 | 6-amino-2-(3,5-dichloro-4-((2-(furan-2-yl)-4-methylquinolin-6-yl)oxy)phen yl)-1,2,4-triazine-3, 5(2H,4H)-dione |
| 73 | 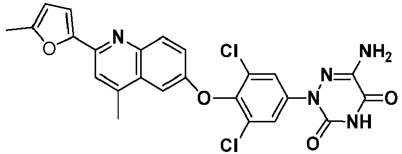 | 6-amino-2-(3,5-dichloro-4-((4-methyl -2-(5-methylfuran-2-yl)quinolin-6-yl) oxy)phenyl)-1,2,4-triazine -3,5(2H,4H)-dione |
| 74 | 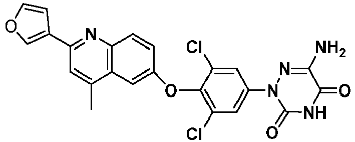 | 6-amino-2-(3,5-dichloro-4-((4-methyl -2-(3-methylfuran-3-yl)quinolin-6-yl) oxy)phenyl)-1,2,4-triazine -3,5(2H,4H)-dione |
| 75 | 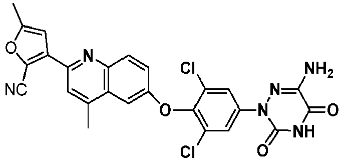 | 3-(6-(6-amino-3,5-dioxo-4,5-dihydro-1,2,4-triazine-2(3H)-yl)-2,6-dichlorop henoxy)-4-methylquinolin-2-yl)-5-methylfuran-2 -carbonitrile |
| 76 | 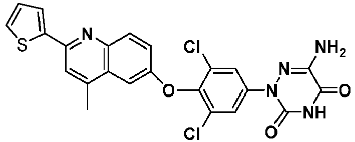 | 6-amino-2-(3,5-dichloro-4-((4-methyl -2-(thiophen-2-yl)quinolin-6-yl)oxy)p henyl)-1,2,4-triazine-3,5(2H,4H)-dio ne |
| 77 | 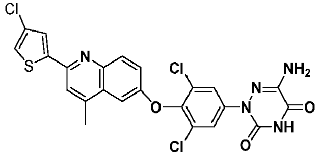 | 6-amino-2-(3,5-dichloro-4-((4-methyl -2-(thiophen-3 -yl)quinolin-6-yl)oxy)p henyl)-1,2,4-triazine-3,5(2H,4H)-dio ne |
| 78 | 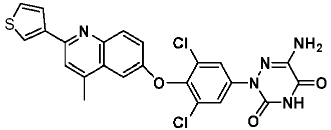 | 5-(6-(4-(6-amino-3,5-dioxo-4,5-dihyd ro-1,2,4-triazine-2(3H)-yl)-2,6-dichlo rophenoxy)-4-methylquinolin-2-yl)thiophen-2-carbo nitrile |
| 79 | 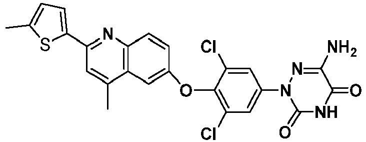 | 6-amino-2-(3,5-dichloro-4-((2-(5-met hylthiophen-2-yl)-4-methylquinolin-6 -yl)oxy)phenyl)-1,2,4-triazine-3,5(2H,4H)-dione |
| 80 | 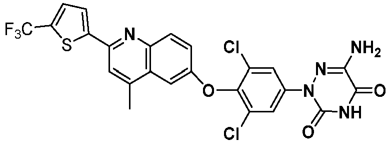 | 6-amino-2-(3,5-dichloro-4-((2-(5-trifl uoromethylthiophen-2-yl)-4-methylqu inolin-6-yl)oxy)phenyl)-1,2,4-triazine -3,5(2H,4H)-dione |
| 81 | 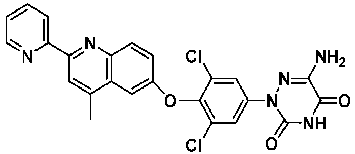 | 6-amino-2-(3,5-dichloro-4-((4-methyl-2-(pyridin-2-yl) quinolin-6-yl)oxy)phenyl) -1,2,4-triazine-3,5(2H,4H)-dione |
| 82 | 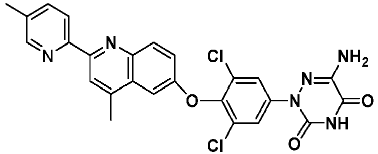 | 6-amino-2-(3,5-dichloro-4-((2-(5-met hylpyridin-2-yl)-4-methylquinolin-6-y l)oxy)phenyl)-1,2,4-triazine-3,5(2H,4H)-dione |
| 83 | 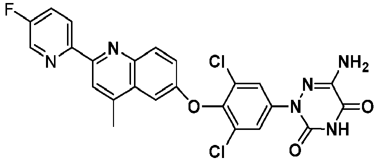 | 6-amino-2-(3,5-dichloro-4-((2-(5-fluo ropyridin-2-yl)-4-methylquinolin-6-yl )oxy)phenyl)-1,2,4-triazine-3,5(2H,4H)-dione |
| 84 | 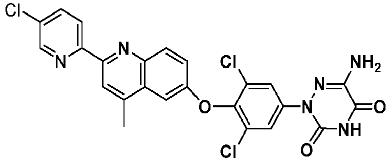 | 6-amino-2-(3,5-dichloro-4-((2-(5-chlo ropyridin-2-yl)-4-methylquinolin-6-yl )oxy)phenyl)-1,2,4-triazine-3,5(2H,4H)-dione |
| 85 | 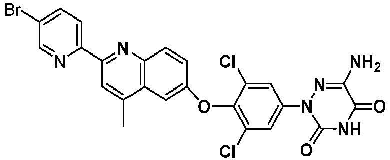 | 6-amino-2-(3,5-dichloro-4-((2-(5-bro mopyridin-2-yl)-4-methylquinolin-6-y l)oxy)phenyl)-1,2,4-triazine-3,5(2H,4H)-dione |
| 86 | 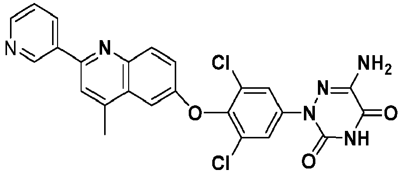 | 6-amino-2-(3,5-dichloro-4-((4-methyl-2-(pyridin-3-yl) quinolin-6-yl)oxy)phenyl) -1,2,4-triazine-3,5(2H,4H)-dione |
| 87 | 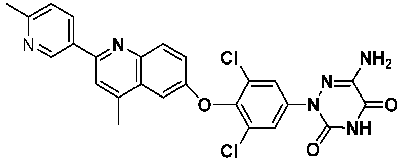 | 6-amino-2-(3,5-dichloro-4-((4-methyl-2-(6-methylpyridin-3-yl) quinolin-6-yl)oxy)phenyl) -1,2,4-triazine-3,5(2H,4H)-dione |
| 88 | 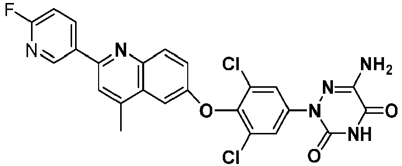 | 6-amino-2-(3,5-dichloro-4-((4-methyl-2-(6-fluoropyridin-3-yl) quinolin-6-yl)oxy)phenyl) -1,2,4-triazine-3,5(2H,4H)-dione |
| 89 | 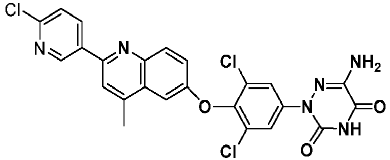 | 6-amino-2-(3,5-dichloro-4-((4-methyl-2-(6-chloropyridin-3-yl) quinolin-6-yl)oxy)phenyl) -1,2,4-triazine-3,5(2H,4H)-dione |
| 90 | 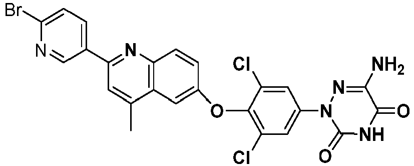 | 6-amino-2-(3,5-dichloro-4-((4-methyl-2-(6-bromopyridin-3-yl) quinolin-6-yl)oxy)phenyl) -1,2,4-triazine-3,5(2H,4H)-dione |
| 91 | 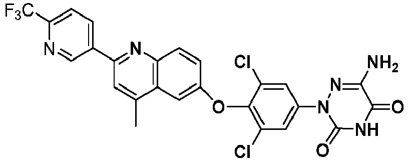 | 6-amino-2-(3,5-dichloro-4-((4-methyl-2-(6-trifluoromethylpyridin-3-yl) quinolin-6-yl)oxy)phenyl) -1,2,4-triazine-3,5(2H,4H)-dione |
| 92 | 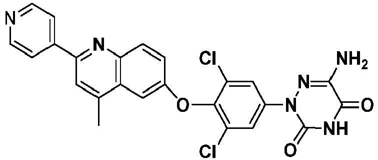 | 6-amino-2-(3,5-dichloro-4-((4-methyl-2-(pyridin-4-yl) quinolin-6-yl)oxy)phenyl) -1,2,4-triazine-3,5(2H,4H)-dione |
| 93 | 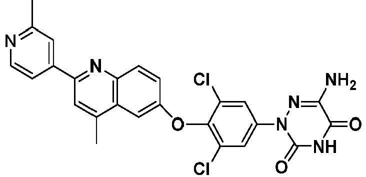 | 6-amino-2-(3,5-dichloro-4-((4-methyl-2-(2-methylpyridin-4-yl) quinolin-6-yl)oxy)phenyl) -1,2,4-triazine-3,5(2H,4H)-dione |
| 94 | 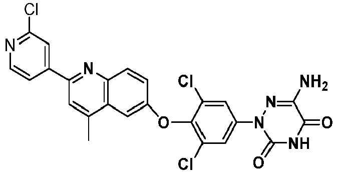 | 6-amino-2-(3,5-dichloro-4-((4-methyl-2-(2-chlorolpyridin-4-yl) quinolin-6-yl)oxy)phenyl) -1,2,4-triazine-3,5(2H,4H)-dione |
| 95 | 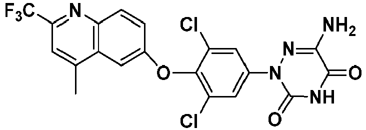 | 6-amino-2-(3,5-dichloro-4-((4-methyl-2-(trifluoromethyl) quinolin-6-yl)oxy)phenyl) -1,2,4-triazine-3,5(2H,4H)-dione |
| 96 | 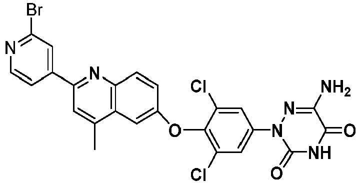 | 6-amino-2-(3,5-dichloro-4-((4-methyl-2-(2-bromopyridin-4-yl) quinolin-6-yl)oxy)phenyl) -1,2,4-triazine-3,5(2H,4H)-dione |
| 97 | 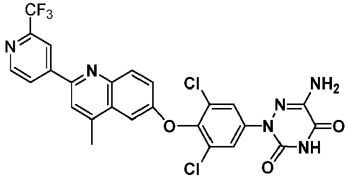 | 6-amino-2-(3,5-dichloro-4-((4-methyl-2-(2-trifluoromethylpyridin-4-yl) quinolin-6-yl)oxy)phenyl) -1,2,4-triazine-3,5(2H,4H)-dione |
| 98 | 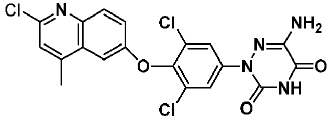 | 6-amino-2-(3,5-dichloro-4-((2-chloro-4-methylq uinolin-6-yl)oxy)phenyl) -1,2,4-triazine-3,5(2H,4H)-dione |
| 99 | 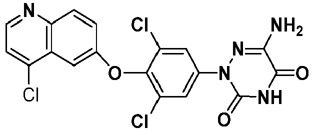 | 6-amino-2-(3,5-dichloro-4-((4-chloro quinolin-6-yl)oxy)phenyl)-1,2,4-triazi ne-3,5(2H,4H)-dione |
| 100 | 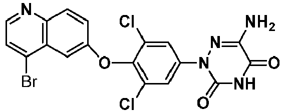 | 6-amino-2-(4-(4-bromoquinolin-6-yl) oxy)-3,5-dichlorophenyl)-1,2,4-triazin e-3,5(2H,4H)-dione |
| 101 | 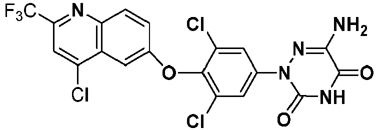 | 6-amino-2-(3,5-dichloro-4-((4-chloro-2-(trifluoro methyl)quinolin-6-yl)oxy)phenyl) -1,2,4-triazine-3,5(2H,4H)-dione |
| 102 | 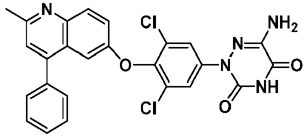 | 6-amino-2-(3,5-dichloro-4-((2-methyl -4-phenylquinolin-6-yl)oxy)phenyl-1,2,4 -triazine-3,5(2H,4H)-dione |
| 103 | 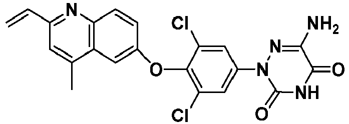 | 6-amino-2-(3,5-dichloro-4-((4-methyl -2-vinylquinolin-6-yl)oxy)phenyl)-1,2,4-triazine-3,5(2H,4H)-dione |
| 104 | 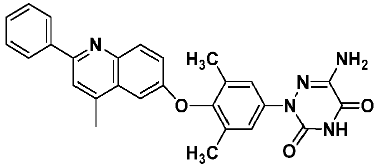 | 6-amino-2-(3,5-dimethyl-4-((4-methyl -2-phenylquinolin-6-yl)oxy)phenyl-1,2,4 -triazine-3,5(2H,4H)-dione |
| 105 | 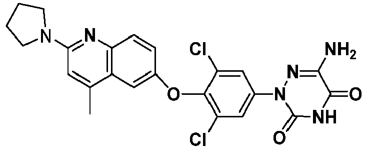 | 6-amino-2-(3,5-dichloro-4-((4-methyl -2-(pyrrolidin-1-yl)quinolin-6-oxy)ph enyl)-1,2,4-triazine-3,5(2H,4H)-dione |
| 106 | 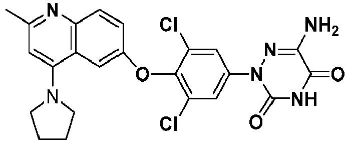 | 6-amino-2-(3,5-dichloro-4-((2-methyl -4-(pyrrolidin-1-yl)quinolin-6-oxy)ph enyl)-1,2,4-triazine-3,5(2H,4H)-dione |
| 107 | 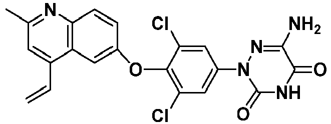 | 6-amino-2-(3,5-dichloro-4-((2-methyl -4-vinylquinolin-6-yl)oxy)phenyl)-1,2,4-triazine-3,5(2H,4H)-dione |
| 108 | 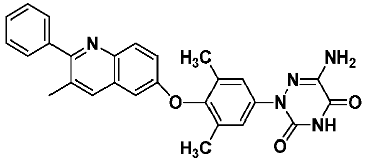 | 6-amino-2-(3,5-dimethyl-4-((3-methyl -2-phenylquinolin-6-yl)oxy)phenyl-1,2,4 -triazine-3,5(2H,4H)-dione |
| 109 | 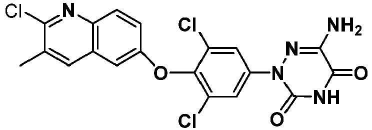 | 6-amino-2-(3,5-dichloro-4-((2-chloro-3-methylq uinolin-6-yl)oxy)phenyl) -1,2,4-triazine-3,5(2H,4H)-dione |
| 110 | 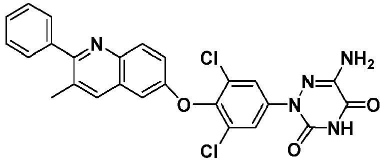 | 6-amino-2-(3,5-dichloro-4-((3-methyl -2-phenylquinolin-6-yl)oxy)phenyl-1,2,4 -triazine-3,5(2H,4H)-dione |
| 111 | 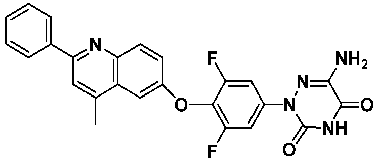 | 6-amino-2-(3,5-difluoro-4-((4-methyl -2-phenylquinolin-6-yl)oxy phenyl)-1,2,4-triazine-3,5(2H,4H)-di one |
| 112 | 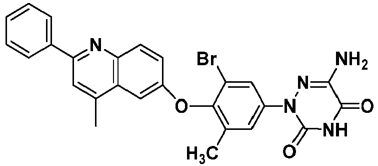 | 6-amino-2-(3-bromo-5-methyl-4-(4-m ethyl-2-phenylquinolin-6-yl)oxy)phenyl-1,2,4 -triazine-3,5(2H,4H)-dione |
| 113 | 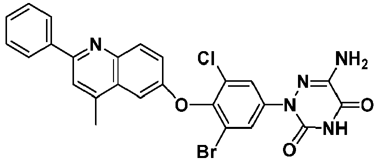 | 6-amino-2-(3-bromo-5-chloro-4-(4-m ethyl-2-phenylquinolin-6-yl)oxy)phenyl-1,2,4 -triazine-3,5(2H,4H)-dione |
| 114 | 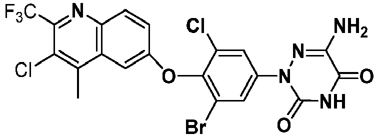 | 6-amino-2-(3-bromo-5-chloro-4-((3-c hloro-4-methyl-2-(trifluoromethyl)qui nolin-6-yl)oxyphenyl)-1,2,4-triazine-3,5(2H,4H)-dione |
| 115 | 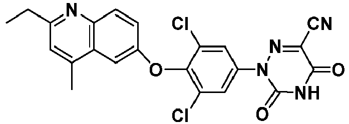 | 2-(3,5-Dichloro-4-((2-ethyl -4-methylquinolin-6-yl)oxy)phenyl) -3,5-dioxo-2,3,4,5-tetrahydro -1,2,4-triazine-6-carbonitrile |
| 116 | 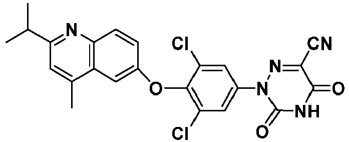 | 2-(3,5-Dichloro-4-((2-isopropyl-4-meth ylquinolin-6-yl)oxy)phenyl) -3,5-dioxo-2,3,4,5-tetrahydro -1,2,4-triazine-6-carbonitrile |
| 117 | 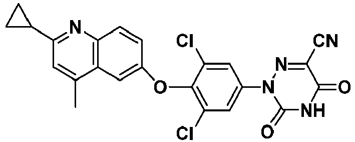 | 2-(3,5-Dichloro-4-((2-cyclopropyl -4-methylquinolin-6-yl)oxy)phenyl) -3,5-dioxo-2,3,4,5-tetrahydro -1,2,4-triazine-6-carbonitrile |
| 118 | 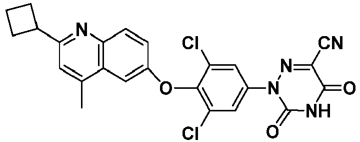 | 2-(3,5-Dichloro-4-((2-cyclobutyl-4-met hylquinolin-6-yl)oxy)phenyl) -3,5-dioxo-2,3,4,5-tetrahydro -1,2,4-triazine-6-carbonitrile |
| 119 | 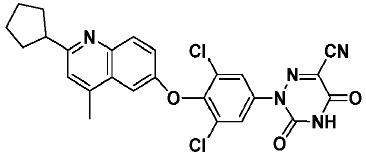 | 2-(3,5-Dichloro-4-((2-cyclopentyl -4-methylquinolin-6-yl)oxy)phenyl) -3,5-dioxo-2,3,4,5-tetrahydro -1,2,4-triazine-6-carbonitrile |
| 120 | 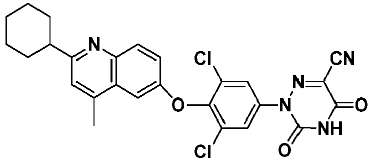 | 2-(3,5-Dichloro-4-((2-cyclohexyl-4-methylquinolin-6-yl) oxy)phenyl) -3,5-dioxo-2,3,4,5-tetrahydro -1,2,4-triazine-6-carbonitrile |
| 121 | 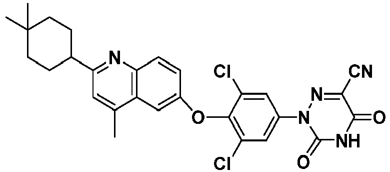 | 2-(3,5-dichloro-4-((2-(4,4-dimethylcy clohexyl)-4-methylquinolin-6-yl)oxy) phenyl)-3,5-dioxo-2,3,4,5-tetrahydro-1,2,4-triazine-6-carbonitrile |
| 122 | 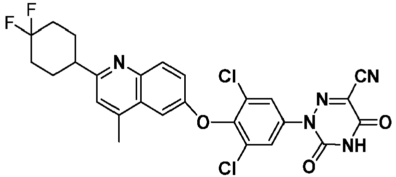 | 2-(3,5-Dichloro-4-((2-(4,4-difluorocyclohexyl) -4-methylquinolin-6-yl)oxy)phenyl) -3,5-dioxo-2,3,4,5-tetrahydro -1,2,4-triazine-6-carbonitrile |
| 123 | 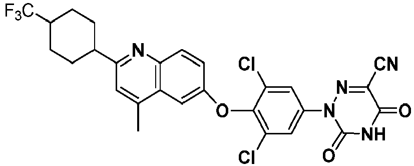 | 2-(3,5-dichloro-4-((4-methyl-2-(4-(tri fluoromethyl)cyclohexyl)quinolin-6-y l)oxy)phenyl)-3,5-dioxo-2,3,4,5-tetra hydro-1,2,4-triazine-6-carbonitrile |
| 124 | 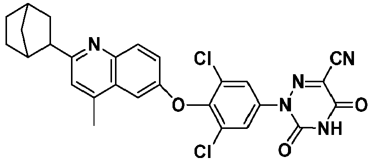 | 2-(4-((2-(bicyclo[2.2.1]heptan-2-yl)-4 -methylquinolin-6-yl)oxy)-3,5-dichlor ophenyl)-3,5-dioxo-2,3,4,5-tetrahydro -1,2,4-triazine-6-carbonitrile |
| 125 | 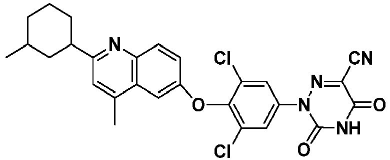 | 2-(3,5-dichloro-4-((4-methyl-2-(3-met hylcyclohexyl)quinolin-6-yl)oxy)phen yl)-3,5-dioxo-2,3,4,5-tetrahydro-1,2,4 -triazine-6-carbonitrile |
| 126 | 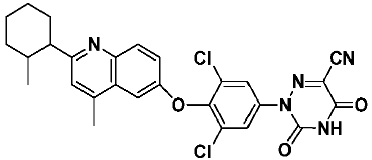 | 2-(3,5-dichloro-4-((4-methyl-2-(2-met hylcyclohexyl)quinolin-6-yl)oxy)phen yl)-3,5-dioxo-2,3,4,5-tetrahydro-1,2,4 -triazine-6-carbonitrile |
| 127 | 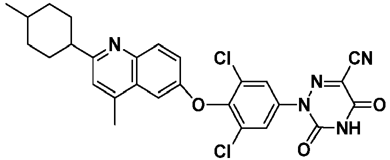 | 2-(3,5-dichloro-4-((4-methyl-2-(4-met hylcyclohexyl)quinolin-6-yl)oxy)phen yl)-3,5-dioxo-2,3,4,5-tetrahydro-1,2,4 -triazine-6-carbonitrile |
| 128 | 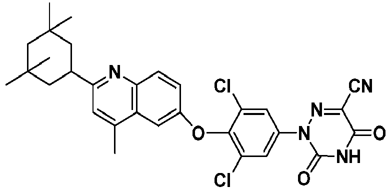 | 2-(3,5-dichloro-4-(4-methyl-2-(3,3,5, 5-tetramethylcyclohexyl)quinolin-6-yl )oxy)phenyl)-3,5-dioxo-2,3,4,5-tetrah ydro-1,2,4-triazine-6-carbonitrile |
| 129 | 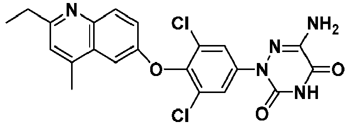 | 6-amino-2-((3,5-dichloro-4-(2-ethyl-4 -methylquinolin-6-yl)oxy)phenyl)-1,2 ,4-triazine-3,5(2H,4H)-dione |
| 130 | 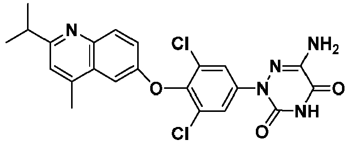 | 6-amino-2-((3,5-dichloro-4-(2-isopro pyl-4-methylquinolin-6-yl)oxy)phenyl )-1,2,4-triazine-3,5( 2H,4H)-dione |
| 131 | 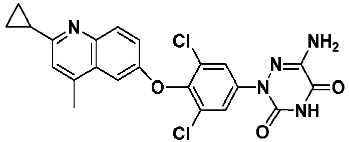 | 6-amino-2-(3,5-dichloro-4-((2-cyclop ropyl-4-methylquinolin-6-yl)oxy)phe nyl)-1,2,4-triazine-3,5( 2H,4H)-dione |
| 132 | 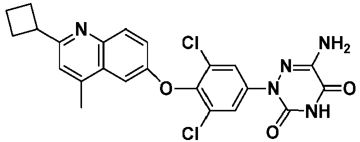 | 6-amino-2-(3,5-dichloro-4-((2-cyclob utyl-4-methylquinolin-6-yl)oxy)pheny l)-1,2,4-triazine-3,5( 2H,4H)-dione |
| 133 | 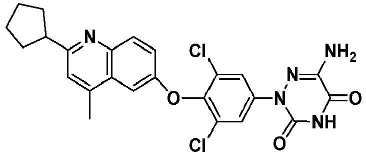 | 6-amino-2-(3,5-dichloro-4-((2-cyclop entyl-4-methylquinolin-6-yl)oxy)phen yl)-1,2,4-triazine-3,5( 2H,4H)-dione |
| 134 | 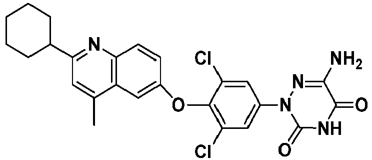 | 6-amino-2-(3,5-dichloro-4-((2-cycloh exyl-4-methylquinolin-6-yl)oxy)phen yl)-1,2,4-triazine-3,5( 2H,4H)-dione |
| 135 | 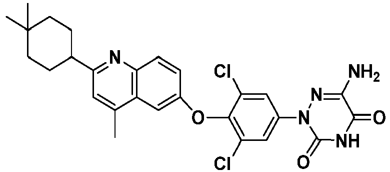 | 6-amino-2-(3,5-dichloro-4-((2-(4,4-di methylcyclohexyl)-4-methylquinolin-6-yl)oxy)phenyl)-1,2,4-triazine-3,5(2 H,4H)-dione |
| 136 | 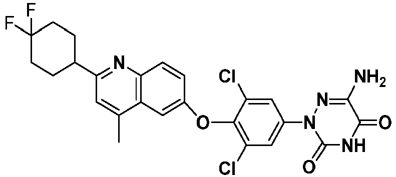 | 6-amino-2-(3,5-dichloro-4-((2-(4,4-di fluorocyclohexyl)-4-methylquinolin-6 -yl)oxy)phenyl)-1,2,4-triazine-3,5(2H ,4H)-dione |
| 137 | 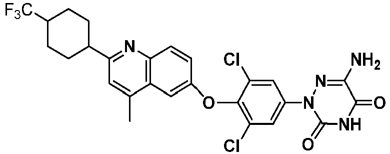 | 6-amino-2-(3,5-dichloro-4-((4-methyl -2-(4-(trifluoromethyl)cyclohexyl)qui nolin-6-yl)oxy)phenyl)-1,2,4-triazine-3,5(2H,4H)-dione |
| 138 | 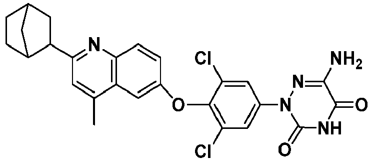 | 6-amino-2-(4-(2-(bicyclo[2.2.1]hepta n-2-yl)-4-methylquinolin-6-yl)oxy)-3, 5-dichlorophenyl)-1,2,4-triazine-3,5(2 H,4H)-dione |
| 139 | 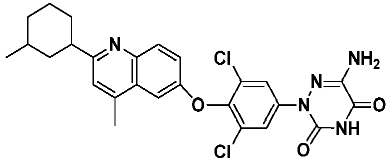 | 6-amino-2-(3,5-dichloro-4-((4-methyl -2-(3-methylcyclohexyl)quinolin-6-yl )oxy)phenyl)-1,2,4-triazine-3,5(2H,4 H)-dione |
| 140 | 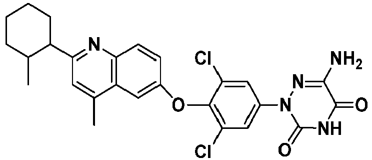 | 6-amino-2-(3,5-dichloro-4-((4-methyl -2-(2-methylcyclohexyl)quinolin-6-yl )oxy)phenyl)-1,2,4-triazine-3,5(2H,4 H)-dione |
| 141 | 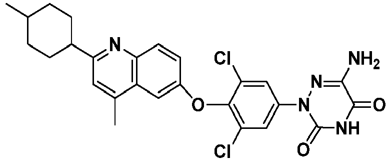 | 6-amino-2-(3,5-dichloro-4-((4-methyl -2-(4-methylcyclohexyl)quinolin-6-yl )oxy)phenyl)-1,2,4-triazine-3,5(2H,4 H)-dione |
| 142 | 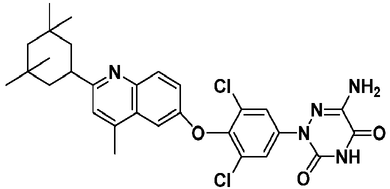 | 6-amino-2-(3,5-dichloro-4-((4-methyl -2-(3,3,5,5-tetramethylcyclohexyl)qui nolin-6-yl)oxy)phenyl)-1,2,4-triazine-3,5(2H,4H)-dione |

8. A compound of formula (II), or a stereoisomer, a deuterated compound, a solvate, a metabolite, a pharmaceutically acceptable salt, a eutectic or a prodrug thereof, 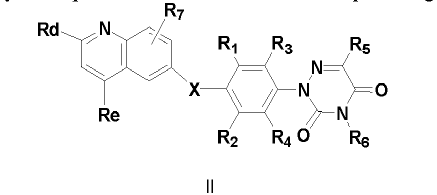 wherein,
Rd and Re are independently selected from the group consisting of: H, halogen, C1-C6 alkyl, and C1-C6 haloalkyl; and at least one of Rd and Re is halogen;
R₇ is one or more (such as 1, 2, 3, or 4) substituents located on the benzene of the quinoline ring and is selected from the group consisting of: H, halogen, C1-C6 alkyl, and C1-C6 haloalkyl;
X is selected from the group consisting of: -O-, -CH₂-, -C(=O)-, -CH(OH)-, and -S-;
R₁, R₂, R₃ and R₄ are independently selected from the group consisting of: H, D, F, Cl, Br, I, -CN, -OH, -NO₂, -OR₁ₐ, -NR_{1b}R_{1c}, -SR_{1b}, C1-C6 alkyl, C1-C6 alkoxy, C2-C6 alkenyl, C2-C6 alkynyl, C3-C8 cycloalkyl, 3-8-membered heterocycloalkyl, C5-C6 aryl, and 5-6-membered heteroaryl;
R₅ is selected from the group consisting of: H, D, F, Cl, Br, I, -CN, -OH, -NO₂, -OR₁ₐ, -NR_{1b}R_{1c}, -SR_{1b}, -S(O)R₁ₐ, -S(O)₂R₁ₐ, -S(O)NR₁ₐR_{1b}, -S(O)₂NR₁ₐR_{1b}, -NHS(O)₂R₁ₐ, -C(O)R₁ₐ, -OC(O)R₁ₐ, -C(O)OR_{1b}, -C(O)NR_{1b}R_{1c}, -NHC(O)R₁ₐ, C1-C6 alkyl, C1-C6 alkoxy, C2-C6 alkenyl , C2-C6 alkynyl, C3-C8 cycloalkyl, 3-8-membered heterocyclyl, C5-C6 aryl and 5-6-membered heteroaryl;
R₆ is selected from the group consisting of: H, D, C1-C6 alkyl, amino substituted C1-C6 alkyl, C3-C8 cycloalkyl, 3-8-membered heterocyclyl, -SR_{1b}, -S(O)R₁ₐ, -S(O)₂R₁ₐ, -S(O)NR₁ₐR_{1b}, -C(O)R₁ₐ, -C(O)OR_{1b}, and -C(O)NR_{1b}R_{1c};
unless otherwise specified, each alkyl, alkoxy, alkenyl, alkynyl, cycloalkyl, heterocyclyl, aryl, and heteroaryl mentioned above may optionally substituted by 1-3 substituents selected from the group consisting of: D, halogen, -CN, -OH, oxo-, -OR₁ₐ, -NR_{1b}R_{1c}, -SR_{1b}, -S(O)R₁ₐ, -S(O)₂R₁ₐ, -S(O)NR₁ₐR_{1b}, -S(O)₂NR₁ₐR_{1b}, -NHS(O)₂R₁ₐ, -C(O)R₁ₐ, - OC(O)R₁ₐ, -C(O)OR_{1b}, - C(O)NR_{1b}R_{1c}, -NHC(O)R₁ₐ, C1-C6 alkyl, and C1-C6 haloalkyl;
R₁ₐ, R_{1b} and R_{1c} are each independently selected from: H, D, C1-C6 alkyl, C2-C6 alkenyl, C2-C6 alkynyl, C3-C8 cycloalkyl, 3-8-membered heterocycloalkyl, C5-C6 aryl and 5-6-membered heteroaryl.

9. The compound according to claim 1, comprising a stereoisomer, a deuterated compound, a solvate, a metabolite, a pharmaceutically acceptable salt, a eutectic or a prodrug thereof, wherein, R₁, R₂, R₃, and R₄ are independently selected from H, D, halogen and C1-C4 alkyl.

10. A pharmaceutical composition, wherein the pharmaceutical composition comprises the compound according to claim 1 or 8, or a stereoisomer, a deuterated compound, a solvate, a metabolite, a pharmaceutically acceptable salt, a eutectic or a prodrug thereof; and pharmaceutically acceptable excipients and/or carriers.

11. A use of the compound according to claim 1 or 8, or a stereoisomer, a deuterated compound, a solvate, a metabolite, a pharmaceutically acceptable salt, a eutectic or a prodrug thereof in the preparation of a medication, wherein, the medication is used for agonizing thyroid hormone receptors, or for preventing, treating or alleviating diseases mediated by thyroid hormone receptors.

12. The use according to claim 11, wherein, the diseases mediated by thyroid hormone receptors are selected from the group consisting of: non-alcoholic fatty liver diseases, atherosclerosis, coronary heart disease, hypertension, hypercholesterolemia, hyperlipidemia, hypertriglyceridemia, dyslipidemia, obesity, diabetes, metabolic disorders, lipid metabolism disorders, type 1A glycogen storage disease, hypothyroidism, and thyroid cancer.
